# EUROPEAN PATENT APPLICATION

(11) **EP 2 298 878 A2**
(43) Date of publication of application: **23.03.2011**
(21) Application number: 10185041.0
(22) Date of filing: 02.11.2001
(51) Int. Cl.: C12N 15/00, C07K 14/00, C07K 16/00, G01N 33/574, C12Q 1/68, A01K 67/027

(54) **COMPOSITIONS AND METHODS FOR THE DIAGNOSIS OF CANCER**

(30) Priority: 03.11.2000 US 245756 P
(62) Divisional of application: 01992774.8
(71) Applicant: Dana Farber Cancer Institute, Boston, MA 02155 (US); OREGON HEALTH SCIENCES UNIVERSITY, Portland, OR 97201 (US)
(72) Inventor: D'Andrea, Alan D., Winchester, MA MA 01890 (US); Taniguchi, Toshiyasu, Seattle, WA 98109-1024 (US); Timmers, Cynthia, Columbus, OH OH 43228 (US); Grompe, Markus, Portland, OR OR 97201 (US)
(74) Representative: Finnie, Isobel Lara

(57) **Abstract**

Methods and compositions for the diagnosis of cancer susceptibilities, defective DNA repair mechanisms and treatments thereof are provided. Among sequences provided here, the FANCD2 gene has been identified, mapped on the 3p chromosome, cloned into recombinant vectors, used to prepare recombinant cells and sequenced. The FANCD2 gene sequence provides probes and primers for screening patients in genetic based test and for diagnosing Fanconi anemia and cancer. It has also been possible to target the FANCD2 *gene in vivo* for preparing experimental mouse models for use in screening new therapeutic agents for treating conditions involving defective DNA repair. Vectors are described for use in gene therapy. The FANCD2 polypeptide has been sequenced and has been shown to exist in two isoforms identified as FANCD2-S and the mono-ubiquinated FANCD-L form. Antibodies including polyclonal and monoclonal antibodies have been prepared that distinguish the two isoforms and have been used in diagnostic tests to determine whether a subject has an intact FA pathway. The FANCD2 has been localized to the nucleus and is associated with BRCA 1 foci.

## Description

The work described herein was supported by the National Institute of Health, NIH Grant vNo. Health grants RO1HL52725-04, RO1 DK43889-09, 1PO1HL48546, and PO1HL54785-04. The US Government has certain rights to the claimed invention

### Technical Field and Background Art

The present invention relates to the diagnosis of cancer susceptibilities in subjects having a defect in the FANCD2 gene and the determination of suitable treatment protocols for those subjects who have developed cancer. Animal models with defects in the FANCD2 gene can be used to screen for therapeutic agents.

Fanconi Anemia (FA) is an autosomal recessive cancer susceptibility syndrome characterized by birth defects, bone marrow failure and cancer predisposition. Cells from FA patients display a characteristic hypersensitivity to agents that produce interstrand DNA crosslinks such as mitomycin C or diepoxybutane. FA patients develop several types of cancers including acute myeloid leukemias and cancers of the skin, gastrointestinal; and gynecological systems. The skin and gastrointestinal tumors are usually squamous cell carcinomas. At least 20% of patients with FA develop cancers. The average age of patients who develop cancer is 15 years for leukemia, 16 years for liver tumors and 23 years for other tumors. (D'Andrea et al., Blood, (1997) Vol. 90, pp. 1725, Garcia-Higuera et al. Curr. Opin. Hematol, (1999) Vol 2, pp. 83-88 and Heijna et al. Am. J. Hum. Genet. Vol 66, pp 1540-1551)

FA is genetically heterogeneous. Somatic cell fusion studies have identified at least seven distinct complementation groups (Joenje et al., (1997) Am. J. Hum. Genet., Vol. 61, pp. 940-944 and Joenje et al., (2000) Am. J. Hum. Genet, Vol. 67, pp. 759-762). This observation has resulted in the hypothesis that the FA genes define a multicomponent pathway involved in cellular responses to DNA cross-links. Five of the FA genes (FANCA, FANCC, FANCE, FANCF and FANCG) have been cloned and the FANCA, FANCC and FANCG proteins have been shown to form a molecular complex with primarily nuclear localization. FANCC also localizes in the cytoplasm. Different FA proteins have few or no known sequence motifs with no strong homologs of the FANCA, FANCC, FANCE, FANCF, and FANCG proteins in non-vertebrate species. FANCF has weak homology of unknown significance to an E. Coli RNA binding protein. The two most frequent complementation groups are FA-A and FA-C which together account for 75%-80% of FA patients. Multiple mutations have been recognized in the FANCA gene that span 80kb and consists of at least 43 exons. FANCC has been found to have 14 exons and spans approximately 80kb. A number of mutations in the FANCC gene have been identified which are correlated with FA of differing degrees of severity. FA-D has been identified as a distinct but rare complementation group. Although FA-D patients are phenotypically distinguishable from patients from other subtypes, the FA protein complex assembles normally in FA-D cells (Yamashita et al., (1998) P.N.A.S., Vol. 95, pp.13085―13090)

The cloned FA proteins encode orphan proteins with no sequence similarity to each other or to other proteins in GenBank and no functional domains are apparent in the protein sequence. Little is known regarding the cellular or biochemical function of these proteins.

Diagnosis of FA is complicated by the wide variability in FA patient phenotype. Further confounding diagnosis, approximately 33% of patients with FA have no obvious congenital abnormalities. Moreover, existing diagnostic tests do not differentiate FA carriers from the general population. The problems associated with diagnosis are described in D'Andrea et al., (1997). Many cellular phenotypes have been reported in FA cells but the most consistent is hypersensitivity to bifunctional alkylating agents such as mitomycin C or diepoxybutane. These agents produce interstrand DNA cross-links (an important class of DNA damage).

Diagnosing cancer susceptibility is complicated because of the large number of regulatory genes and biochemical pathways that have been implicated in the formation of cancers. Different cancers depending on how they arise and the genetic lesions involved may determine how a subject responds to any particular therapeutic treatments. Genetic lesions that are associated with defective repair mechanisms may give rise to defective cell division and apoptosis which in turn may increase a patient's susceptibility to cancer. FA is a disease condition in which multiple pathological outcomes are associated with defective repair mechanisms in addition to cancer susceptibility

An understanding of the molecular genetics and cell biology of Fanconi Anemia pathway can provide insights into prognosis, diagnosis and treatment of particular classes of cancers and conditions relating to defects in DNA repair mechanisms that arise in non-FA patients as well as FA patients

### Summary of the Invention

In a first embodiment of the invention there is provided an isolated nucleic acid molecule that includes a polynucleotide selected from (a) a nucleotide sequence encoding a polypeptide having an aminoacid sequence as shown in SEQ ID NO: 4 (b) a nucleotide sequence at least 90% identical to the polynucleotide of (b); (c) a nucleotide sequence complementary to the polynucleotide of (b); (d) a nucleotide sequence at least 90% identical to the nucleotide sequence shown in SEQ ID No: 5-8,187-188; and (e) a nucleotide sequence complementary to the nucleotide sequence of (d). The polynucleotide may be an RNA molecule or a DNA molecule, such as a cDNA.

In another embodiment of the invention, an isolated nucleic acid molecule is provided that consists essentially of a nucleotide sequence encoding a polypeptide having an amino acid sequence sufficiently similar to that of SEQ ID No: 4 to retain the biological property of conversion from a short form to a long form of FANCD2 in the nucleus of a cell for facilitating DNA repair. Alternately, the isolated nucleic acid molecule consists essentially of a polynucleotide having a nucleotide sequence at least 90% identical to SEQ ID NO: 9-191 or complementary to a nucleotide sequence that is at least 90% identical to SEQ ID NO: 9-191.

In an embodiment, a method is provided for making a recombinant vector that includes inserting any of the isolated nucleic acid molecules described above into a vector. A recombinant vector product may be made by this method and the vector may be introduced to form a recombinant host cell into a host cell.

In an embodiment of the invention, a method is provided for making an FA-D2 cell line, that includes (a) obtaining cells from a subject having a biallelic mutation in a complementation group associated with FA-D2; and (b) infecting the cells with a transforming virus to make the FA-D2 cell line where the cells may be selected from fibroblasts and lymphocytes and the transforming virus selected from Epstein Barr virus and retrovirus. The FA-D2 cell line may be characterized by determining the presence of a defective FANDC2 in the cell line for example by performing a diagnostic assay selected from (i) a Western blot or nuclear immunofluorescence using an antibody specific for FANCD2 and (ii) a DNA hybridization assay.

In an embodiment of the invention, a recombinant method is provided for producing a polypeptide, that includes culturing a recombinant host cell wherein the host cell includes any of the isolated nucleic acid molecules described above.

In an embodiment of the invention, an isolated polypeptide, including an aminoacid sequence selected from (a) SEQ ID NO: 4; (b) an aminoacid sequence at least 90% identical to (a); (c)
an aminoacid sequence which is encoded by a polynucleotide having a nucleotide sequence which is at least 90% identical to at least one of SEQ ID NO: 5-8, 187-188; (d) an aminoacid sequence which is encoded by a polynucleotide having a nucleotide sequence which is at least 90% identical to a complementary sequence to at least one of SEQ ID NO: 5-8, 187-188; and (e) a polypeptide fragment of (a) - (d) wherein the fragment is at least 50 aminoacids in length.

The isolated polypeptide may be encoded by a DNA having a mutation selected from nt 376 A to G, nt 3707 G to A, nt904C to T and nt 958C to T. Alternatively, the polypeptide may be characterized by a polymorphism in DNA encoding the polypeptide, the polymorphism being selected from nt 1122A to G, nt 1440T to C, ntl509C to T, nt2141C to T, nt2259T to C, nt4098T to G, nt4453G to A. Alternatively, the polypeptide may be characterized by a mutation at aminoacid 222 or aminoacid 561.

In an embodiment of the invention, an antibody preparation is described having a binding specificity for a FANCD2 protein where the antibody may be a monoclonal antibody or a polyclonal antibody and wherein the FANCD2 may be FANCD2-S or FANCD2-L.

In an embodiment of the invention, a diagnostic method is provided for measuring FANCD2 isoforms in a biological sample where the method includes (a) exposing the sample to a first antibody for forming a first complex with FANCD2-L and optionally a second antibody for forming a second complex with FANCD2-S; and (b) detecting with a marker, the amount of the first complex and the second complex in the sample. The sample may be intact cells or lyzed cells in a lysate. The biological sample may be from a human subject with a susceptibility to cancer or having the initial stages of cancer. The sample may be from a cancer in a human subject, wherein the cancer is selected from melanoma, leukemia, astocytoma, glioblastoma, lymphoma, glioma, Hodgkins lymphoma, chronic lymphocyte leukemia and cancer of the pancreas, breast, thyroid, ovary, uterus, testis, pituitary, kidney, stomach, esophagus and rectum. The biological sample may be from a human fetus or from an adult human and may be derived from any of a blood sample, a biopsy sample of tissue from the subject and a cell line. The biological sample may be derived from heart, brain, placenta, liver, skeletal muscle, kidney, pancreas, spleen, thymus, prostate, testis, uterus, small intestine, colon, peripheral blood or lymphocytes. The marker may be a fluorescent marker, the fluorescent marker optionally conjugated to the FANCD2-L antibody, a chemiluminescent marker optionally conjugated to the FANCD2-L antibody and may bind the first and the second complex to a third antibody conjugated to a substrate. Where the sample is a lysate, it may be subjected to a separation procedure to separate FANCD2 isoforms and the seperated isoforms may be identified by determining binding to the first or the second FANCD2 antibody.

In an embodiment of the invention, a diagnostic test is provided for identifying a defect in the Fanconi Anemia pathway in a cell population from a subject, that includes selecting an antibody to FANCD2 protein and determining whether the amount of an FAND2-L isoform is reduced in the cell population compared with amounts, in a wild type cell population; such that if the amount of the FANCD2-L protein is reduced, then determining whether an amount of any of FANCA, FANCB, FANCC, FANCD1, FANCE, FANCF or FANCG protein is altered in the cell population compared with the wild type so as to identify the defect in the Fanconi Anemia pathway in the cell population. In one example, the amount of an isoform relies on a separation of the FANCD2-L and FANCD2-S isoforms where the separation may be achieved by gel electrophoresis or by a migration binding banded test strip.

In an embodiment of the invention, a screening assay for identifying a therapeutic agent, is provided that includes selecting a cell population in which FAND2-L is made in reduced amounts; exposing the cell population to individual members of a library of candidate therapeutic molecules; and identifying those individual member molecules that cause the amount of FANCD2-L to be increased in the cell population. In one example, the cell population is an *in vitro* cell population. In another example, the cell population is an *in vivo* cell population, the *in vivo* population being within an experimental animal, the experimental animal having a mutant FANCD2 gene. In a further example, the experimental animal is a knock-out mouse in which the mouse FAND2 gene has been replaced by a human mutant FANCD2 gene. In another example, a chemical carcinogen is added to the cell population in which FANCD2 is made in reduced amounts, to determine if any member molecules can cause the amount of FANCD2-L to be increased so as to protect the cells form the harmful effects of the chemical carcinogen.

In an embodiment of the invention, an experimental animal model is provided in which the animal FANCD2 gene has been removed and optionally replaced by any of the nucleic acid molecules described above

In an embodiment of the invention, a method is provided for identifying in a cell sample from a subject, a mutant FANCD2 nucleotide sequence in a suspected mutant FANCD2 allele which comprises comparing the nucleotide sequence of the suspected mutant FANCD2 allele with the wild type FANCD2 nucleotide sequence wherein a difference between the suspected mutant and the wild type sequence identifies a mutant FANCD2 nucleotide sequence in the cell sample. In one example, the suspected mutant allele is a germline allele. In another example, identification of a mutant FANCD2 nucleotide sequence is diagnostic for a predisposition for a cancer in the subject or for an increased risk of the subject bearing an offspring with Fanconi Anemia. In another example, the suspected mutant allele is a somatic allele in a tumor type and identifying a mutant FANCD2 nucleotide sequence is diagnostic for the tumor type. In another example, the nucleotide sequence of the wild type and the suspected mutant FANCD2 nucleotide sequence is selected from a gene, a mRNA and a cDNA made from a mRNA. In another example, comparing the polynucleotide sequence of the suspected mutant FANCD2 allele with the wild type FANCD2 polynucleotide sequence, further includes selecting a FANCD2 probe which specifically hybridizes to the mutant FANCD2 nucleotide sequence, and detecting the presence of the mutant sequence by hybridization with the probe. In another example, comparing the polynucleotide sequence of the suspected mutant FANCD2 allele with the wild type FANCD2 polynucleotide sequence, further comprises amplifying all or part of the PANCD2 gene using a set of primers specific for wild type FANCD2 DNA to produce amplified FANCD2 DNA and sequencing the FANCD2 DNA so as to identify the mutant sequence. In another example, where the mutant FANCD2 nucleotide sequence is a germline alteration in the FANCD2 allele of the human subject, the alteration is selected from the alterations set forth in Table 3 and where the mutant FANCD2 nucleotide sequence is a somatic alteration in the FANCD2 allele of the human subject, the alteration is selected from the alterations set forth in Table 3.

In an embodiment of the invention, a method is provided for diagnosing a susceptibility to cancer in a subject which comprises comparing the germline sequence of the FANCD2 gene or the sequence of its mRNA in a tissue sample from the subject with the germline sequence of the FANCD2 gene or the sequence of its mRNA wherein an alteration in the germline sequence of the FANCD2 gene or the sequence of its mRNA of the subject indicates the susceptibility to the cancer. An alteration may be detected in a regulatory region of the FANCD2 gene. An alteration in the germline sequence may be determined by an assay selected from the group consisting of (a) observing shifts in electrophoretic mobility of single-stranded DNA on non-denaturing polyacrylamide gels, (b) hybridizing a FANCD2 gene probe to genomic DNA isolated from the tissue sample, (c) hybridizing an allele-specific probe to genomic DNA of the tissue sample, (d) amplifying all or part of the FANCD2 gene from the tissue sample to produce an amplified sequence and sequencing the amplified sequence, (e) amplifying all or part of the FANCD2 gene from the tissue sample using primers for a specific FANCD2 mutant allele, (f) molecularly cloning all or part of the FANCD2 gene from the tissue sample to produce a cloned sequence and sequencing the cloned sequence, (g) identifying a mismatch between (i) a FANCD2 gene or a FANCD2 mRNA isolated from the tissue sample, and (ii) a nucleic acid probe complementary to the human wild-type FANCD2 gene sequence, when molecules (i) and (ii) are hybridized to each other to form a duplex, (h) amplification of FANCD2 gene sequences in the tissue sample and hybridization of the amplified sequences to nucleic acid probes which comprise wild-type FANCD2 gene sequences, (i) amplification of FANCD2 gene sequences in the tissue sample and hybridization of the amplified sequences to nucleic acid probes which comprise mutant FANCD2 gene sequences, (j) screening for a deletion mutation in the tissue sample, (k) screening for a point mutation in the tissue sample, (1) screening for an insertion mutation in the tissue sample, and (m) in situ hybridization of the FANCD2 gene of said tissue sample with nucleic acid probes which comprise the FANCD2 gene.

In an embodiment of the invention, a method is provided for diagnosing a susceptibility for cancer in a subject, includes:(a) accessing genetic material from the subject so as to determine defective DNA repair; (b) determining the presence of mutations in a set of genes, the set comprising FAND2 and at least one of FANCA, FANCB, FANCC, FANCD2, FANCDE, FANDF, FANDG, BRACA1 and ATM; and (c) diagnosing susceptibility for cancer from the presence of mutations in the set of genes.

In an embodiment of the invention, a method is provided for detecting a mutation in a neoplastic lesion at the FANCD2 gene in a human subject which includes:
comparing the sequence of the FANCD2 gene or the sequence of its mRNA in a tissue sample from a lesion of the subject with the sequence of the wild-type FANCD2 gene or the sequence of its mRNA, wherein an alteration in the sequence of the FANCD2 gene or the sequence of its mRNA of the subject indicates a mutation at the FANCD2 gene of the neoplastic lesion. A therapeutic protocol may be provided for treating the neoplastic lesion according to the mutation at the FANCD2 gene of the neoplastic lesion.

In an embodiment of the invention, a method is provided for confirming the lack of a FANCD2 mutation in a neoplastic lesion from a human subject which comprises comparing the sequence of the FANCD2 gene or the sequence of its mRNA in a tissue sample from a lesion of said subject with the sequence of the wild-type FANCD2 gene or the sequence of its RNA, wherein the presence of the wild-type sequence in the tissue sample indicates the lack of a mutation at the FANCD2 gene.

In an embodiment of the invention, a method is provided for determining a therapeutic protocol for a subject having a cancer, that includes (a) determining if a deficiency in FANCD2-L occurs in a cell sample from the subject by measuring FANCD2 isoforms using specific antibodies (b) if a deficiency is detected in (a), then determining whether the deficiency is a result of genetic defect in non-cancer cells; and (c) if (b) is positive, reducing the use of a therapeutic protocol that causes increased DNA damage so as to protect normal tissue in the subject and if (b) is negative, and the deficiency is contained within a genetic defect in cancer cells only, then increasing the use of a therapeutic protocol that causes increased DNA damage so as to adversely affect the cancer cells.

In an embodiment of the invention, a method of treating a FA pathway defect in a cell target is provided that includes: administering an effective amount of FANCD2 protein or an exogenous nucleic acid to the target. The FA pathway defect may be a defective FANCD2 gene and the exogenous nucleic acid vector may further include introducing a vector according to those described above. The vector may be selected from a mutant herpesvirus, a E1/E4 deleted recombinant adenovirus, a mutant retrovirus, the viral vector being defective in respect of a viral gene essential for production of infectious new virus particles. The vector may be contained in a lipid micelle.

In an embodiment of the invention, a method is provided for treating a patient with a defective PANCD2 gene, that includes providing a polypeptide described in SEQ ID No: 4, for functionally correcting a defect arising from a condition arising from the defective FANCD2 gene.

In an embodiment of the invention, a cell based assay for detecting a FA pathway defect is provided that includes obtaining a cell sample from a subject; exposing the cell sample to DNA damaging agents; and detecting whether FANCD2-L is upregulated, the absence of upregulation being indicative of the FA pathway defect. In the cell-based assay, amounts of FANCD2 may be measured by an analysis technique selected from: immunoblotting for detecting nuclear foci; Western blots to detect amounts of FANCD2 isoforms and quantifying mRNA by hybridising with DNA probes.

In an embodiment of the invention, a kit is provided for use in detecting a cancer cell in a biological sample, that includes (a) primer pair which binds under high stringency conditions to a sequence in the FANCD2 gene, the primer pair being selected to specifically amplify an altered nucleic acid sequence described in Table 7; and containers for each of the primers.

### Brief Description of the Figures

The foregoing features of the invention will be more readily understood by reference to the following detailed description, taken with reference to the accompanying drawings, in which:

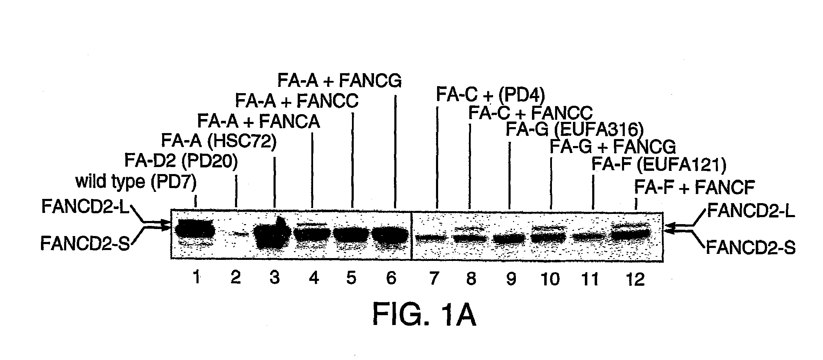

Figure 1A provides a Western blot demonstrating that the Fanconi Anemia protein complex is required for the monoubiquitination of FANCD2. Normal (WT) cells (lane 1) express two isoforms of the FANCD2 protein, a low molecular weight isoform (FANCD2-S) (155 kD) and a high molecular weight isoform (FANCD2-L) (162 kD). Lanes 3, 7, 9,11 show that FA cell lines derived from type A, C, G, and F patients only express the FANCD2-S isoform. Lanes 4,8,10,12 show the restoration of the high molecular weight isoform FANCD2-L following transfection of cell lines with corresponding FAcDNA.

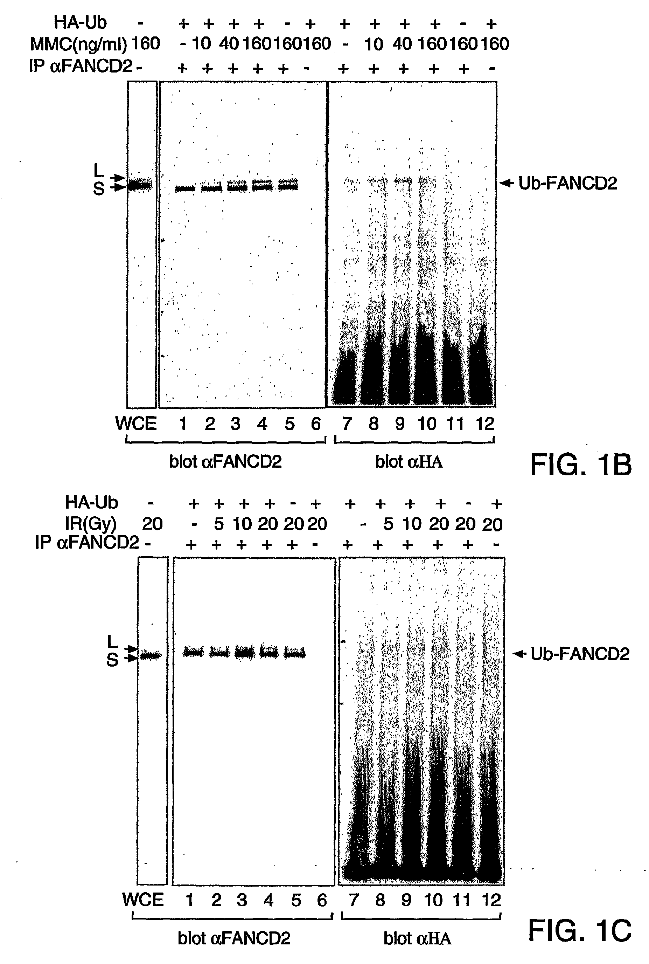

Figure 1B shows a Western blot obtained after HeLa cells were transfected with a cDNA encoding HA-ubiquitin. After transfection, cells were treated with the indicated dose of mitomycin C (MMC). Cellular proteins were immunoprecipitated with a polyclonal antibody (E35) to FANCD2, as indicated. FANCD2 was immunoprecipitated, and immune complexes were blotted with anti-FANCD2 or anti-HA monoclonal antibody.
Figure 1C shows a Western blot obtained after HeLa cells were transfected with a cDNA encoding HA-ubiquitin. After transfection, cells were treated with the indicated dose of ionizing radiation (IR). FANCD2 was immunoprecipitated, and immune complexes were blotted with anti-FANCD2 or anti-HA monoclonal antibody.
Figure ID shows a Western blot obtained after FA-G fibroblast line (FAG326SV) or corrected cells (FAG326SV plus FANCG cDNA) were transfected with the HA-Ub cDNA, FANCD2 was immunoprecipitated, and immune complexes were blotted with anti-FANCD2 or anti-HA antisera.

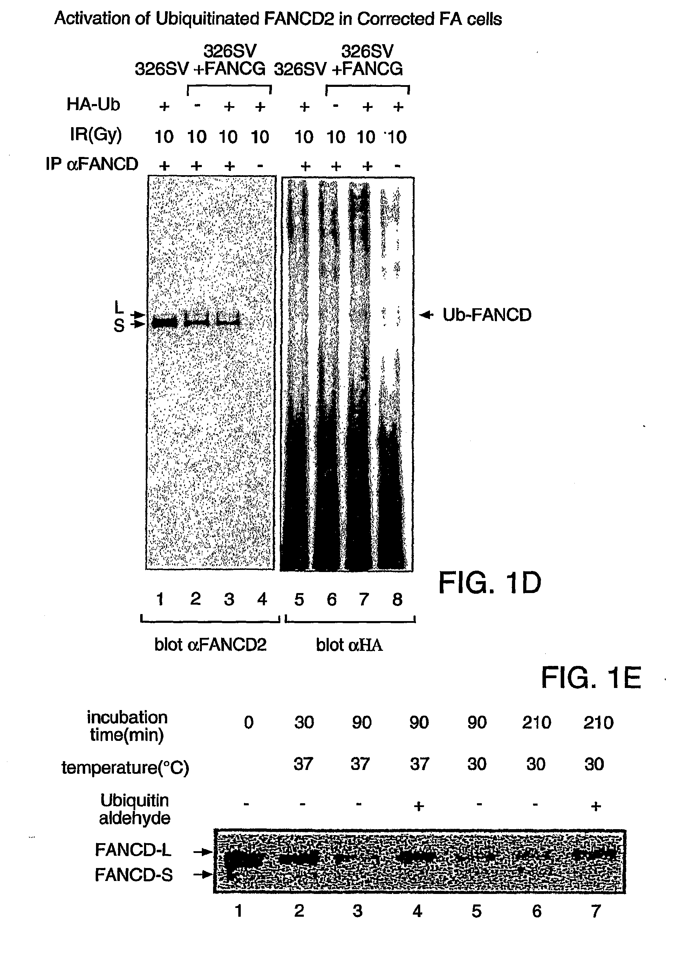

Figure 1E shows a Western blot obtained after treatment of Hela cells with 1mM hydroxyurea for 24hours. HeLa cell lysates were extracted and incubated at the indicated temperature for the indicated time period with or without 2.5µM ubiquitin aldehyde. The FANCD2 protein was detected by immunoblot with monoclonal anti-FANCD2 (F117).

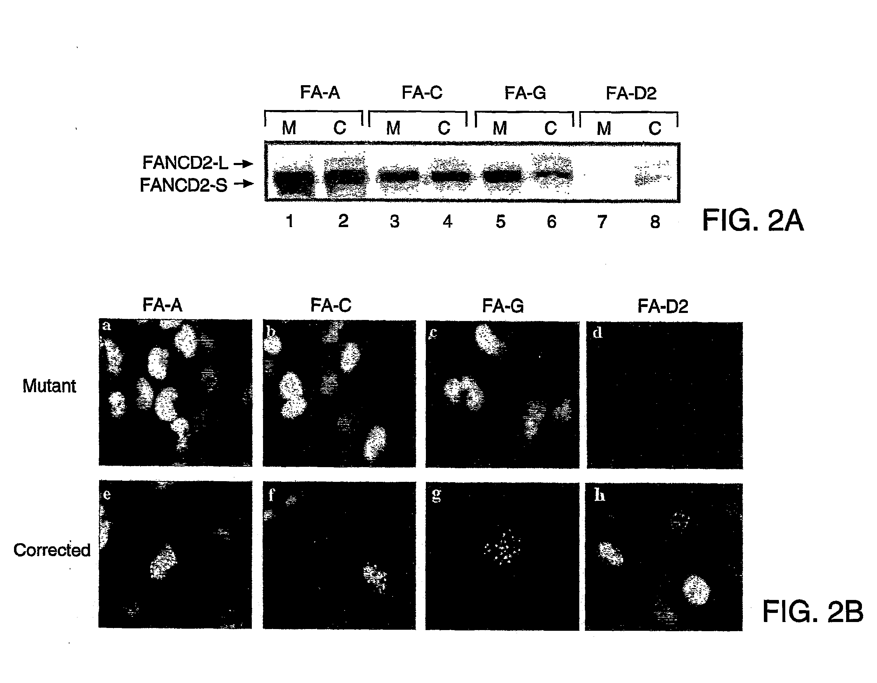

Figure 2 demonstrates that the Fanconi Anemia pathway is required for the formation of FANCD2 nuclear foci. Top panel shows anti-FANCD2 immunoblots of SV40 transformed fibroblasts prepared as whole cell extracts. Panels a-h show immunofluorescence with the affinity-purified anti-FANCD2 antiserum. The uncorrected (mutant, M) FA fibroblasts were FA-A (GM6914), FA-G (FAG326SV), FA-C (PD426), and FA-D (PD20P). The FA-A, FA-G, and FA-C fibroblasts were functionally complemented with the corresponding FA cDNA. The FA-D cells were complemented with neomycin-tagged human chromosome 3p (Whitney and al, 1995).

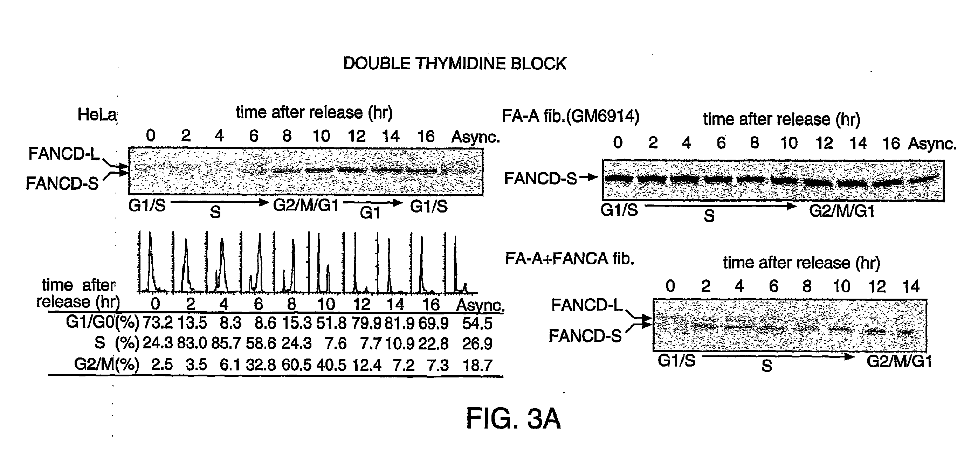

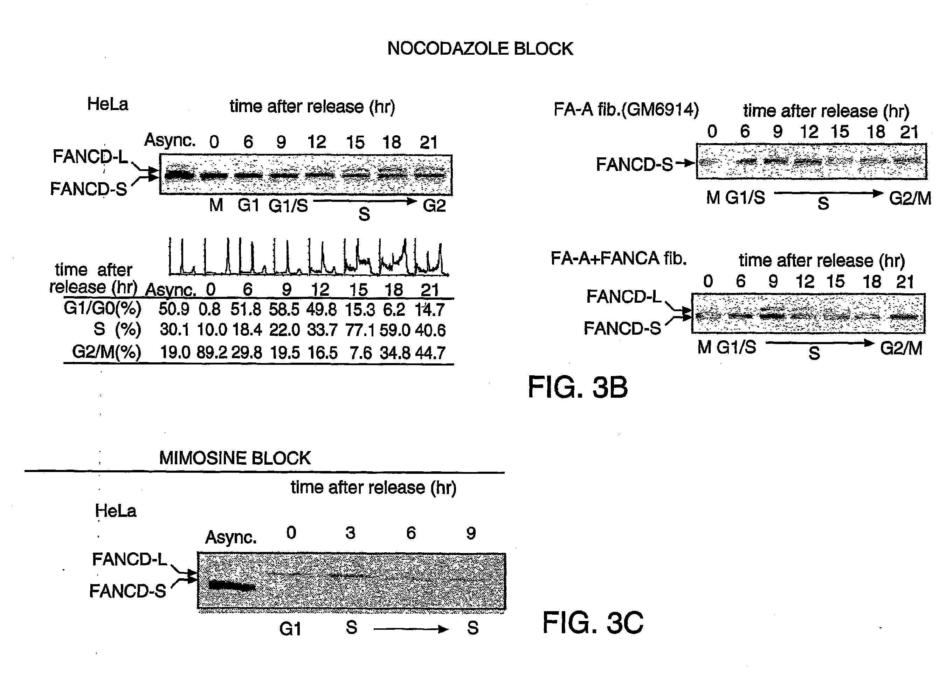

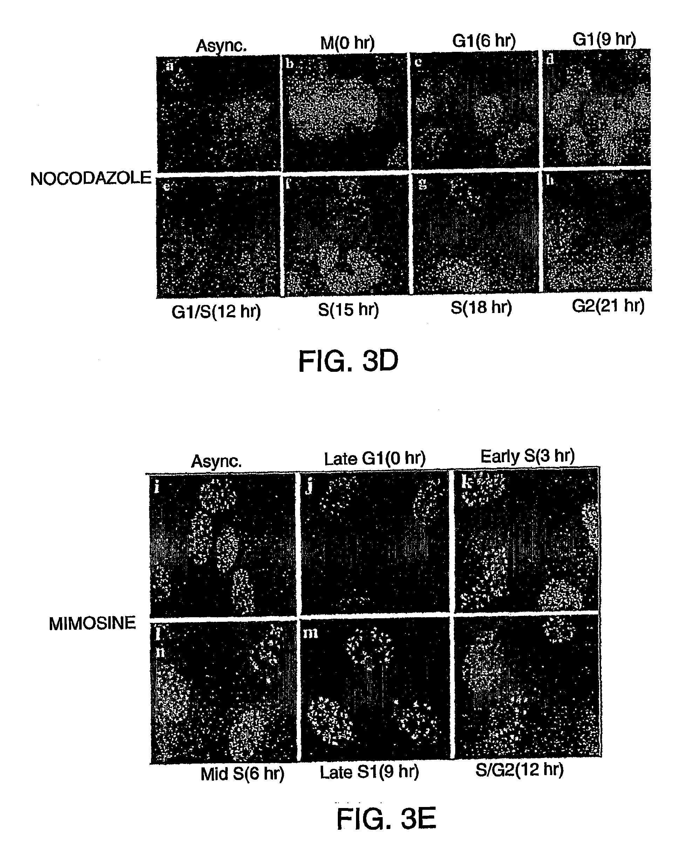

Figure 3 shows the cell cycle dependent expression of the two isoforms of the FANCD2 protein. (a) HeLa cells, SV40 transformed fibroblasts from an FA-A patient (GM6914), and GM6914 cells corrected with FANCA cDNA were synchronized by the double thymidine block method. Cells corresponding to the indicated phase of the cell cycle were lysed, and processed for FANCD2 immunoblotting (b) Synchrony by nocodazole block (c) Synchrony by mimosine block (d) HeLa cells were synchronized in the cell cycle using nocodozole or (e) mimosine, and cells corresponding to the indicated phase of the cell cycle were immunostained with the anti-PANCD2 antibody and analyzed by immunofluorescence.

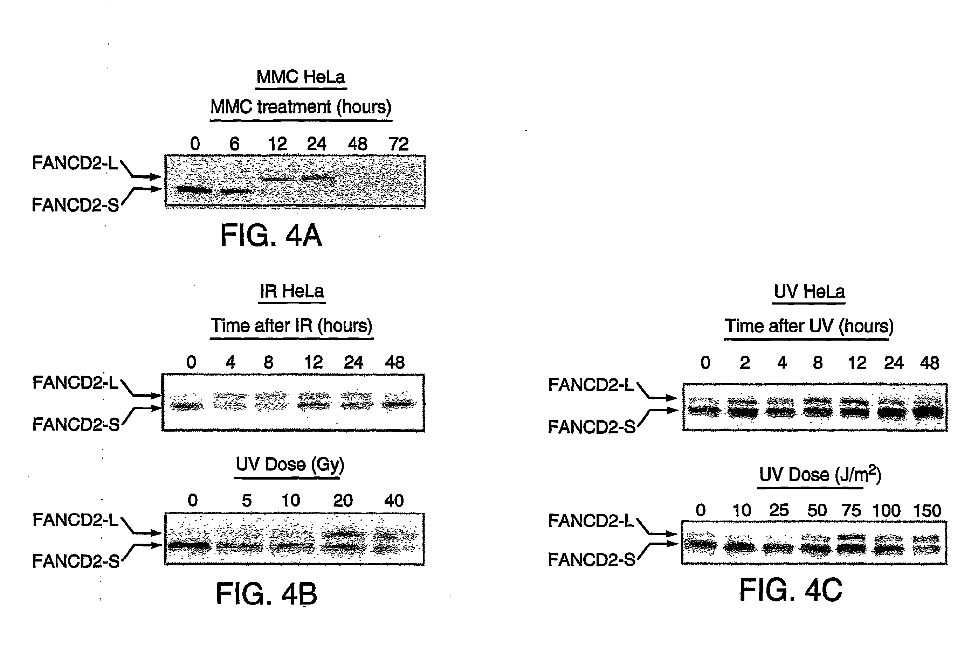

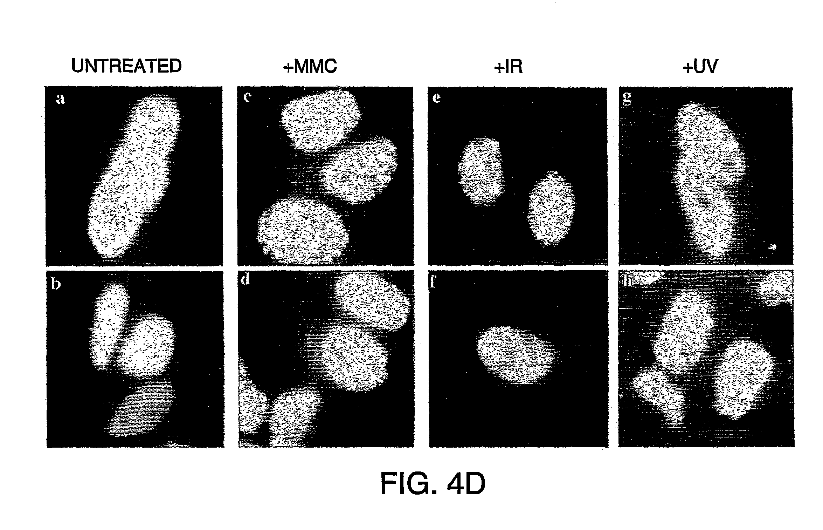

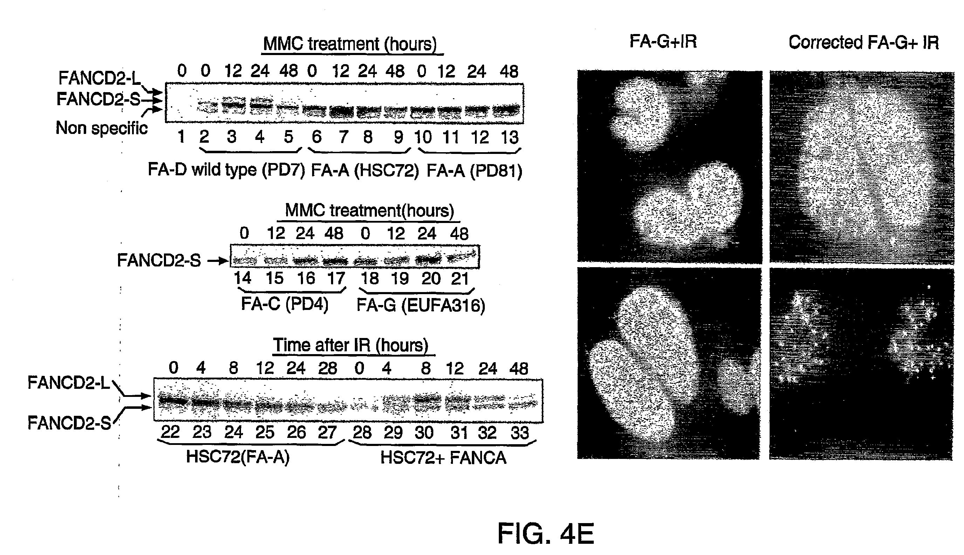

Figure 4. shows the formation of activated FANCD2 nuclear foci following cellular exposure to MMC, Ionizing Radiation, or Ultraviolet Light. Exponentially-growing HeLa cells were either untreated or exposed to the indicated DNA damaging agents, (a) Mitomycin C (MMC), (b) γ-irradiation (IR), or (c) Ultraviolet Light (UV), and processed for FANCD2 immunoblotting or FANCD2 immunostaining. (a) Cells were continuosly exposed to 40 ng/ml MMC for 0-72 hours as indicated, or treated for 24 hours and fixed for immunofluorescence. (b) and (c) Cells were exposed to γ-irradiation (10 Gy, B) or UV light (60 J/m² C) and collected after the indicated time (upper panels) or irradiated with the indicated doses and harvested one hour later (lower panels). For immunofluorescence analysis cells were fixed 8 hours after treatment (B, 10 Gy, C, 60 J/m²). (d) The indicated EBV-transformed lymphoblast lines from a normal individual (PD7) or from various Fanconi Anemia patients were either treated with 40ng/ml of Mitomycin C continuously (lanes 1-21) or exposed to 15 Gy of γ-irradiation (lanes 22-33) and processed for FANCD2 immunoblotting. The upregulation of FANCD-L after MMC or IR treatment was seen in PD7 (lanes 2-5) and in the corrected FA-A cells (lanes 28-33), but was not observed in any of the mutant Fanconi Anemia cell lines. Similarly, IR-induced FANCD2 nuclear foci were not detected in FA fibroblasts (FA-G + IR) but were restored after functional complementation (FA-G + FANCG).

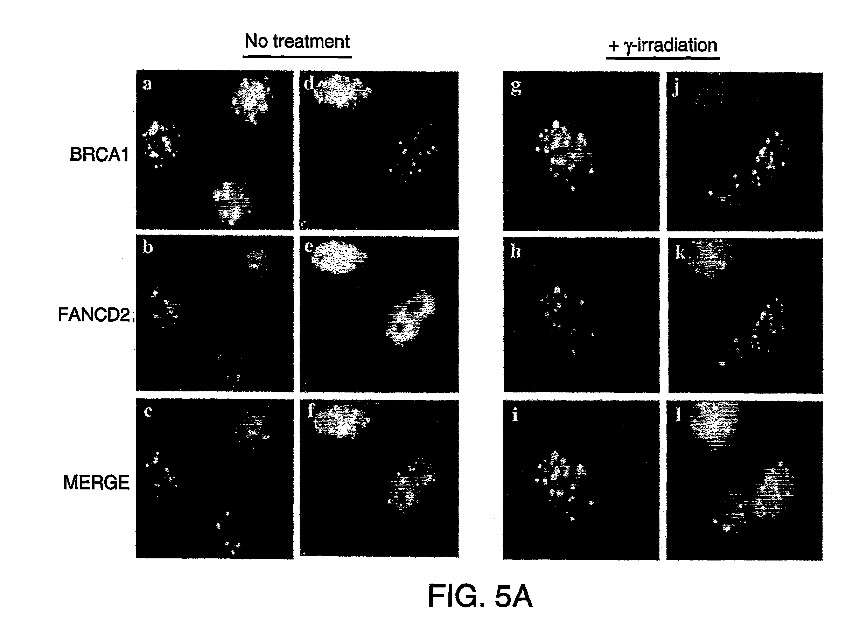

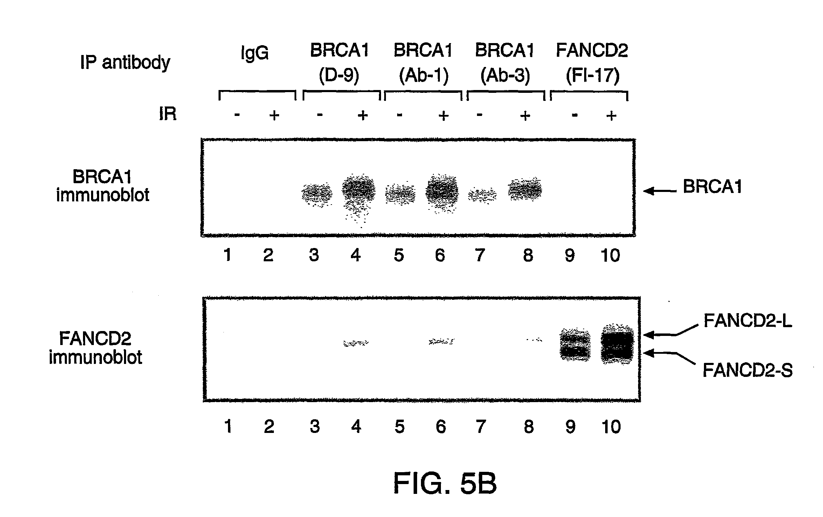

Figure 5 shows co-localization of activated FANCD2 and BRCA1 in Discrete Nuclear Foci following DNA damage. HeLa cells were untreated or exposed to Ionizing Radiation (10 Gy) as indicated, and fixed 8 hours later. (a) Cells were double-stained with the D-9 monoclonal anti-BRCA1 antibody (green, panels a, d, g, h) and the rabbit polyclonal anti-FANCD2 antibody (red, panels b, e, h, k), and stained cells were analyzed by immunofluorescence. Where green and red signals overlap (Merge, panels c, f, i,1) a yellow pattern is seen, indicating colocalization of BRCA1 and FANCD2. (b) Co-immunoprecipitation of FANCD2 and BRCA1. HeLa cells were untreated (-1R) or exposed to 15 Gy of γγ irradiation (+ IR) and collected 12 hours later. Cell lysates were prepared, and cellular proteins were immunoprecipitated with either the monoclonal FANCD2 antibody (FI-17, lanes 9-10), or any one of three independently-derived monoclonal antibodies to human BRCA1 (lanes 3-8): D-9 (Santa Cruz), Ab-1 and Ab-3 (Oncogene Research Products). The same amount of purified mouse IgG (Sigma) was used in control samples (lanes 1-2). Immune complexes were resolved by SDS-PAGE and were immunoblotted with anti-FANCD2 or anti-BRCA1 antisera. The FANCD-L isoform preferentially coimmunoprecipitated with BRCA1.

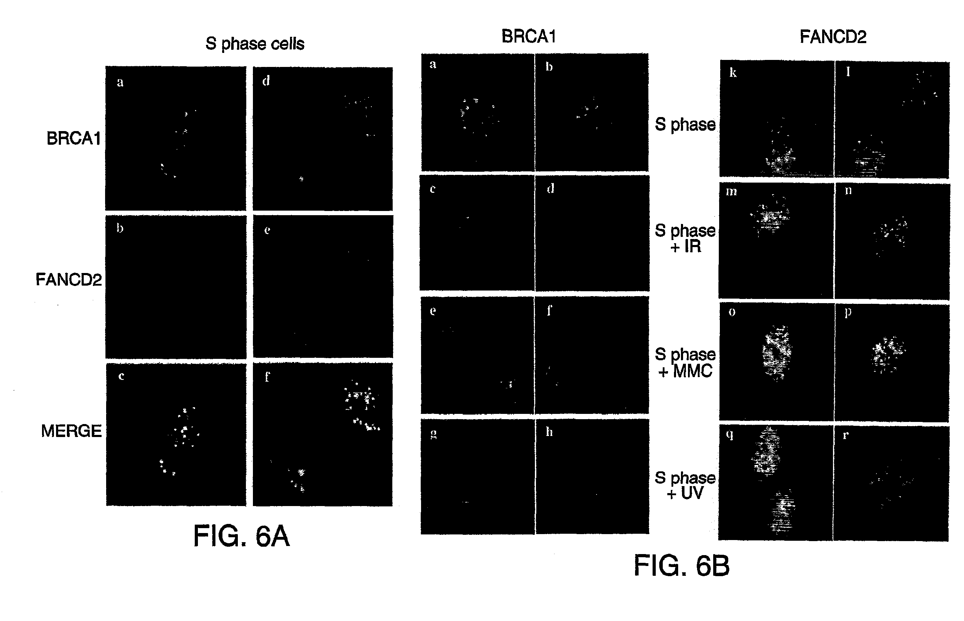

Figure 6 shows the co-localization of activated FANCD2 and BRCA1 in discrete nuclear foci during S phase. (a) HeLa cells were synchronized in late G1 with mimosine and released into S phase. S phase cells were double-stained with the monoclonal anti-BRCA1 antibody (green, panels a, d) and the rabbit polyclonal anti-FANCD2 antibody (red, panels b, e), and stained cells were analyzed by immunofluorescence. Where green and red signals overlap (merge, panels c, f), a yellow pattern is seen, indicating co-localization of BRCA1 and FANCD2. (b) HeLa cells synchronized in S phase were either untreated (a, b, k,1) or exposed to IR (50 Gy, panels c, d, m, n), MMC (20 µg/ml, panels e, f, o, p), or UV (100 j/m2, panels g, h, q, r) as indicated and fixed1 hour later. Cells were subsequently immunostained with an antibody specific for FANCD2 or BRCA1.

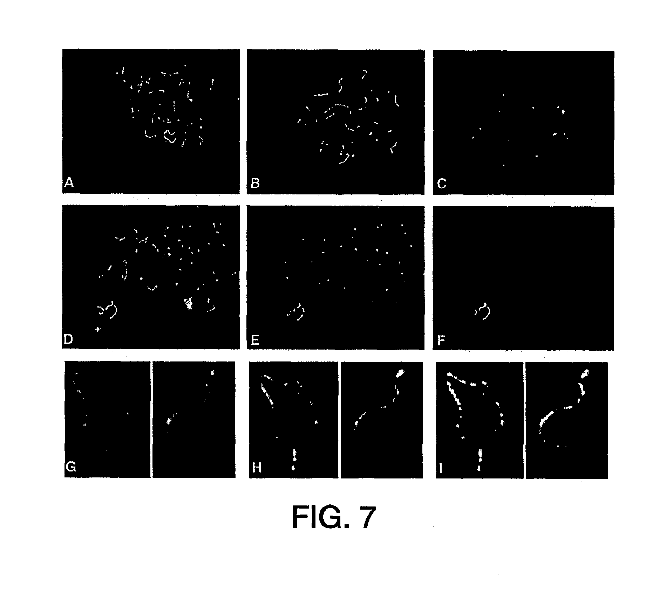

Figure 7 shows that FANCD2 forms foci on synaptonemal complexes that can co-localize with BRCA1 during meiosis I in mouse spermatocytes. (a) Anti-SCP3 (white) and anti-FANCD2 (red) staining of synaptonemal complexes in a late pachytene mouse nucleus. (b) SCP3 staining of late pachytene chromosomes. (c) Staining of this spread with preimmune serum for the anti-FANCD2 E35 antibody. (d) Anti-SCP3 staining of synaptonemal complexes in a mouse diplotene nucleus. (e) Costaining of this spread with E35 anti-FANCD2 antibody. Note staining of both the unpaired sex chromosomes and the telomeres of the autosomes with anti-FANCD2. (f) Costaining of this spread with anti-BRCA1 antibody. The sex chromosomes are preferentially stained. (g) Anti-FANCD2 staining of late pachytene sex chromosome synaptonemal complexes. (h) Anti-BRCA1 staining of the same complexes. (i) Anti-FANCD2 (red) and anti-BRCA1 (green) co-staining (co-localization reflected by yellow areas).

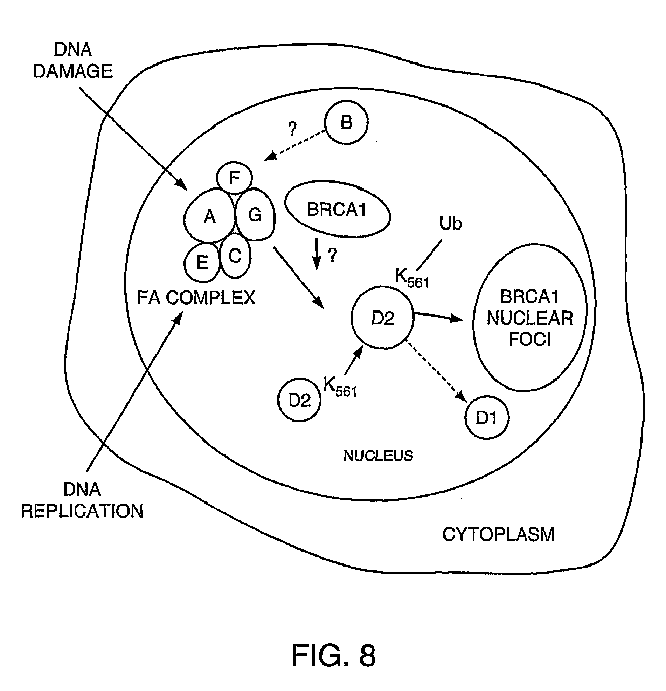

Figure 8 provides a schematic interaction of the FA proteins in a cellular pathway. The FA proteins (A, C, and G) bind in a functional nuclear complex. Upon activation of this complex, by either S phase entry or DNA damage, this complex enzymatically modifies (monoubiquitinates) the D protein. According to this model, the activated D protein is subsequently targeted to nuclear foci where it interacts with the BRCA1 protein and other proteins involved in DNA repair.

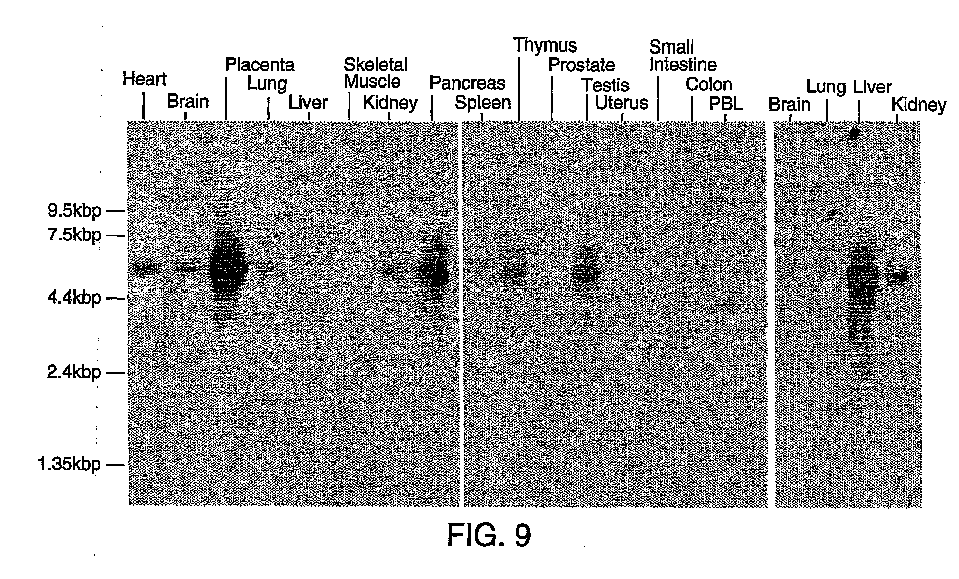

Figure 9 shows a Northern blot of cells from heart, brain, placenta, liver, skeletal muscle, kidney, pancreas, spleen, thymus, prostate, testis, uterus, small intestine, colon and peripheral blood lymphocytes from a human adult and brain, lung, liver and kidney from a human fetus probed with a full-length FANCD2 cDNA and exposed for 24 hours.

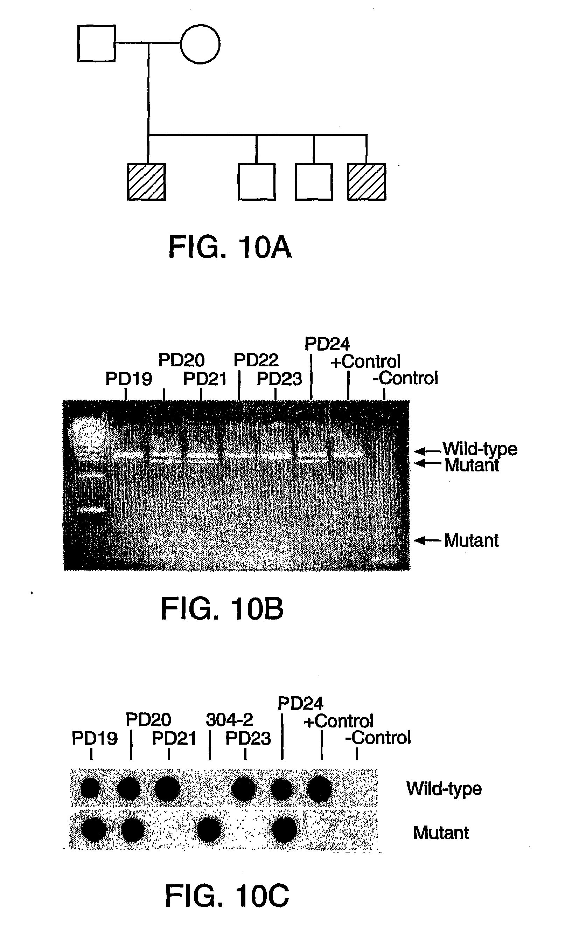

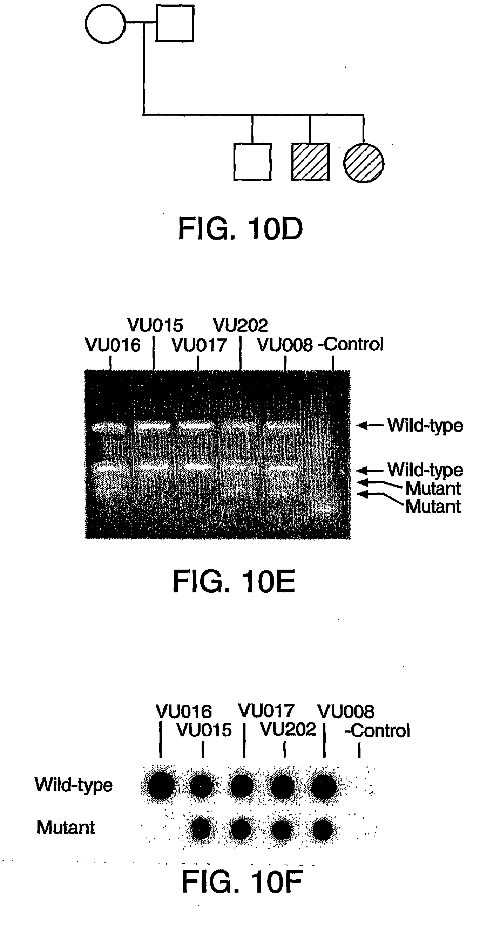

Figure 10 shows allele specific assays for mutation analysis of 2 FANCD2 families where the family pedigrees (a, d) and panels b, c, e and f are vertically aligned such that the corresponding mutation analysis is below the individual in question. Panels a-c depict the PD20 and panels d-f the VU008 family. Panels b and e show the segregation of the maternal mutations as detected by the creation of a new *Msp*I site (PD20) or *Dde*I site (VU008). The paternally inherited mutations in both families were detected with allele specific oligonucleotide hybridization (panels c and f).

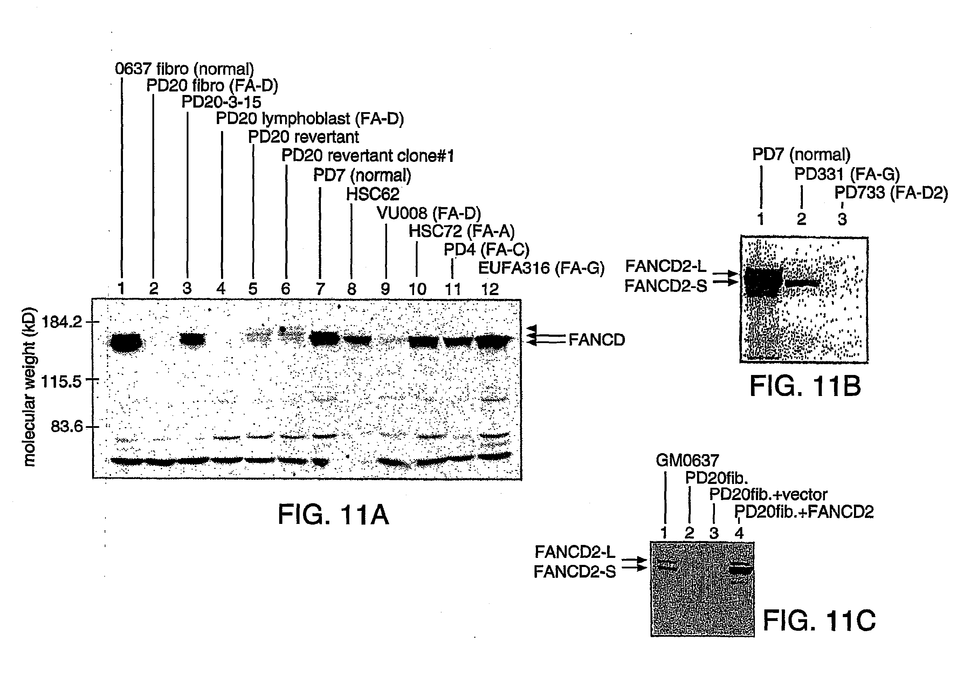

Figure 11 shows a Western blot analysis of the FANCD2 protein in human Fanconi Anemia cell lines. Whole cell lysates were generated from the indicated fibroblast and lymphoblast lines. Protein lysates (70 *µ*g) were probed directly by immunoblotting with the anti-FANCD2 antiserum. The FANCD2 proteins (155 kD and 162 kD) are indicated by arrows. Other bands in the immunoblot are non-specific. (a) Cell lines tested included wild-type cells (lanes 1,7), PD20 Fibroblasts (lane 2), PD20 lymphoblasts (lane 4), revertant MMC-resistant PD20 lymphoblasts (lane 5, 6), and chromosome 3p complemented PD20 fibroblasts (lane 3). Several other FA group D cell lines were analyzed including HSC62 (lane 8) and VU008 (lane 9). FA-A cells were HSC72 (lane 10), FA-C cells were PD4 (lane 11), and FA-G cells were EUFA316 (lane 12). (b) Identification of a third FANCD2 patient. FANCD2 protein was readily detectable in wild-type and FA group G cells but not in PD733 cells. (c) Specificity of the antibody. PD20i cells transduced with a retroviral FANCD2 expression vector displayed both isoforms of the FANCD2 protein (lane 4) in contrast to empty vector controls (lane 3) and untransfected PD20i cells (lane 2). In wild-type cells the endogenous FANCD2 protein (two isoforms) was also immunoreactive with the antibody (lane 1).

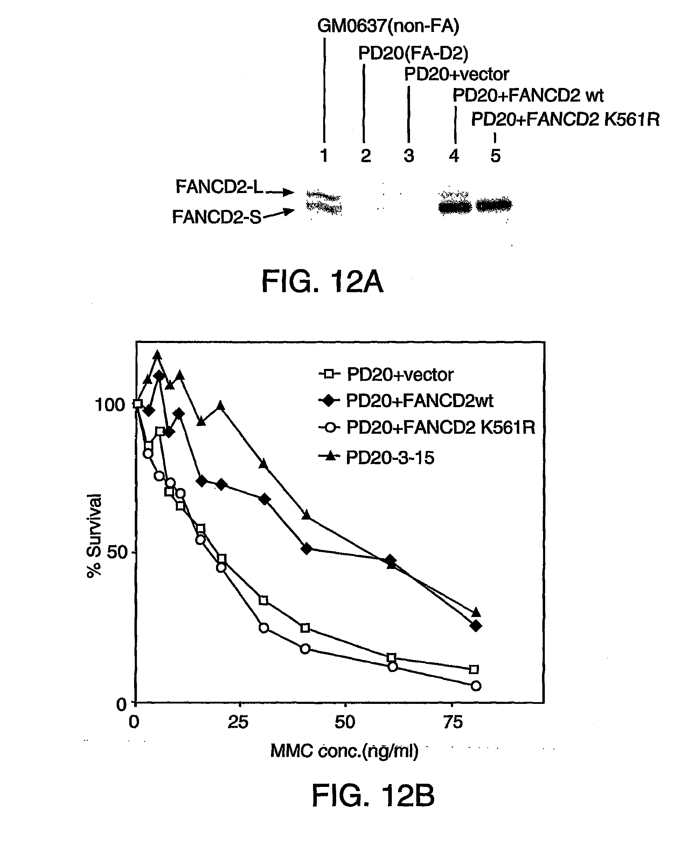

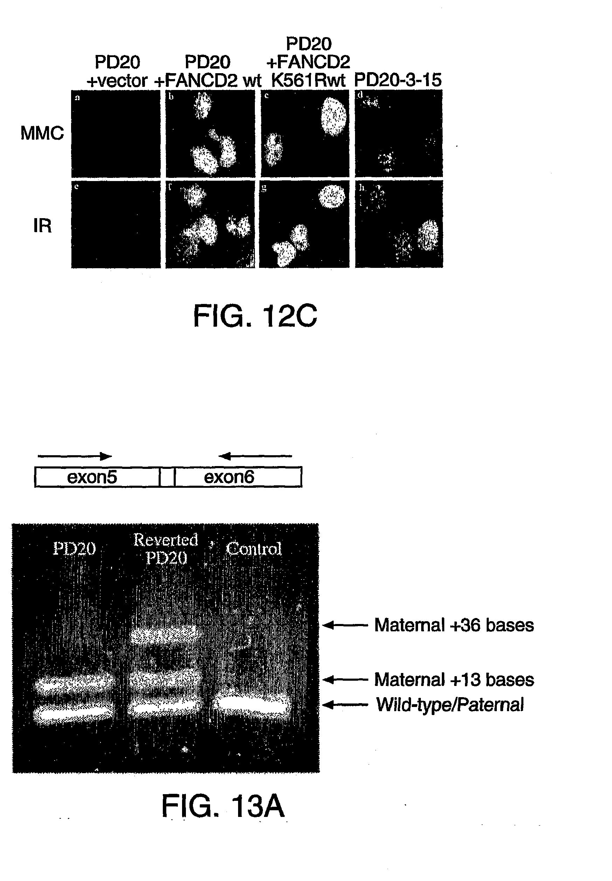

Figure 12 shows functional complementation of FA-D2 cells with the cloned FANCD2 cDNA. The SV40-transformed FA-D2 fibroblast line, PD20i, was transduced with pMMP-puro (PD20 + vector) or pMMP-FANCD2 (PD20 + FANCD2wt). Puromycin-selected cells were subjected to MMC sensitivity analysis. Cells analyzed were the parental PD20F cells (Δ), PD20 corrected with human chromosome 3p (o), and PD20 cells transduced with either pMMP-puro ( ) or pMMP-FANCD2(wt)-puro (◆).

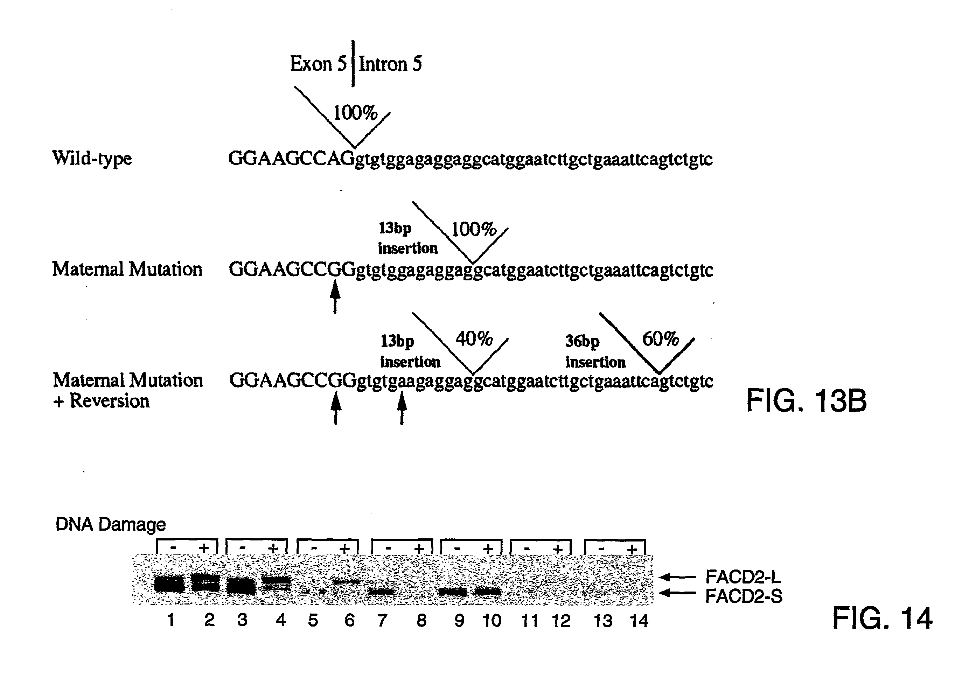

Figure 13 shows a molecular basis for the reversion of PD20 Lymphoblasts. (a) PCR primers to exons 5 and 6 were used to amplify cDNA. Control samples (right lane) yielded a single band of 114 bp, whereas PD20 cDNA (left lane) showed 2 bands, the larger reflecting the insertion of 13 bp of intronic sequence into the maternal allele. Reverted, MMC resistant lymphoblasts (middle lane) from PD20 revealed a third, in-frame splice variant of 114 + 36 bp (b) Schematic representation of splicing at the FANCD2 exon 5/intron 5 boundary. In wild-type cDNA 100% of splice events occur at the proper exon/intron boundary, whereas the maternal A->G mutation (indicated by arrow) leads to aberrant splicing, also in 100%. In the reverted cells all cDNAs with the maternal mutation also had a second sequence change (fat arrow) and showed a mixed splicing pattern with insertion of either 13 bp (~40% of mRNA) or 36 bp (~60% of mRNA).
Figure 14 shows an FANCD2 Western blot of cancer cell lines derived from patients with ovarian cancer.

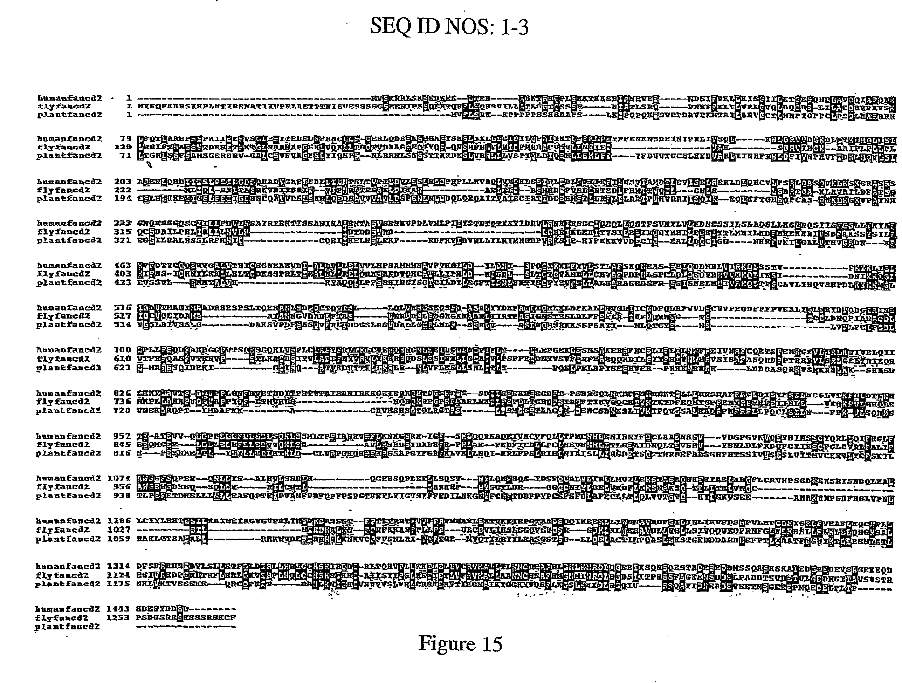

Figure 15 shows a sequence listing for amino acid sequence of human FANCD2 and alignment with fly and plant homologues using the BEAUTY algorithm (Worley, et al., 1995, Genome Res.Vol. 5, pp.173-184). (SEQ. ID. NO. 1-3) Black boxes indicate amino acid identity and gray similarity. The best alignment scores were observed with hypothetical proteins in D melanogaster (p=8.4 x 10⁻⁵⁸, accession number AAF55806) and A thaliana (p=9.4 x 10⁻⁴⁵, accession number B71413).

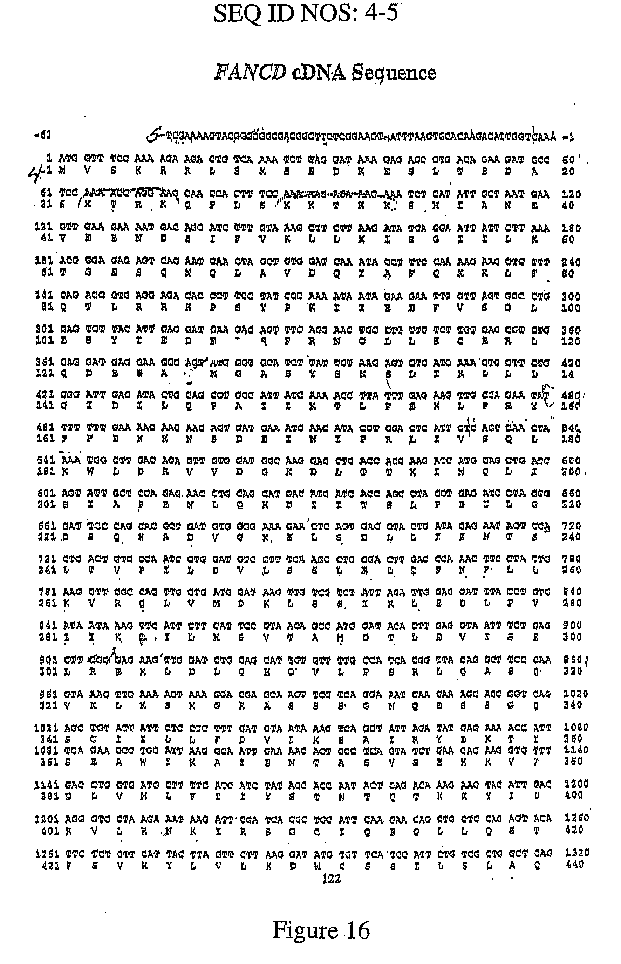

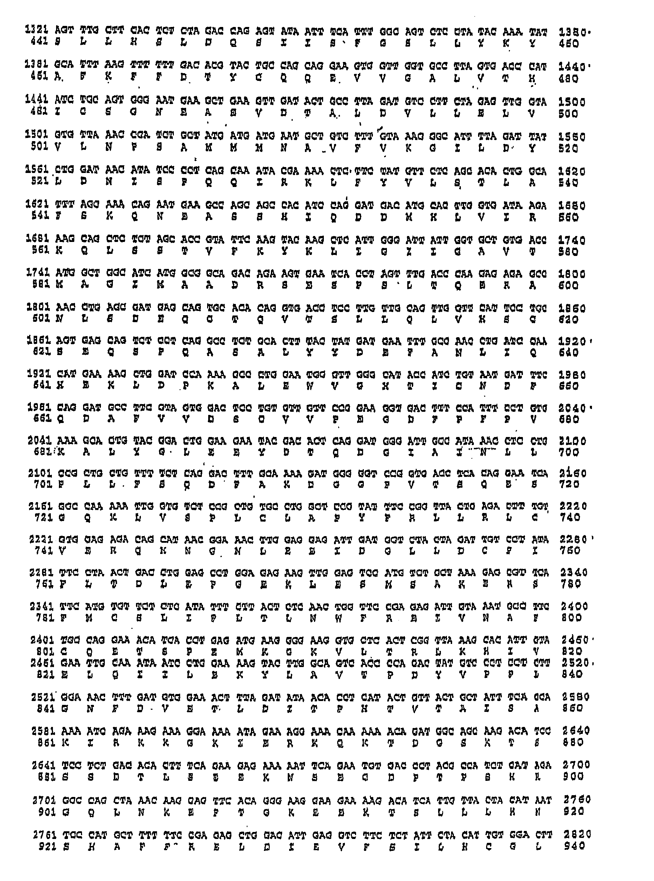

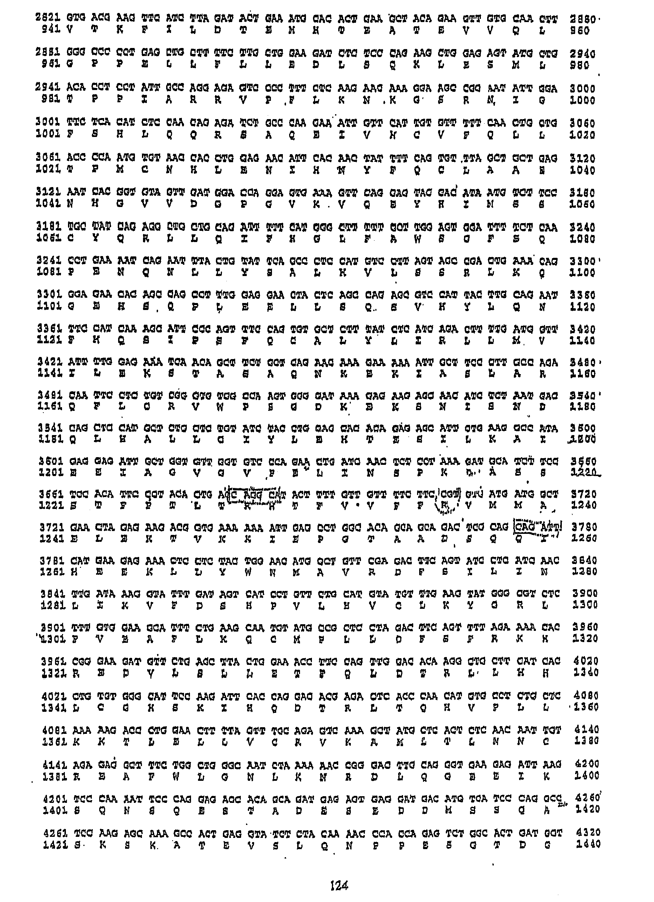

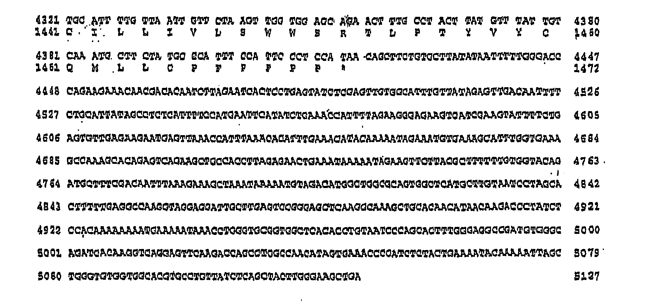

Figure 16 is the FANCD cDNA sequence -63 to 5127 nucleotides (SEQ ID NO: 5) and polypeptide encoded by this sequence from amino acid 1 to 1472 (SEQ ID NO: 4).

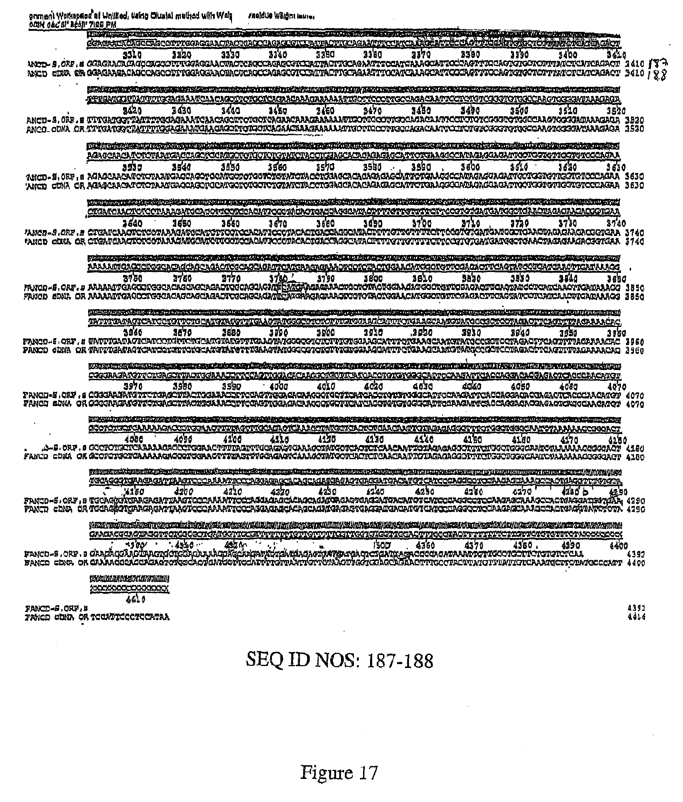

Figure 17 is the nucleotide sequence for FANCD-S.ORF (SEQ ID NO: 187) compared with FANCD cDNA (SEQ ID NO: 188).

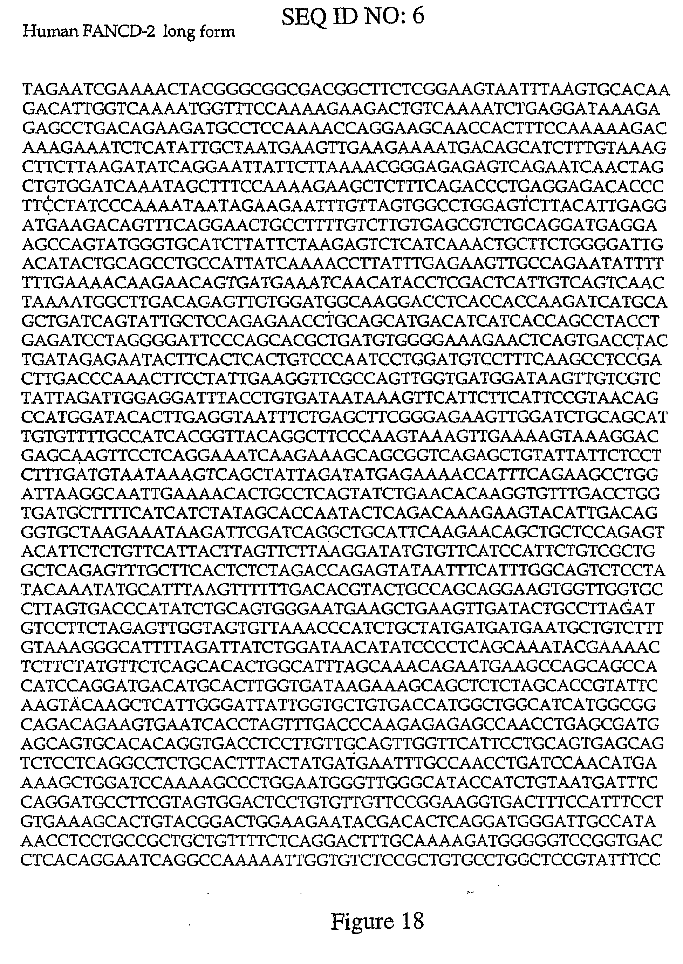

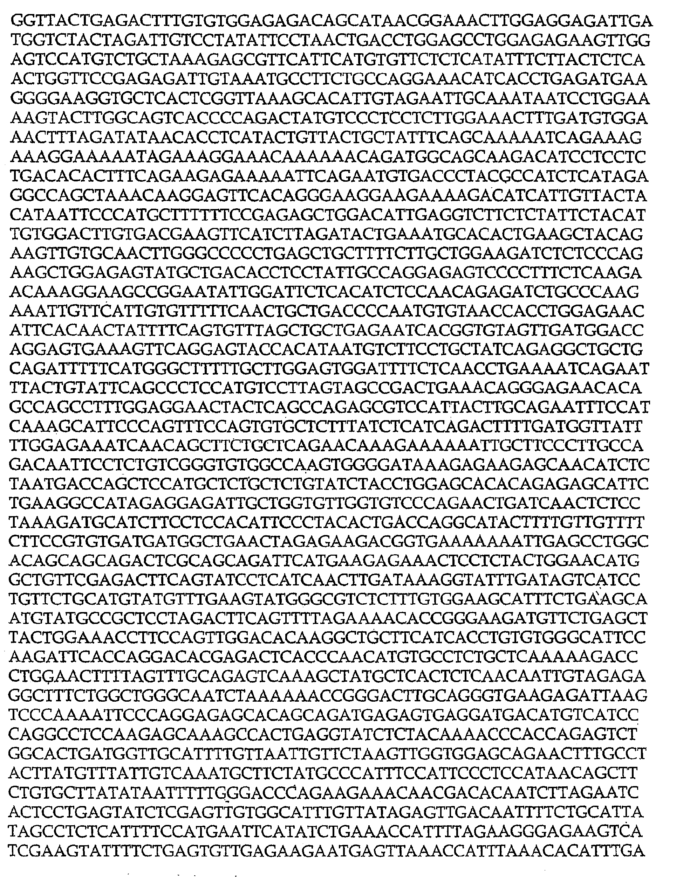

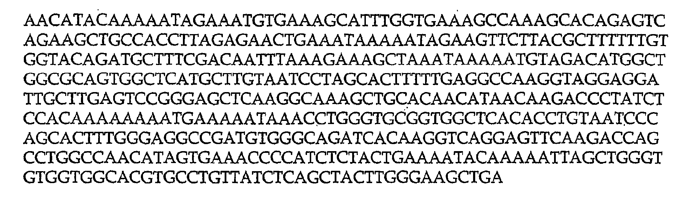

Figure 18 is the nucleotide sequence for human FANCD2-L (SEQ ID NO: 6).

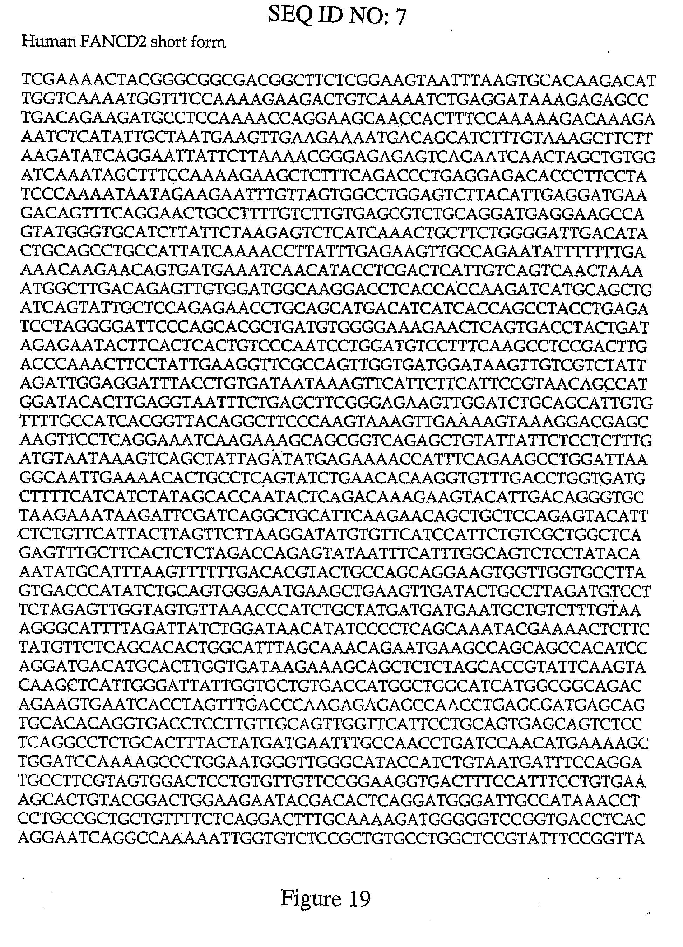

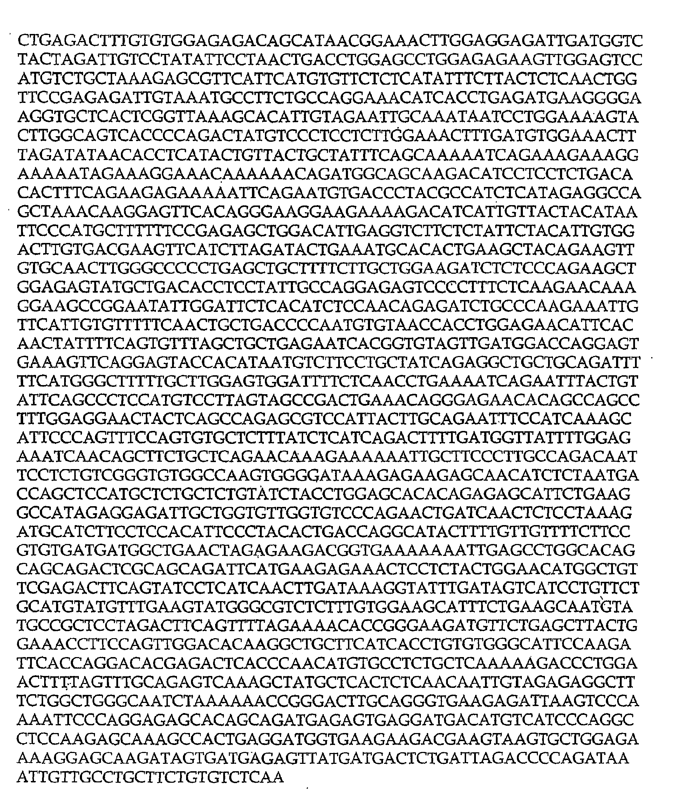

Figure 19 is the nucleotide sequence for human FANCD2-S (SEQ ID NO: 7).

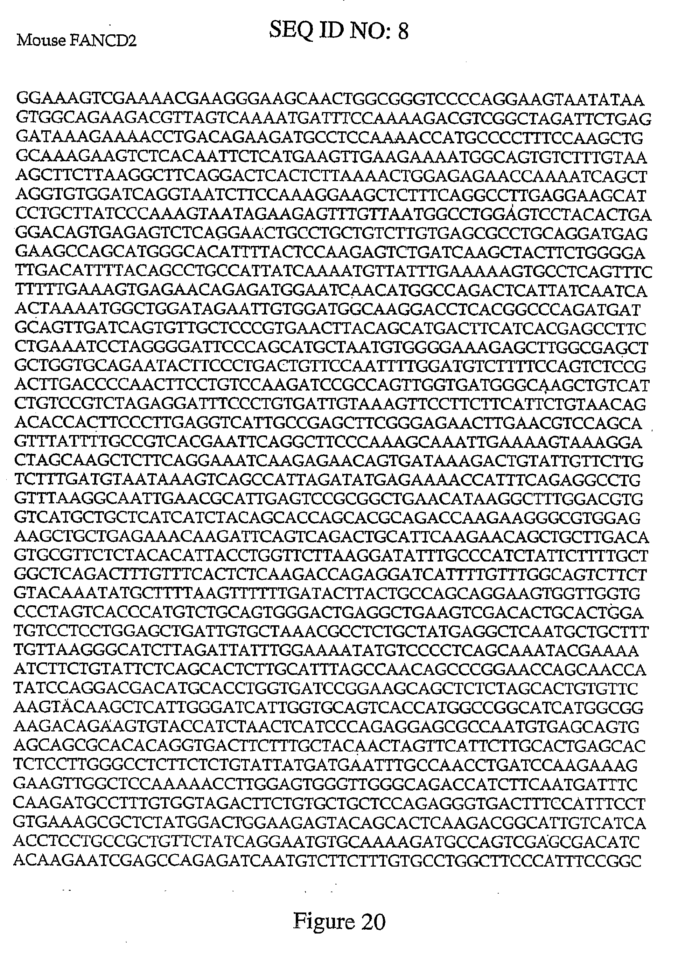

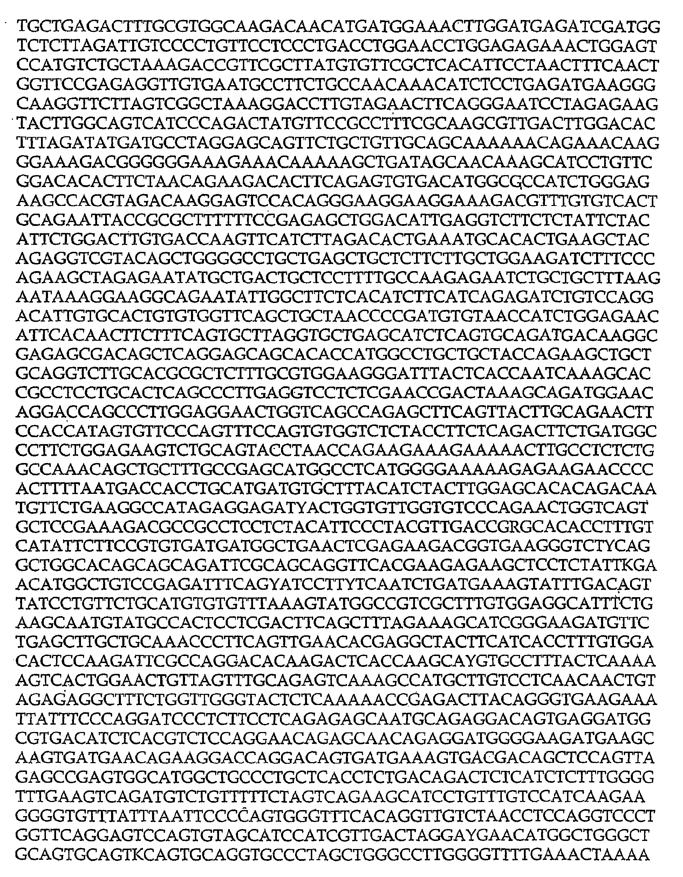

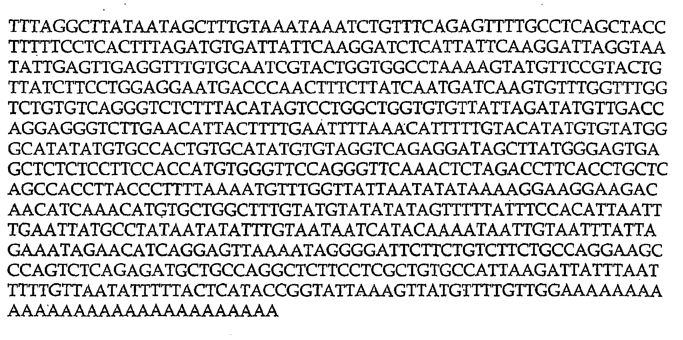

Figure 20 is the nucleotide sequence for mouse FANCD2 (SEQ 1D NO: 8).

### Detailed Description of Specific Embodiments

Definitions. As used in this description and the accompanying claims, the following terms shall have the meanings indicated, unless the context otherwise requires:
"FANCD2-L therapeutic agent" shall mean any of a protein isoform, and includes a peptide, a peptide derivative, analogue or isomer of the FANCD2-L protein and further include any of a small molecule derivative, analog, isomer or agonist that is functionally equivalent to FANCD2-L. Also included in the definition is a nucleic acid encoding FANCD2 which may be a full length or partial length gene sequence or cDNA or may be a gene activating nucleic acid or a nucleic acid binding molecule including an aptamer of antisense molecule which may act to modulate gene expression.
"Nucleic acid encoding FANCD-2" shall include the complete cDNA or genomic sequence of FANCD2 or portions thereof for expressing FANCD2-L protein as defined above. The nucleic acid may further be included in a nucleic acid carrier or vector and includes nucleic acid that has been suitably modified for effective delivery to the target site.
"Stringent conditions of hybridization" will generally include temperatures in excess of 30°C, typically in excess of 37°C, and preferably in excess of 45°C. Stringent salt conditions will ordinarily be less than 1000 mM, typically less than 500 mM, and preferably less than 200 mM.
"Substantial homology or similiarity" for a nucleic acid is when a nucleic acid or fragment thereof is "substantially homologous" ("or substantially similar") to another if, when optimally aligned (with appropriate nucleotide insertions or deletions) with the other nucleic acid (or its complementary strand), there is nucleotide sequence identity in at least about 60% of the nucleotide bases, usually at least about 70%, more usually at least about 80.
"Antibodies" includes polyclonal and/or monoclonal antibodies and fragments thereof including single chain antibodies and including single chain antibodies and Fab fragments, and immunologic binding equivalents thereof, which have a binding specificity sufficient to differentiate isoforms of a protein. These antibodies will be useful in assays as well as pharmaceuticals.
"Isolated" is used to describe a protein, polypeptide or nucleic acid which has been separated from components which accompany it in its natural state. An "isolated" protein or nucleic acid is substantially pure when at least about 60 to 75% of a sample exhibits a single aminoacid or nucleotide sequence.
"Regulatory sequences" refers to those sequences normally within 100 kb of the coding region of a locus, but they may also be more distant from the coding region, which affect the expression of the gene (including transcription of the gene, and translation, splicing, stability or the like of the messenger RNA).
"Polynucleotide" includes RNA, cDNA, genomic DNA, synthetic forms, and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, internucleotide modifications such as uncharged linkages (e.g., methyl phosphonates, phosphotriesters, phosphoamidates, carbamates, etc.), charged linkages (e.g., phosphorothioates, phosphorodithioates, etc.), pendent moieties (e.g., polypeptides), and modified linkages (e.g., alpha anomeric nucleic acids, etc.). Also included are synthetic molecules that mimic nucleic acids in their ability to bind to a designated sequence via hydrogen bonding and other chemical interactions.
"Mutation" is a change in nucleotide sequence within a gene, or outside the gene in a regulatory sequence compared to wild type. The change may be a deletion, substitution, point mutation, mutation of multiple nucleotides, transposition, inversion, frame shift, nonsense mutation or other forms of abberration that differentiate the nucleic acid or protein sequence from that of a normally expressed gene in a functional cell where expression and functionality are within the normally occurring range.
"Subject" refers to an animal including mammal, including human.
"Wild type FANCD2" refers to a gene that encodes a protein or an expressed protein capable of being monoubquinated to form FANCD2-L from FANCD-S within a cell.

We have found that some Fanconi Anemia has similarities with a group of syndromes including ataxia telangiectasia (AT), Xeroderma pigmentosum (XP), Cockayne syndrome (CS), Bloom's syndrome, myelodysplastic syndrome, aplastic anemia, cancer susceptibility syndromes and HNPCC (see Table 2). These syndromes have an underlying defect in DNA repair and are associated with defects in maintenance of chromosomal integrity. Defects in pathways associated with DNA repair and maintenance of chromosomal integrity result in genomic instability, and cellular sensitivity to DNA damaging agents such as bifunctional alkylating agents that cause intrastrand crosslinking. Moreover, deficiencies in DNA repair mechanisms appear to substantially increase the probability of initiating a range of cancers through genetic rearrangements. This observation is pertinent with regard to the clinical use of DNA cross-linking drugs including mitomycin C, cisplatin, cyclophosphamide, psoralen and UVA irradiation.

Although Fanconi Anemia is a rare disease, the pleiotropic effects of FA indicate the importance of the wild type function of FA proteins in the pathway for diverse cellular processes including genome stability, apoptosis, cell cycle control and resistance to DNA crosslinks. The cellular abnormalities in FA include sensitivity to cross-linking agents, prolongation of G2 phase of cell cycle, sensitivity to oxygen including poor growth at ambient O₂, overproduction of O₂ radicals, deficient O₂ radical defense, deficiency in superoxide dismutase; sensitivity to ionizing radiation (G2 specific); overproduction of tumor necrosis factor, direct defects in DNA repair including accumulations of DNA adducts, and defects in repair of DNA cross-links, genomic instability including spontaneous chromosome breakage, and hypermutability by deletion mechanism, increased aptosis, defective p53 induction, intrinsic stem cell defect, including decreased colony growth *in vitro;* and decreased gonadal stem cell survival.

These features are reflective of the involvement of FA in maintenance of hematopoietic and gonadal stem cells, as well as the normal embryonic development of many different structures, including the skeleton and urogenital systems. Cell samples from patients were analyzed to determine defects in the FA complementation group D. Lymphoblasts from one patient gave rise to the PD20 cell line which was found to be mutated in a different gene from HSC 62 derived from another patient with a defect in the D complementation group Mutations from both patients mapped to the D complementation group but to different genes hence the naming of two FANCD proteins-FANCD1 (HSC 62) and FANCD2 (PD20) (Timmers et al., (2001) Molecular Cell, Vol. 7, pp. 241-248).. We have shown that FANCD2 is the endpoint of the FA pathway and is not part of the FA nuclear complex nor required for its assembly or stability and that FANCD2 exists in two isoforms, FANCD2-S and FANCD2-L. We have also shown that transformation of the protein short form (FAND2-S) to the protein long form (FANCD2-L) occurs in response to the FA complex (Fig. 8). Defects in particular proteins associated with the FA pathway result in failure to make an important post translationally modified form of FANCD2 identified as FANCD2-L. The two isoforms of FANCD2 are identified as the short form and the long form.

Failure to make FANCD2-L correlates with errors in DNA repair and cell cycle abnormalities associated with diseases listed above.

To understand more about the role of FANCD2 in the aforementioned syndromes, we cloned the FANCD2 gene and determined the protein sequence. The FANCD2 gene has an open reading frame of 4,353 base pairs and forty four exons which encodes a novel 1451 amino acid nuclear protein, with a predicted molecular weight of 166kD. Western blot analysis revealed the existence of 2 protein isoforms of 162 and 155 kD. The sequence corresponding to the 44 Intron/Exon Junctions are provided in Table 6 (SEQ ID NO: 9-94).

Unlike previously cloned FA proteins, FANCD2 proteins from several non-vertebrate eurkaryotes showed highly significant alignment scores with proteins in *D. melanogaster, A. thaliana, and C. elegans.* The drosophila homologue, has 28% amino acid identity and 50% similarity to FANCD2 (Fig. and SEQ ID NO: 1-3) and no functional studies have been carried out in the respective species. No proteins similar to FANCD2 were found in *E. coli* or S. *cerevisiae.*

We obtained the FANCD2 DNA sequence (SEQ ID No: 5) by analyzing the chromosome 3p locus in PD20 and VU008, two FA cell lines having biallelic mutations in the FANCD2 gene (Fig. 10). The cell lines were assigned as complementation group D because lymphoblasts from the patients failed to complement HSC62, the reference cell line for group D. FANCD2 mutations were not detected in this group D reference cell line which indicates that the gene mutated in HSC62 is the gene encoding FANCD1 and in PD20 and VU008 is FANCD2 (Fig. 11). Microcell mediated chromosome transfer was used to identify the mutations (Whitney et al. Blood (1995) Vol. 88, 49-58). Detailed analysis of five microcell hybrids containing small overlapping deletions encompassing the locus narrowed the candidate region of the FANCD2 gene to 200Kb. The FANCD2 gene was isolated as follows: Three candidate ESTs were localized in or near this *FANCD2* critical region. Using 5' and 3' RACE to obtain full-length cDNAs, the genes were sequenced, and the expression pattern of each was analyzed by northern blot. EST SGC34603 had ubiquitous and low level expression of a 5kb and 7 kb mRNA similar to previously cloned FA genes. Open reading frames were found for TIGR-A004X28, AA609512 and SGC34603 and were 234, 531 and 4413 bp in length respectively. All 3 were analyzed for mutations in PD20 cells by sequencing cloned RT-PCR products. Whereas no sequence changes were detected in TIGR-A004X28 and AA609512, five sequence changes were found in SGC34603. Next, we determined the structure of the SGC34603 gene by using cDNA sequencing primers on BAC 177N7 from the critical region.

Based on the genomic sequence information, PCR primer pairs were designed (Table 7), the exons containing putative mutations were amplified, and allele-specific assays were developed to screen the PD20 family as well as 568 control chromosomes. Three of the alleles were common polymorphisms; however, 2 changes were not found in the controls and thus represented potential mutations (Table 3). The first was a maternally inherited G change at nt 376. In addition to changing an amino acid (S126G), this alteration was associated with mis-splicing and insertion of 13 bp from intron 5 into the mRNA. 43/43 (100%) independently cloned RT-PCR products with the maternal mutation contained this insertion, whereas only 3 % (1/31) of control cDNA clones displayed mis-spliced mRNA. The 13 bp insertion generated a frame-shift and predicts a severely truncated protein only 180 amino acids in length. The second alteration was a paternally inherited missense change at position 1236 (R1236H). The segregation of the mutations in the PD20 core family is depicted in Figure 10. Because the SGC34603 gene of PD20 contained both a maternal and a paternal allele not present on 568 control chromosomes and because the maternal mutation was associated with mis-splicing in 100% of cDNAs analyzed, we concluded that SGC34603 is the *FANCD2* gene.

*The protein encoded by FANCD2 is absent in PD20:* To further confirm the identity of SGC34603 as FANCD2, an antibody was raised against the protein, and Western blot analysis was performed (Figure 11). The specificity of the antibody was shown by retroviral transduction and stable expression FANCD2 in PD20 cells (Figure 11c). In wild-type cells this antibody detected two bands (155 and 162kD) which we call FANCD2 -S and ―L (best seen in Figure 11c). FANCD2 protein levels were markedly diminished in all MMC-sensitive cell lines from patient PD20 (Figure 11a, lanes 2, 4) but present in all wild-type cell lines and FA cells from other complementation groups. Furthermore, PD20 cells corrected by microcell-mediated transfer of chromosome 3 also made normal amounts of protein (Fig. 11a, lane 3).

*Functional complementation of FA-D2 cells with the FANCD2 cDNA:* We next assessed the ability of the cloned FANCD2 cDNA to complement the MMC sensitivity of FA-D2 cells (Figure 12). The full length FANCD2 cDNA was subcloned into the retroviral expression vector, pMMP-puro, as previously described (Pulsipher, et al., (1998) Mol. Med., Vol. 4, pp. 468-479). The transduced PD-20 cells expressed both isoforms of the FANCD2 protein, FANCD2-S and FANCD2-L (Fig. 12c). Transduction of FA-D2 (PD20) cells with pMMP-FANCD2 corrected the MMC sensitivity of the cells. These results further show that the cloned FANCD2 cDNA encodes the FANCD2-S protein, which can be post-translationally-modified to the FANCD2-L isoform. This important modification is discussed in greater detail below.

*Analysis of a phenotypically reverted PD20 clone:* We next generated additional evidence demonstrating that the sequence variations in PD20 cells were not functionally neutral polymorphisms. Towards this end we performed a molecular analysis of a revertant lymphoblast clone (PD20-cl.1) from patient PD20 which was no longer sensitive to MMC. Phenotypic reversion and somatic mosaicism are frequent findings in FA and have been associated with intragenic events such as mitotic recombination or compensatory frame-shifts. Indeed, ~60% of maternally derived SGC34603 cDNAs had a novel splice variant inserting 36 bp of intron 5 sequence rather than the usually observed 13 bp (Figure 13). The appearance of this in-frame splice variant correlated with a de novo base change at position IVS5+6 from G to A (Figure 13) and restoration of the correct reading frame was confirmed by Western blot analysis. In contrast to all MMC sensitive fibroblasts and lymphoblasts from patient PD20, PD20-cl.1 produced readily detectable amounts of FANCD2 protein of slightly higher molecular weight than the normal protein.

*Analysis of cell lines from other "FANCD" patients:* The antibody was also used to screen additional FA patient cell lines, including the reference cell line for FA group D, HSC and 2 other cell lines identified as group D by the European Fanconi Anemia Registry (EUFAR). VU008 did not express the FANCD2 protein and was found to be a compound heterozygote, with a missense and nonsense mutation, both in exon 12, and not found on 370 control chromosomes (Table 3, Figure 11). The missense mutation appears to destabilize the FANCD2 protein, as there is no detectable FANCD2 protein in lysates from VU008 cells. A third patient PD733 also lacked FANCD2 protein (Figure 11b, lane 3) and a splice mutation leading to absence of exon 17 and an internal deletion of the protein was found. The correlation of the mutations with the absence of FANCD2 protein in cell lysates derived from these patients substantiates the identity of *FANCD2* as a FA gene. In contrast, readily detectable amounts of both isoforms of the FANCD2 protein were found in HSC 62 (Fig.11a, lane 8) and VU423 cDNA and genomic DNA from both cell lines were extensively analyzed for mutations, and none were found. In addition, a whole cell fusion between VU423 and PD 20 fibroblasts showed complementation of the chromosome breakage phenotype (Table 5). Taken together these data show that FA group D are genetically heterogeneous and that the gene(s) defective in HSC 62 and VU423 are distinct from *FANCD2.*

The identification and sequencing of the FANCD2 gene and protein provides a novel target for therapeutic development, diagnostic tests and screening assays for diseases associated with failure of DNA repair and cell cycle abnormalities including but not limited to those listed in Table 2.

The following description provides novel and useful insights into the biological role of FANCD2 in the FA pathway which provides a basis for diagnosis and treatment of the aforementioned syndromes.

Evidence that FA cells have an underlying molecular defect in cell cycle regulation include the following: (a) FA cells display a cell cycle delay with 4N DNA content which is enhanced by treatment with chemical crosslinking agents, (b) the cell cycle arrest and reduced proliferation of FA cells can be partially corrected by overexpression of a protein, SPHAR, a member of the cyclin family of proteins and (c) caffeine abrogates the G2 arrest of FA cells. Consistent with these results, caffeine constitutively activates cdc2 and may override a normal G2 cell cycle checkpoint in FA cells. Finally, the FANCC protein binds to the cyclin dependent kinase, cdc2. We propose that the FA complex may be a substrate or modulator of the cyclinB/cdc2 complex.

Additionally, evidence that FA cells have an underlying defect in DNA repair is suggested by (a) FA cells that are sensitive to DNA cross-linking agents and ionizing radiation (IR), suggesting a specific defect in the repair of cross-linked DNA or double strand breaks, (b) DNA damage of FA cells which results in a hyperactive p53 response, suggesting the presence of defective repair yet intact checkpoint activities; and (c) FA cells with a defect in the fidelity of non-homologous end joining and an increased rate of homologous recombination (Garcia-Higuera et al. Mol. Cell. (2001), Vol. 7, pp. 249-262), (Grompe et al., Hum.Mol.Genet. (2001) Vol. 10, pp. 1-7).

Despite these general abnormalities in cell cycle and DNA repair. The mechanism by which FA pathway regulates these activities has remained elusive. Here we show that the FANCD2 protein functions downstream of the FA protein complex. In the presence of the assembled FA protein complex, the FANCD2 protein is activated to a high molecular weight, monoubiquitinated isoform which appears to modulate an S phase specific DNA repair response. The activated FANCD2 protein accumulates in nuclear foci in response to DNA damaging agents and co-localizes and co-immunoprecipitates with a known DNA repair protein, BRCA1. These results resolve previous conflicting models of the FA pathway (D'Andrea et al., 1997) and demonstrate that the FA proteins cooperate in a cellular response to DNA damage.

The FA pathway includes the formation of the FA multisubunit nuclear complex which in addition to A/C/G, we have shown also includes FANCF as a subunit of the complex (Figure 8). The FA pathway becomes "active" during the S phase to provide S-phase specific repair response or checkpoint response. The normal activation of the FA pathway which relies on the FA multisubunit complex results in the regulated monoubiquitination of the phosphoprotein-FANCD2 via a phosphorylation step to a high molecular weight activated isoform identified as FANCD-2L (Fig. 1). Monoubiquitination is associated with cell trafficking. FANCD2-L appears to modulate an S-phase specific DNA repair response (Fig. 3). The failure of FA cells to activate the S phase-specific activation of FANCD2 is associated with cell cycle specific abnormalities. The activated FANCD2 protein accumulates in nuclear foci in response to the DNA damaging agents, MMC and IR, and colocalizes and co-immunoprecipitates with a known DNA repair protein, BRCA1 (Fig. 4-6). These results resolve previous conflicting models of FA protein function (D'Andrea et. al.,1997) and strongly support a role of the FA pathway in DNA repair.

We have identified for the first time, an association between FANCD2 isoforms with respect to the FA pathway and proteins that are known diagnostic molecules for various cancers. A similar pathway with respect to DNA damage for the BRCA 1 protein which is activated to a high molecular weight, post-translationally-modified isoform in S phase or in response to DNA damage suggests that activated FANCD2 protein interacts with BRCA1. More particularly, the regulated monoubiquitination of FANCD2 appears to target the FANCD2 protein to nuclear foci containing BRCA1. FANCD2 co-immunoprecipitates with BRCA1, and may further bind with other "dot" proteins, such as RAD50, Mre11, NBS, or RAD51. Recent studies demonstrate that BRCA1 foci are composed of a large (2 Megadalton) multi-protein complex (Wang et al., (2000) Genes Dev., Vol. 14, pp. 927-939). This complex includes ATM, ATM substrates involved in DNA repair functions (BRCA1), and ATM substrates involved in checkpoint functions (NBS). It is further suggested that damage recognition and activation of the FA pathway involve kinases which respond to DNA damage including ATM, ATR, CHK1, or CHK2.

We have found that the DNA damaging reagents, IR and MMC, activate independent post-translational modifications of FANCD2 result in distinct functional consequences. IR activates the ATM ―dependent phosphorylation of FANCD2 at Serine 222 resulting in an S phase checkpoint response. MMC activates the BRACA-1 dependent and FA pathway dependent monouniquitination of FANCD2 at lysine 561, resulting in the assembly of FANCD2/BRCA1 nuclear foci and MMC resistance. FANCD2 therefore has two independent functional roles in the maintenance of chromosomal stability resulting from two discrete post-translational modifications provide a link between two additional cancer susceptibility genes (ATM and BRCA1) in a common pathway. Several additional lines of evidence support an interaction between FANCD2 and BRCA1. First, the BRCA1 (-/-) cell line, HCC1937 (Scully, et al., (1999) Mol. Cell, Vol. 4, pp. 1093-1099) has a "Fanconi Anemia-like" phenotype, with chromosome instability and increased tri-radial and tetra-radial chromosome formations. Second, although FA cells form BRCA1 foci (and RAD51 foci) normally in response to IR, BRCA1 (-/-) cells have no detectable BRCA1 foci and a greatly decreased number of FANCD2 foci compared to normal cells. Functional complementation of BRCA1 (-/-) cells restored BRCA1 foci and FANCD2 foci to normal levels, and restored normal MMC resistance.

The amount of FANCD2-L is determined in part by the amount of FAND2-S that is synthesized from *the fancd2* gene and in part by the availability of the FA complex to monoubiquinated FANCD2-S to form PANCD2-L. The association of FANCD2-L with nuclear foci including BRCA and ATM and determining the role of FANCD2-L in DNA repair make this protein a powerful target for looking at potential cancer development in patients for a wide range of cancers. Such cancers include those that arise through lesions on chromosome 3p as well as cancers on other chromosomes such that mutations result in interfering with production of upstream members of the FA pathway such as FANCG, FANCC or FANCA. Cancer lines and primary cells from cancer patients including tumor biopsies are being screened for FANCD-L and abnormal levels of this protein is expected to correlate with early diagnosis of disease. Because FANCD2 protein is a final step in a pathway to DNA repair, it is envisaged that any abnormality in a protein in the one or more pathways that lead to the conversion of FANCD2 -S to FANCD-L will be readily detected by measuring levels of FANCD2. Moreover, levels of FANCD2 affect how other proteins such as BRCA and ATM functionally interact in the nucleus with consequences for the patient. Analysis of levels of FANCD2 in a patient is expected to aid a physician in a clinical decision with respect to understanding the class of cancer presented by the patient. For instance, if a cancer cell fails to generate the monoubiquinated FANCD2-L isoform, the cell may have increased chromosome instability and perhaps increased sensitivity to irradiation or chemotherapeutic agents. This information will assist the physician in procedure improved treatment for the patient.

Fanconi Anemia is associated not only with a broad spectrum of different cancers but also with congenital abnormalities. Development of the fetus is a complex but orderly process. Certain proteins have a particularly broad spectrum of effects because they disrupt this orderly progression of development. The FA pathway plays a significant role in development and disruption of the FA pathway results in a multitude of adverse effects. Errors in the FA pathway are detectable through the analysis of the FAND2-L protein from fetal cells. FANCD2 represents a diagnostic marker for normal fetal development and a possible target for therapeutic intervention.

Consistent with the above, we have shown that FANCD2 plays a role in the production of viable sperm. FANCD2 forms foci on the unpaired axes of chromosomes XY bivalents in late pachytene and in diplotene murine spermatocytes. (Fig. 7) Interestingly, FANCD2 foci are also seen at the autosomal telomeres in diplonema. Taken together with the known fertility defects in FA patients and FA-C knockout mice, our observations suggest that activated FANCD2 protein is required for normal progression of spermatocytes through meiosis I. Most of the FANCD2 foci seen on the XY axes were found to colocalize with BRCA1 foci, suggesting that the two proteins may function together in meiotic cells. Like BRCA1, FANCD2 was detected on the axial (unsynapsed) elements of developing synaptonemal complexes. Since recombination occurs in synapsed regions, FANCD2 may function prior to the initiation of recombination, perhaps to help prepare chromosomes for synapsis or to regulate subsequent recombinational events. The relatively synchronous manner in which FANCD2 assembles on meiotic chromosomes, and forms dot structures in mitotic cells, suggests a role of FANCD2 in both mitotic and meiotic cell cycle control.

Embodiments of the invention are directed to the use of the post translationally modified isoform: FANCD-2L as a diagnostic target for determining the integrity of the FA pathway. Ubiquitination of FANCD2 and the formation of FANCD2 nuclear foci are downstream events in the FA pathway, requiring the function of several FA genes. We have found that biallelic mutations of any of the upstream FA genes (FANCA, FANCB, FANCC, FANCE, FANCF and FANCG) block the posttranslational modification of FANCD2 the unubiquitinated FANCD2 (PANCD2-S) form to the ubiquitinated (FANCD2-L). Any of these upstream defects can be overridden by transfecting cells with FANCD2 cDNA. (Fig. la)

We have demonstrated for the first time the existence of FANCD2 and its role in the FA pathway. We have shown that FANCD2 accumulates in nuclear foci in response to DNA damaging agents where it is associated with other DNA repair proteins such as BRCA 1 and ATM. We have also demonstrated that FANCD2 exists in two isoforms in cells where a reduction in one of the two isoforms, FANCD2- L is correlated with Fanconi Anemia and with increased cancer susceptibility. We have used these findings to propose a number of diagnostic tests for use in the clinic that will assist with patient care.

These tests include: (a) genetic and prenatal counseling for parents concerned about inherited Fanconi Anemia in a future offspring or in an existing pregnancy; (b) genetic counseling and immunodiagnostic tests for adult humans to determine increased susceptibility to a cancer correlated with a defective FA pathway; and (c) diagnosing an already existing cancer in a subject to provide an opportunity for developing treatment protocols that are maximally effective for the subject while minimizing side effects.

The diagnostic tests described herein rely on standard protocols known in the art for which we have provided novel reagents to test for FANCD2 proteins and nucleotide sequences. These reagents include antibodies specific for FANCD2 isoforms, nucleotide sequences from which vectors, probes and primers have been derived for detecting genetic alterations in the FANCD2 gene and cells lines and recombinant cells for preserving and testing defects in the FA pathway.

We have prepared monoclonal and polyclonal antibody preparations as described in Example 1 that are specific for FANCD2-L and FANCD2-S proteins. In addition, FANCD2 isoform specific antibody fragments and single chain antibodies may be prepared using standard techniques. We have used these antibodies in wet chemistry assays such as immunoprecipitation assays, for example Western blots, to identify FANCD2 isoforms in biological samples (Figure 1). Conventional immunoassays including enzyme linked immunosorbent assays (ELISA), radioimmune assays (RIA), immunoradiometric assays (IRMA) and immunoenzymatic assays (IEMA) and further including sandwich assays may also be used. Other immunoassays may utilize a sample of whole cells or lyzed cells that are reacted with antibody in solution and optionally analyzed in a liquid state within a reservoir. Isoforms of FANCD2 can be identified in *situ* in intact cells including cell lines, tissue biopsies and blood by immunological techniques using for example fluorescent activated cell sorting, and laser or light microscopy to detect immunofluorescent cells. (Figures 1-7, 9-14). For example, biopsies of tissues or cell monolayers, prepared on a slide in a preserved state such as embedded in paraffin or as frozen tissue sections can be exposed to antibody for detecting FANCD2-L and then examined by fluorescent microscopy.

In an embodiment of the invention, patient-derived cell lines or cancer cell lines are analyzed by immunoblotting and immunofluorescence to provide a novel simple diagnostic test for detecting altered amounts of FANCD2 isoforms. The diagnostic test also provides a means to screen for upstream defects in the FA pathway and a practical alternative to the currently employed DEB/MMC chromosome breakage test for FA because individuals with upstream defects in the FA pathway are unable to ubiquitinate FANCD2. Other assays may be used including assays that combine retroviral gene transfer to form transformed patient derived cell lines (Pulsipher et al. (1998). Mol Med 4, 468-79 together with FANCD2 immunoblotting to provide a rapid subtyping analysis of newly diagnosed patients with any of the syndromes described in Table 2, in particular, that of FA.

The above assays may be performed by diagnostic laboratories, or, alternatively, diagnostic kits may be manufactured and sold to health care providers or to private individuals for self-diagnosis. The results of these tests and interpretive information are useful for the healthcare provider in diagnosis and treatment of a patient's condition.

Genetic tests can provide for a subject, a rapid reliable risk analysis for a particular condition against an epidemiological baseline. Our data suggests that genetic heterogeneity occurs in patients with FA within the FANCD2 complementation group. We have found a correlation between genetic heterogeneity and disease as well as genetic heterogeneity and abnormal post-translational modifications that result in the presence or absence of FANCD2-L. This correlation provides the basis for prognostic tests as well as diagnostic tests and treatments for any of the syndromes characterized by abnormal DNA repair. For example, nucleic acid from a cell sample obtained from drawn blood or from other cells derived from a subject can be analyzed for mutations in the FANCD2 gene and the subject may be diagnosed to have an increased susceptibility to cancer

We have located the FANCD2 gene at 3p25.3 on chromosome 3p in a region which correlates to a high frequency of cancer. Cytogenetic and loss of heterozygosity (LOH) studies have demonstrated that deletions of chromosome 3p occur at a high frequency in all forms of lung cancer (Todd et al. Cancer Res. Vol. 57, pp. 1344-52). For example, homozygous deletions were found in three squamous cell lines within a region of 3p21. Homozygous deletions were also found in a small cell tumor at 3p12 and a 3p14.2. (Franklin et al. (1997) Cancer Res. 57:1344-52). The present mapping of FANCD2 is supportive of the theory that this chromosomal region contains important tumor suppressor genes. Further support for this has been provided by a recent publication of Sekine et al., Human Molecular Genetics, (2001) 10, pp. 1421-1429 who reported localization of a novel susceptibility gene for familial ovarian cancer to chromosome 3p22-p25. The reduction or absence of FANCD2-L is here proposed to be diagnostic for increased risk of tumors resulting from mutations not only at the FANCD2 site (3p25.3) but also at other sites in the chromosomes possibly arising from defects in DNA repair following cell damage arising from exposure to environmental agents and normal aging processes.

As more individuals and families are screened for genetic defects in the FANCD2 gene, a data base will be developed in which population frequencies for different mutations will be gathered and correlations made between these mutations and health profile for the individuals so that the predictive value of genetic analysis will continually improve. An example of an allele specific pedigree analysis for FANCD2 is provided in Figure 10 for two families.

Diagnosis of a mutation in the FANCD2 gene may initially be detected by a rapid immunological assay for detecting reduced amounts of FANCD2-L proteins. Positive samples may then be screened with available probes and primers for defects in any of the genes in the FA pathway. Where a defect in the FANCD2 gene is implicated, primers or probes such as provided in Table 7 may be used to detect a mutation. In those samples, where a mutation is not detected by such primers or probes, the entire FANCD2 gene may be sequenced to determine the presence and location of the mutation in the gene.

Nucleic acid screening assays for use in identifying a genetic defect in the FANCD2 gene locus may include PCR and non PCR based assays to detect mutations. There are many approaches to analyzing cell genomes for the presence of mutations in a particular allele. Alteration of a wild-type FANCD2 allele, whether, for example, by point mutation, deletion or insertions can be detected using standard methods employing probes. (US Patent 6,033,857). Standard methods include: (a) fluorescent *in situ* hybridization (FISH) which may be used on whole intact cells.; and (b) allele specific oligonucleotides (ASO) may be used to detect mutations using hybridization techniques on isolated nucleic acid (Conner et al., 1989, Hum.Genet. 85: 55-74). Other techniques include (a) observing shifts in electrophoretic mobility of single-stranded DNA on non-denaturing polyacrylamide gels, (b) hybridizing a FANCD2 gene probe to genomic DNA isolated from the tissue sample, (c) hybridizing an allele-specific probe to genomic DNA of the tissue sample, (d) amplifying all or part of the FANCD2 gene from the tissue sample to produce an amplified sequence and sequencing the amplified sequence, (e) amplifying all or part of the FANCD2 gene from the tissue sample using primers for a specific FANCD2 mutant allele, (f) molecular cloning all or part of the FANCD2 gene from the tissue sample to produce a cloned sequence and sequencing the cloned sequence, (g) identifying a mismatch between (i) a FANCD2 gene or a FANCD2 mRNA isolated from the tissue sample, and (ii) a nucleic acid probe complementary to the human wild-type FANCD2 gene sequence, when molecules (i) and (ii) are hybridized to each other to form a duplex, (h) amplification of FANCD2 gene sequences in the tissue sample and hybridization of the amplified sequences to nucleic acid probes which comprise wild-type FANCD2 gene sequences, (I) amplification of PANCD2 gene sequences in the tissue sample and hybridization of the amplified sequences to nucleic acid probes which comprise mutant FANCD2 gene sequences, (j) screening for a deletion mutation in the tissue sample, (k) screening for a point mutation in the tissue sample, (1) screening for an insertion mutation in the tissue sample, and (m) in situ hybridization of the FANCD2 gene of the tissue sample with nucleic acid probes which comprise the FANCD2 gene.

It is often desirable to scan a relatively short region of a gene or genome for point mutations: The large numbers of oligonucleotides needed to examine all potential sites in the sequence can be made by efficient combinatorial methods (Southern, E. M et al., (1994). Nucleic Acids Res. 22:,1368-1373). Arrays may be used in conjunction with ligase or polymerase to look for mutations at all sites in the target sequence.(US 6,307,039) Analysis of mutations by hybridization can be performed for example by means of gels, arrays or dot blots.

The entire gene may be sequenced to identify mutations. (US 6,033,857). Sequencing of the FANCD2 locus can be achieved using oligonucleotide tags from a minimally cross hybridizing set which become attached to their complements on solid phase supports when attached to target sequence (US 6,280,935).

Other approaches to detecting mutations in the FANCD2 gene include those described in US 6,297,010, US 6,287,772 and US 6,300,076). It is further contemplated that the assays may employ nucleic acid microchip technology or analysis of multiple samples using laboratories on chips.

A subject who has developed a tumor maybe screened using nucleic acid diagnostic tests or antibody based tests to detect a FANCD2 gene mutation or a deficiency in FANCD2-L protein. On the basis of such screening samples may be obtained from subjects having a wide range of cancers including melanoma, leukemia, astocytoma, glioblastoma, lymphoma, glioma, Hodgkins lymphoma, chronic lymphocyte leukemia and cancer of the pancreas, breast, thyroid, ovary, uterus, testis, pituitary, kidney, stomach, esophagus and rectum. The clinician has an improved ability to select a suitable treatment protocol for maximizing the treatment benefit for the patient. In particular, the presence of a genetic lesion or a deficiency in FANCD2-L protein may be correlated with responsiveness to various existing chemotherapeutic drugs and radiation therapies.

New therapeutic treatments may be developed by screening for molecules that modulate the monoubiquitination of FANCD2-S to give rise to FANCD2-L in cell assays (Examples 11-12) and in knock-out mouse models (Example 10). Such molecules may include those that bind directly to FANCD2 or to molecules such as BRACA-2 that appears to interact with BRACA-1 which in turn appears to be activated by FANCD2.

In addition, to screening assays that rely on defects in the FANCD2 gene or protein, an observed failure of the ubiquitination reaction that is necessary for the formation of FANCD2-L may result from a defect in the FA pathway at any point preceding the post translational modification of FANCD2 including FANCD2-S itself. Knowing the terminal step in the reactions, enables a screening assay to be formulated in which small molecules are screened in cells containing "broken FA pathway" or *in vitro* until a molecule is found to repair the broken pathway. This molecule can then be utilized as a probe to identify the nature of the defect. It may further be used as a therapeutic agent to repair the defect. For example, we have shown that cell cycle arrest and reduced proliferation of FA cells can be partially corrected by overexpression of a protein, SPHAR, a member of the cyclin family of proteins This can form the basis of an assay which is suitable as a screen for identifying therapeutic small molecules.

Cells which are deficient in the posttranslational modified FANCD2 are particularly sensitive to DNA damage. These cells may serve as a sensitive screen for determining whether a compound (including toxic molecules) has the capability for damaging DNA.. Conversely, these cells also serve as a sensitive screen for determining whether a compound can protect cells against DNA damage.

FA patients and patients suffering from syndromes associated with DNA repair defects die from complications of bone marrow failure. Gene transfer is a therapeutic option to correct the defect. Multiple defects may occur throughout the FA pathway. We have shown that the terminal step is critical to proper functioning of the cell and the organism. In an embodiment of the invention, correction of defects anywhere in the FA pathway may be satisfactorily achieved by gene therapy or by therapeutic agents that target the transformation of FANCD2-S to FANCD2-L so that this transformation is successfully achieved.

Gene therapy may be carried out according to generally accepted methods, for example, as described by Friedman in "Therapy for Genetic Disease," T. Friedman, ed., Oxford University Press (1991), pp. 105-121. Targeted tissues for *ex vivo* or *in vivo* gene therapy include bone marrow for example, hematopoietic stem cells prior to onset of anemia and fetal tissues involved in developmental abnormalities. Gene therapy can provide wild-type FAND2-L function to cells which carry mutant FANCD2 alleles. Supplying such a function should suppress neoplastic growth of the recipient cells-or ameliorate the symptoms of Fanconi Anemia.

The wild-type FANCD-2 gene or a part of the gene may be introduced into the cell in a vector such that the gene remains extrachromosomal. In such a situation, the gene may be expressed by the cell from the extrachromosomal location. If a gene portion is introduced and expressed in a cell carrying a mutant FANCD-2 allele, the gene portion may encode a part of the FANCD-2 protein which is required for non-neoplastic growth of the cell. Alternatively, the wild-type FANCD-2 gene or a part thereof may be introduced into the mutant cell in such a way that it recombines with the endogenous mutant FANCD-2 gene present in the cell.

Viral vectors are one class of vectors for achieving gene therapy. Viral-mediated gene transfer can be combined with direct *in vivo* gene transfer using liposome delivery, allowing one to direct the viral vectors to the tumor cells and not into the surrounding nondividing cells. Alternatively, a viral vector producer cell line can be injected into tumors (Culver et al., 1992). Injection of producer cells would then provide a continuous source of vector particles. This technique has been approved for use in humans with inoperable brain tumors.

The vector may be injected into the patient, either locally at the site of the tumor or systemically (in order to reach any tumor cells that may have metastasized to other sites). If the transfected gene is not permanently incorporated into the genome of each of the targeted tumor cells, the treatment may have to be repeated periodically.

Vectors for introduction of genes both for recombination and for extrachromosomal maintenance are known in the art, (for example as disclosed in US 5,252,479 and PCT 93/07282, and US 6,303,379) and include viral vectors such as retroviruses, herpes viruses (US 6,287,557) or adenoviruses (US 6,281,010) or a plasmid vector containing the FANCD2 ―L.

A vector carrying the therapeutic gene sequence or the DNA encoding the gene or piece of the gene may be injected into the patient either locally at the site of a tumor or systemically so as to reach metastasized tumor cells. Targeting may be achieved without further manipulation of the vector or the vector may be coupled to a molecule having a specificity of binding for a tumor where such molecule may be a receptor agonist or antagonist and may further include a peptide, lipid (including liposomes) or saccharide including an oligopolysaccharide or polysaccharide) as well as synthetic targeting molecules. The DNA may be conjugated via polylysine to a binding ligand. If the transfected gene is not permanently incorporated into the genome of each of the targeted tumor cells, the treatment may have to be repeated periodically.

Methods for introducing DNA into cells prior to introduction into the patient may be accomplished using techniques such as electroporation, calcium phosphate coprecipitation and viral transduction as described in the art (US 6,033,857), and the choice of method is within the competence of the routine experimenter

Cells transformed with the wild-type FANCD 2 gene or mutant FANCD 2 gene can be used as model systems to study remission of diseases resulting from defective DNA repair and drug treatments which promote such remission.

As generally discussed above, the FANCD 2 gene or fragment, where applicable, may be employed in gene therapy methods in order to increase the amount of the expression products of such genes in abnormal cells. Such gene therapy is particularly appropriate for use in pre-cancerous cells, where the level of FANCD 2-L polypeptide may be absent or diminished compared to normal cells and where enhancing the levels of FANCD2-L may slow the accumulation of defects arising from defective DNA repair and hence postpone initiation of a cancer state. It may also be useful to increase the level of expression of the FANCD 2 gene even in those cells in which the mutant gene is expressed at a "normal" level, but there is a reduced level of the FANCD 2-L isoform. The critical role of FANCD2-L in normal DNA repair provides an opportunity for developing therapeutic agents to correct a defect that causes a reduction in levels of FANCD2-L. One approach to developing novel therapeutic agents is through rational drug design. Rational drug design can provide structural analogs of biologically active polypeptides of interest or of small molecules with which they interact (e.g., agonists, antagonists, inhibitors or enhancers) in order to fashion more active or stable forms of the polypeptide, or to design small molecules which enhance or interfere with the function of a polypeptide in vivo. Hodgson, 1991. Rational drug design may provide small molecules or modified polypeptides which have improved FANCD2-L activity or stability or which act as enhancers, inhibitors, agonists or antagonists of FANCD2-L activity. By virtue of the availability of cloned FANCD2 sequences, sufficient amounts of the FANCD2-L polypeptide may be made available to perform such analytical studies as x-ray crystallography. In addition, the knowledge of the FANCD2-L protein sequence provided herein will guide those employing computer modeling techniques in place of, or in addition to x-ray crystallography.

Peptides or other molecules which have FANCD2-L activity can be supplied to cells which are deficient in the protein in a therapeutic formulation. The sequence of the FANCD2-L protein is disclosed for several organisms (human, fly and plant) (SEQ ID NO: 1-3). FANCD2 could be produced by expression of the cDNA sequence in bacteria, for example, using known expression vectors with additional posttranslational modifications. Alternatively, FANCD2-L polypeptide can be extracted from FANCD2-L -producing mammalian cells. In addition, the techniques of synthetic chemistry can be employed to synthesize FANCD2-L protein. Other molecules with FANCD2-L activity (for example, peptides, drugs or organic compounds) may also be used as a therapeutic agent. Modified polypeptides having substantially similar function are also used for peptide therapy.

Similarly, cells and animals which carry a mutant FANCD2 allele or make insufficient levels of FANCD2-L can be used as model systems to study and test for substances which have potential as therapeutic agents. The cells which may be either somatic or germline can be isolated from individuals with reduced levels of FANCD2-L. Alternatively, the cell line can be engineered to have a reduced levels of FANCD2-L, as described above. After a test substance is applied to the cells, the DNA repair impaired transformed phenotype of the cell is determined.

The efficacy of novel candidate therapeutic molecules can be tested in experimental animals for efficacy and lack of toxicity. Using standard techniques, animals can be selected after mutagenesis of whole animals or after genetic engineering of germline cells or zygotes to form transgenic animals. Such treatments include insertion of mutant FANCD2 alleles, usually from a second animal species, as well as insertion of disrupted homologous genes. Alternatively, the endogenous FANCD2 gene of the animals may be disrupted by insertion or deletion mutation or other genetic alterations using conventional techniques (Capecchi, 1989 Science Vol. 244, pp 1288-1292; Valancius and Smithies, 1991). After test substances have been administered to the animals, the growth of tumors must be assessed. If the test substance prevents or suppresses pathologies arising from defective DNA repair, then the test substance is a candidate therapeutic agent for the treatment of the diseases identified herein.

The present invention is described by reference to the following Examples, which are offered by way of illustration and are not intended to limit the invention in any manner. Standard techniques well known in the art or the techniques specifically described below were utilized.

All references cited herein are incorporated by reference.

### EXAMPLES

### Example 1: Experimental protocols used in Examples 2-8

*Cell Lines and Culture Conditions.* Epstein-Barr virus (EBV) transformed lymphoblasts were maintained in RPMI media supplemented with 15% heat-inactivated fetal calf serum (FCS) and grown in a humidified 5% CO2-containing atmosphere at 37°C. A control lymphoblast line (PD7) and FA lymphoblast lines (FA-A (HSC72), FA-C (PD-4), FA-D (PD-20), FA-F (EUFA121), and FA-G (EUFA316)) have been previously described (de Winter et al., 1998, Nat.Genet. 20: 281-283) (Whitney et al., 1995, Nat.Genet. 11: 341-343) (Yamashita et al., 1994, P.N.A.S. 91: 6712-6716) (de Winter et al., 2000, Am.J.Hum.Genet. 67: 1306-1308). PD81 is a lymphoblast cell line from an FA-A patient. The SV40-transformed FA fibroblasts, GM6914, PD426, FAG326SV and PD20F, as well as Hela cells, were grown in DMEM supplemented with 15% FCS. FA cells (both lymphoblasts and fibroblasts) were functionally complemented with pMMP retroviral vectors containing the corresponding FANC cDNAs, and functional complementation was confirmed by the MMC assay (Garcia-Higuera et al., 1999, Mol.Cell.Biol. 19: 4866-4873) (Kuang et al., 2000, Blood 96:1625-1632).

*Cell Cycle Synchronization.* HeLa cells, GM6914 cells, and GM6914 cells corrected with the pMMP-FANCA retrovirus were synchronized by the double thymidine block method as previously described, with minor modifications (Kupfer et al., 1997, Blood 90: 1047-1054). Briefly, cells were treated with 2mM thymidine for 18 hours, thymidine-free media for 10 hours, and additional 2mM thymidine for 18 hours to arrest the cell cycle at the G1/S boundary. Cells were washed twice with PBS and then released in DMEM +15% FCS and analyzed at various time intervals.

Alternatively, HeLa cells were treated with 0.5 mM mimosine (Sigma) for 24 hours for synchronization in late G1 phase (Krude, 1999), washed twice with PBS, and released into DMEM +15% FCS. For synchronization in M phase, a nocodazole block was used (Ruffner et al.,1999, Mol.Cell.Biol. 19: 4843-4854). Cells were treated with 0.1 µg/ml nocodazole (Sigma) for 15 hours, and the non-adherent cells were washed twice with PBS and replated in DMEM +15%.

*Cell Cycle Analysis.* Trypsinized cells were resuspended in 0.5ml of PBS and fixed by adding 5ml of ice-cold ethanol. Cells were next washed twice with PBS with 1% bovine serum albumin fractionV (1%BSA/PBS) (Sigma), and resuspended in 0.24 ml of 1%BSA/PBS. After adding 30 µ1 of 500 µg/ml propidium iodide (Sigma) in 38 mM sodium citrate (pH7.0) and 30 µl of 10mg/ml DNase free RNaseA (Sigma), samples were incubated at 37°C for 30 min. DNA content was measured by FACScan (Beckton Dickinson), and data were analyzed by the CellQuest and Modfit LT program (Becton Dickinson).

*Generation of an anti-FANCD2 antiserum.* A rabbit polyclonal antiserum against FANCD2 was generated using a GST-FANCD2 (N-terminal) fusion protein as an antigen source. A 5' fragment was amplified by polymerase chain reaction (PCR) from the full length FANCD2 cDNA with the primers (SEQ ID NO: 95) DF4EcoRI (5' AGCCTCgaattcGTTTCCAAAAGAAGACTGTCA-3') and (SEQ ID NO: 96) DR816Xh (5'-GGTATCctcgagTCAAGACGACAACTTATCCATCA-3'). The resulting PCR product of 841 bp, encoding the amino-terminal 272 amino acids of the FANCD2 polypeptide was digested with EcoRI/XhoI and subcloned into the EcoRI/XhoI sites of the plasmid pGEX4T-1 (Pharmacia). A GST-FANCD2 (N-terminal) fusion protein of the expected size (54 kD) was expressed in E. coli strain DH5γ, purified over glutathione-S-sepharose, and used to immunize a New Zealand White rabbit. An FANCD2-specific immune antiserum was affinity-purified by passage over an AminoLink Plus column (Pierce) loaded with GST protein and by passage over an AminoLink Plus column loaded with the GST-FANCD2 (N-terminal) fusion protein.

*Generation of anti-FANCD2 MoAbs.* Two anti-FANCD2 monoclonal antibodies were generated as follows. Balb/c mice were immunized with a GST-FANCD2 (N-terminal) fusion protein, which was the same fusion protein used for the generation of the rabbit polyclonal antiserum (E35) against FANCD2. Animals were boosted with immunogen for the four days before fusion, splenocytes were harvested, and hybridization with myeloma cells was performed. Hybridoma supernatants were collected and assayed using standard ELISA assay as the initial screen and immunoblot analysis of FANCD2 as the secondary screen. Two anti-human FANCD2 monoclonal antibodies (MoAbs) (F117 and F114) were selected for further study. Hybridoma supernatants from the two positive cell lines were clarified by centrifugation. Supernatants were used as MoAbs for western blotting. MoAbs were purified using an affinity column for IgG. MoAbs were stored as 0.5 mg/ml stocks in phosphate buffered saline (PBS). Anti-HA antibody (HA.11) was from Babco.

*Immunoblotting.* Cells were lysed with IX sample buffer (50 mM Tris-HCl pH6.8, 86mM 2-mercaptoethanol, 2% sodium dodecyl sulfate (SDS), boiled for 5 min, and subjected to 7.5% polyacrylamide SDS gel electrophoresis. After electrophoresis, proteins were transferred to nitrocellulose using a submerged transfer apparatus (BioRad) filled with 25 mM Tris base, 200 mM glycine, 20% methanol. After blocking with 5% non-fat dried milk in TBS-T (50mM Tris-HCl, pH 8.0, 150mM NaCl, 0.1% Tween 20) the membrane was incubated with the primary antibody diluted in TBS-T (1:1000 dilution for the affinity-purified anti-FANCD2 polyclonal antibody (E35) or anti-HA (HA.11), 1:200 dilution for the anti-FANCD2 mouse monoclonal antibody F117), washed extensively and incubated with the appropriate horseradish peroxidase- linked secondary antibody (Amersham). Chemiluminescence was used for detection.

*Generation of DNA Damage.* Gamma irradiation was delivered using a Gamma cell 40 apparatus. UV exposure was achieved using a Stratalinker (Stratagene) after gently aspirating the culture medium. For Mitomycin C treatment cells were continuously exposed to the drug for the indicated time. Hydroxyurea (Sigma) was added to a final concentration of 1 mM for 24 hours.

*Detection of Monoubiquatinated FANCD2.* HeLa cells (or the FA-G fibroblasts, FAG326SV) were transfected using FuGENE6 (Roche), following the manufacturer's protocol. HeLa cells were plated onto 15 cm tissue culture dishes and were transfected with 15 µg of a HA-tagged ubiquitin expression vector (pMT 123) (Treier et al.,1994, Cell 78: 787-798) per dish. Twelve hours following transfection, cells were treated with the indicated concentration of MMC (0,10,40,160ng/ml) or the indicated dose of IR (0,5,10,10,20Gy). After 24 hour-incubation with MMC, or two hours after IR treatment, whole cell extracts were prepared in Lysis Buffer (50 mM TrisHCl pH 7.4, 150 mM NaCl, 1% (v/v) Triton X-100) supplemented with protease inhibitors (1 µg/ml leupeptin and pepstatin, 2 µg/ml aprotinin, 1 mM phenylmethylsulfonylfluoride) and phosphatase inhibitors (1 mM sodium orthovanadate, 10 mM sodium fluoride). Using the polyclonal antibody to FANCD2 (E35), immunoprecipitation (IP) was performed essentially as described (Kupfer et al., 1997) except that each IP was normalized to contain 4mg of protein. As a negative control, preimmune serum from the same rabbit was used in IP reaction. Immunoblotting was done using anti-HA (HA.11), or anti-FANCD2 (F117) monoclonal antibody.

*Ubiquitin Aldehyde Treatment.* HeLa cells were treated with 1mM hydroxyurea for 24 hours, and whole cell extracts were prepared in Lysis Buffer supplemented with protease inhibitors and phosphatase inhibitors. 200µg of cell lysate in 67µl of reaction with 6.7µl of 25µM ubiquitin aldehyde (BostonBiochem) in DMSO or with 6.7µl of DMSO were incubated at 30°C or at 37°C for the indicated periods. Sixty-seven microliters of 2X sample buffer was added to each sample, and the samples were boiled for 5 min, separated by 7.5% SDS-PAGE, and immunoblotted for FANCD2 using the FI17 monoclonal anti-human FANCD2 antibody.

*Immunofluorescence Microscopy.* Cells were fixed with 2% paraformaldehyde in PBS for 20 min., followed by permeabilization with 0.3% Triton-X-100 in PBS (10 min). After blocking in 10% goat serum, 0.1% NP-40 in PBS (blocking buffer), specific antibodies were added at the appropriate dilution in blocking buffer and incubated for 2-4 hours at room temperature. FANCD2 was detected using the affinity-purified E35 polyclonal antibody (1/100). For BRCA1 detection, we used a commercial monoclonal antibody (D-9, Santa Cruz) at 2 µg/ml. Cells were subsequently washed three times in PBS + 0.1% NP-40 (10-15 min each wash) and species-specific fluorescein or Texas red-conjugated secondary antibodies (Jackson Immunoresearch) were diluted in blocking buffer (anti-mouse 1/200, anti-rabbit 1/1000) and added. After 1 hour at room temperature three more 10-15 min washes were applied and the slides were mounted in Vectashield (Vector laboratories). Images were captured on a Nikon microscope and processed using Adobe Photoshop software.

*Meiotic Chromosome Staining.* Surface spreads of pachytene and diplotene spermatocytes from male mice between the ages of 16 and 28 days old were prepared as described by (Peters et al., 1997). A polyclonal goat antibody to the mouse SCP3 protein was used to visualize axial elements and synaptonemal complexes in the meiotic preparations. The M118 mouse monoclonal antibody against mouse BRCAl was generated by standard techniques, by immunizing mice with murine BRCAl protein. The affinity-purified E35 rabbit polyclonal antibody was used in 1:200 dilution to detect FANCD. Antibody incubation and detection procedures were a modification of the protocol of (Moens et al., 1987, J.Cell.Biol. 105: 93-103) as described by (Keegan et al., 1996, Genes Dev. 10: 2423-2437). Combinations of donkey-anti mouse IgG-FITC- congugated, Donkey-anti rabbit IgG-TRITC-congugated, and Donkey-anti goat IgG-Cy5-congugated secondary antibodies were used for detection (Jackson ImmunoResearch Laboratories). All preparations were counterstained with 4', 6' diamino-2-phenylindole (DAPI, Sigma) and mounted in aDABCO (Sigma) antifade solution. The preparations were examined on a Nikon E1000 microscope (60X CFI Plan Apochromat and 100X CFI Plan Fluor oil-immersion objectives). Each fluorochrome (FITC, TRITC, Cy5 and DAPI) image was captured separately as an 800 x 1000 pixel 12-bit source image via IPLab software (Scanalytics) controlling a cooled-CCD camera (Princeton Instruments MicroMax) and the separate 12 bit grey scale images were resampled, 24-bit pseudocolored and merged using Adobe Photoshop.

### Example 2: The FA genes interact in a common cellular pathway.

Normal lymphoblasts express two isoforms of the FANCD2 protein, a short form (FANCD2-S, 155 kD) and a long form (FANCD2-L, 162 kD). Figure 1 shows what happened when whole cell extracts were prepared from a lymphoblast line and cellular proteins were immunoprecipitated with an anti-FANCD2 antiserum. Normal wild type cells expressed two isoforms of the FANCD2 protein - a low molecular weight isoform FANCD2-S (155 kD isoform) and a high molecular weight isoform (FANCD2-L) (162kD isoform). FANCD2-S is the primary translation product of the cloned FANCD2 cDNA. We next evaluated a large series of FA lymphoblasts and fibroblasts for expression of the FANCD2 isoforms (Table 5). Correction of these FA cell lines with the corresponding FA cDNA resulted in functional complementation and restoration of the high molecular weight isoform, FANCD2-L.

As previously described, FA cells are sensitive to the DNA crosslinking agent, MMC, and in some cases, to ionizing radiation (IR). Interestingly, FA cells from multiple complementation groups (A, C, G, and F) only expressed the FANCD2-S isoform (Figure 1A, lanes 3,7,9,11). FA cells from complementation groups B and E also express only the FANCD2-S. Functional correction of the MMC and IR sensitivity of these FA cells with the corresponding FANC cDNA restored the FA protein complex (Garcia-Higuera et al., 1999) and restored the high molecular weight isoform (FANCD2-L) (Figure 1A, lanes 4,8,10,12). Taken together, these results demonstrate that the FA protein complex, containing FANCA, FANCC, FANCF, and FANCG, directly or indirectly regulates the expression of the two isoforms of FANCD2. The six cloned FA genes therefore appear to interact in a common pathway.

### Example 3: The FA protein complex is required for the monoubiquitination of FANCD2

The high molecular weight isoform of FANCD2 could result from one or more mechanisms, including alternative splicing of the FANCD2 mRNA or post-translational modification(s) of the FANCD2 protein. Treatment with phosphatase did not convert FANCD2-L to FANCD2-S, demonstrating that phosphorylation alone does not account for the observed difference in their molecular mass.

In order to identify other possible post-translational modifications of FANCD2, we initially sought cellular conditions which regulate the conversion of FANCD2-S to FANCD2-L (Figure 1 B, C). Since FA cells are sensitive to MMC and IR, we reasoned that these agents might regulate the conversion of FANCD2-S to FANCD2-L in normal cells. Interestingly, HeLa cells treated with MMC (Figure 1B, lanes 1-6) or IR (Figure 1C, lanes 1-6) demonstrated a dose-dependent increase in the expression of the FANCD2-L isoform.

To determine whether FANCD2-L is a ubiquitinated isoform of FANCD2-S, we transfected HeLa cells with a cDNA encoding HA-Ubiquitin (Treier et al., 1994). Cellular exposure to MMC (Figure 1B, lanes 7-10) or IR (Figure 1C, lanes 7-10) resulted in a dose-dependent increase in the HA-ubiquitin conjugation of FANCD2. Only the FANCD2-L isoform, and not the FANCD2-S isoform, was immunoreactive with an anti-HA antibody. Although FANCD2 was not ubiquinated in FA cells, FANCD2 ubiquination was restored upon functional complementation of these cells. Although FANCD2 was not ubiquitinated in FA cells, FANCD2 ubiquitination was restored upon functional complementation of these cells. Since the FANCD2-S and FANCD2-L isoforms differ by 7kD, the FANCD2-L probably contains a single ubiquitin moiety (76 amino acids) covalently bound by an amide linkage to an internal lysine residue of FANCD2.

To confirm the monoubiquitination, we isolated FANCD2-L protein from HeLa cells and analyzed its tryptic fragments by mass spectrometry (Wu et al., 2000, Science 289, 11a). Ubiquitin tryptic fragments were unambiguously identified, and a site of monoubiquitination (K561 of PANCD2) was also identified. Interestingly, this lysine residue is conserved among FANCD2 sequences from human, *Drosophila,* and *C. elegans,* suggesting that the ubiquitination of this site is critical to the FA pathway in multiple organisms. Mutation of this lysine residue, FANCD2 (K561R), resulted in loss of FANCD2 monoubiquitination.

### Example 4: Formation of Nuclear Foci Containing FANCD2 requires an intact FA pathway.

We examined the immunofluorescence pattern of the FANCD2 protein in uncorrected, MMC-sensitive FA fibroblasts and functionally-complemented fibroblasts (Figure 2).

The corrected FA cells expressed both the FANCD2-S and FANCD2-L isoforms (Figure 2A, lanes 2,4,6,8). The endogenous FANCD2 protein was observed exclusively in the nucleus of human cells, and no cytoplasmic staining was evident (Figure 2B, a-h). The PD-20 (FA-D) cells have decreased nuclear immunofluorescence (Figure 2B, d), consistent with the decreased expression of FANCD2 protein in these cells by immunoblot (Figure 2A, lane 7). In PD-20 cells functionally-corrected with the FANCD2 gene by chromosome transfer, the FANCD2 protein stained in two nuclear patterns. Most corrected cells had a diffuse nuclear pattern of staining, and a minor fraction of cells stained for nuclear foci (see dots, panel h). Both nuclear patterns were observed with three independently-derived anti-FANCD2 antisera (1 polyclonal, 2 monoclonal antisera). FA fibroblasts from subtypes A, G, and C showed only the diffuse pattern of FANCD2 nuclear immunofluorescence. Functional complementation of these cells with the FANCA, FANCG, or FANCC cDNA, respectively, restored the MMC resistance of these cells (Table 6), and restored the nuclear foci in some cells. The presence of the high molecular weight FANCD2-L isoform therefore correlates with the presence of FANCD2 nuclear foci, suggesting that only the monoubiquitinated. FANCD2-L isoform is selectively localized to these foci.

### Example 5: The FANCD2 protein is localized to nuclear foci during S phase of the Cell Cycle

Since only a fraction of the asynchronous functionally-complemented cells contained FANCD2 nuclear foci, we reasoned that these foci might assemble at discrete times during the cell cycle. To test this hypothesis, we examined the formation of the FANCD2-L isoform and FANCD2 nuclear foci in synchronized cells (Figure 3). HeLa cells were synchronized at the G1/S boundary, released into S phase, and analyzed for formation of the FANCD2-L isoform (Figure 3A). The FANCD2-L isoform was expressed specifically during late G1 phase and throughout S phase. Synchronized, uncomplemented FA cells (FA-A fibroblasts, GM6914) expressed normal to increased levels of FANCD2-S protein but failed to express FANCD2-L at any time during the cell cycle. Functional complementation of these FA-A cells by stable transfection with the FANCA cDNA restored S phase-specific expression of FANCD2-L. The S phase-specific expression of the FANCD2-L isoform was confirmed when HeLa cells were synchronized by other methods, such as nocodazole arrest (Figure 3B) or mimosine exposure (Figure 3B). Cells arrested in mitosis did not express FANCD2-L, suggesting that the FANCD2-L isoform is removed or degraded prior to cell division (Figure 3B, mitosis). Taken together, these results demonstrate that the monoubiquitination of the FANCD2 protein is highly regulated during the cell cycle, and that this modification requires an intact FA pathway.

The cell cycle dependent expression of the FANCD2-L isoform also correlated with the formation of FANCD2 nuclear foci (Figure 3 C). Nocodazole arrested (mitotic) cells express no FANCD2-L isoform and exhibit no FANCD2 nuclear foci (Figure 3C, 0 hour). When these synchronized cells were allowed to traverse S phase (15 to 18 hours), an increase in FANCD2 nuclear foci was observed.

### Example 6: The FANCD2 protein is localized to nuclear foci in response to DNA damage.

We examined the accumulation of the FANCD2-L isoform and FANCD2 nuclear foci in response to DNA damage (Figure 4). Previous studies have shown that FA cells are sensitive to agents which cause DNA interstrand crosslinks MMC) or double strand breaks (IR) but are relatively resistant to ultraviolet light (UV) and monofunctional alkylating agents. MMC activated the conversion of FANCD2-S to FANCD2-L in asynchronous HeLa cells (Figure 4A). Maximal conversion to FANCD2-L occurred 12-24 hours after MMC exposure, correlating with the time of maximal FANCD2 nuclear focus formation. There was an increase in FANCD2 nuclear foci corresponding to the increase in FANCD2-L. Ionizing radiation also activated a time-dependent and dose-dependent increase in FANCD2-L in HeLa cells, with a corresponding increase in FANCD2 foci (Figure 4B). Surprisingly, ultraviolet (UV) light activated a time-dependent and dose-dependent conversion of FANCD2-S to FANCD2-L, with a corresponding increase in FANCD2 foci (Figure 4C).

We tested the effect of DNA damage on FA cells (Figure 4D). FA cells from multiple complementation groups (A, C, and G) failed to activate the FANCD2-L isoform and failed to activate FANCD2 nuclear foci in response to MMC or IR exposure. These data suggest that the cellular sensitivity of FA cells results, at least in part, from their failure to activate FANCD2-L and FANCD2 nuclear foci.

### Example 7: Co-localization of activated FANCD2 and BRCA1 protein.

Like FANCD2, the breast cancer susceptibility protein, BRCA1, is upregulated in proliferating cells and is activated by post-translational modifications during S phase or in response to DNA damage. BRCA has a carboxy terminus 20aminoacids which contain a highly acidic HMG ―like domain suggesting a possible mechanism for chromatin repair The BRCA1 protein colocalizes in IR-inducible foci (IRIFs) with other proteins implicated in DNA repair, such as RAD51 or the NBS/Mre11/RAD50 complex. Cells with biallelic mutations in BRCA1 have a defect in DNA repair and are sensitive to DNA damaging agents such as IR and MMC (Table 5). Taken together, these data suggest a possible functional interaction between the FANCD2 and BRCA1 proteins. BRCA foci are large (2mDa) multiprotein complexes including ATM and ATM substrates involved in DNA repair (BRCA1) and checkpoint functions (NBS)

In order to determine whether the activated FANCD2 protein colocalizes with the BRCA1 protein, we performed double immunolabelling of HeLa cells (Figure 5). In the absence of ionizing radiation, approximately 30-50% of cells contained BRCA1 nuclear foci (Figure 5A). In contrast, only rare cells traversing S phase contained FANCD2 dots (b,e). These nuclear foci were also immunoreactive with antisera to both BRCA1 and FANCD2 (c,f). Following IR exposure, there was an increase in the number of cells containing nuclear foci and the number of foci per cell. These nuclear foci were larger and more fluorescent than foci observed in the absence of IR. Again, these foci contained both BRCA1 and FANCD2 protein (i,l). An interaction of FANCD2-L and BRCA1 was further confirmed by coimmunoprecipitation of the proteins (Figure 5B) from exponentially growing HeLa cells exposed to IR.

We examined the effect of BRCA1 expression on the formation of FANCD2-L and nuclear foci (Figure 6). The BRCA1 (-/-) cell line, HCC1937, expresses a mutant form of the BRCA1 protein with a carboxy terminal truncation. Although these cells expressed a low level of FANCD2-L (Figure 6A), IR failed to activate an increase in FANCD2-L levels. Also, these cells had a decreased number of IR-inducible FANCD2 foci (Figure 6B, panels c, d). Correction of these BRCA1 (-/-) cells by stable transfection with the BRCA1 cDNA restored IR-inducible FANCD2 ubiquitination and nuclear foci (Figure 6B, panels k,l). These data suggest that the wild-type BRCA1 protein is required as an "organizer" for IR-inducible FANCD2 dot formation and further suggests a functional interaction between the proteins.

### Example 8: Co-localization of FANCD2 and BRCA1 on Meiotic Chromosomes.

The association of FANCD2 and BRCA1 in mitotic cells suggested that these proteins might also colocalize during meiotic prophase. Previous studies have demonstrated that the BRCA1 protein is concentrated on the unsynapsed/axial elements of human synaptonemal complexes in zygotene and pachytene spermatocytes. To test for a possible colocalization of FANCD2 and BRCA1 in meiotic cells, we examined surface spreads of late pachytene and early diplotene mouse spermatocytes for the presence of FANCD2 and BRCA1 protein (Figure 7). We found that the rabbit polyclonal anti-FANCD2 antibody E35 specifically stained the unpaired axes of the X and Y chromosomes in late pachynema (Figure 7a) and in diplonema (Figures 7d, 7e and 7g). Under the same experimental conditions, preimmune serum did not stain synaptonemal complexes (Figures 7b and 7c). The M118 anti-BRCA1 antibody stained the unpaired sex chromosomes in mouse pachytene and diplotene spermatocytes (Figures 7f and 7h). FANCD2 Ab staining of the unsynapsed axes of the sex chromosomes was interrupted, giving a beads-on-a-string appearance (Figure 7g). A consecutive examination of 20 pachytene nuclei indicated that most (∼65°%) of these anti-FANCD2 foci colocalized with regions of intense anti-BRCA1 staining, further supporting an interaction between these proteins (Figures 7g, 7h, and 7i). These results provide the first example of a FANC protein (activated FANCD2) which binds to chromatin.

### Example 9: Experimental protocols for obtaining and analyzing the DNA and protein sequence for FANCD2.

Northern Hybridizations. Human adult and fetal multi-tissue mRNA blots were purchased from Clontech (Palo Alto, CA). Blots were probed with ³²P labeled DNA from EST clone SGC34603. Standard hybridization and washing conditions were used. Equal loading was confirmed by re-hybridizing the blot with an actin cDNA probe.

Mutation Analysis. Total cellular RNA was reverse transcribed using a commercial kit (Gibco/BRL). The 5' end section of FANCD2 was amplified from the resulting patient and control cDNA with a nested PCR protocol. The first round was performed with primers (SEQ ID NO: 97) MG471 5'-AATCGAAAACTACGGGCG-3' and (SEQ ID NO: 98) MG457 5'-GAGAACACATGAATGAACGC-3'. The PCR product from this round was diluted 1:50 for a subsequent round using primers (SEQ ID NO: 99) MG492 5'-GGCGACGGCTTCTCGG AAGTAATTTAAG-3' and (SEQ ID NO: 100) MG472 5'-AGCGGCAGGAGGTTTATG-3'. The PCR conditions were as follows: 94°C for 3 min, 25 cycles of 94°C for 45 sec, 50°C for 45 sec, 72°C for 3 min and 5min of 72°C at the end. The 3' portion of the gene was amplified as described above but with primers, (SEQ ID NO: 101) MG474 5'-TGGCGGCAGACAGAAGTG-3' and (SEQ ID NO: 102) MG475 5'-TGGCGGCAGACAGAAGTG-3'. The second round of PCR was performed with (SEQ ID NO: 103) MG491 5'-AGAGAGCCAACCTGAGCGATG-3' and (SEQ ID NO: 104) MG476 5'-GTGCCAGACTCTGGTGGG-3'. The PCR products were gel-purified, cloned into the pT-Adv vector (Clontech) and sequenced using internal primers.

Allele specific assays: Allele specific assays were performed in the PD20 family and 290 control samples (=580 chromosomes). The PD20 family is of mixed Northern European descent and VU008 is a Dutch family. Control DNA samples were from unrelated individuals in CEPH families (n=95), samples from unrelated North American families with either ectodermal dysplasia (n=95) or Fanconi Anemia (n=94). The maternal nt376a→g mutation in the PD 20 family created a novel MspI restriction site. For genomic DNA, the assay involved amplifying genomic DNA using the primers (SEQ ID NO: 105) MG792 5'- AGGA GACACCCTTC CTATCC-3' located in exon 4 and (SEQ ID NO: 106) MG803 5'- GAAG TTGGCAAAACAGACTG-3' which is in intron 5. The size of the PCR product was 340 bp, yielding two fragments of 283 bp and 57 bp upon MspI digestion if the mutation was present. For analysis of the reverted cDNA clones, PCR was performed using primers (SEQ ID NO: 107) MG924 5'-TGTCTTGTGAGCGTCTGCAGG-3' and (SEQ ID NO: 108) MG753 5'-AGGTT TTGATAATGGCAGGC-3'. The paternal exon 37 mutation (R1236H) in PD20 and exon12 missense mutation (R302W) in VU008 were tested by allele specific oligonucleotide (ASO) hybridization (Wu, et al., 1989, DNA 8: 135-142). For the exon 12 assay, genomic DNA was amplified with primers (SEQ ID NO: 109) MG979 5'-ACTGGACTGTGCCTACCCACTATG-3' and (SEQ ID NO: 110) MG984 5'-CCTGTGTGAGGATGAGCTCT-3'. Primers (SEQ ID NO: 171) MG818 5'-AGAGGTAGGGAAGGAAGCTAC-3' and (SEQ ID NO: 172) MG813 5'- CCAAAGT CCACTTCTTGAAG-3' were used for exon 37. Wild-type (SEQ ID NO: 111) (5'-TTCTCCCGAAGCTCAG-3' for R302W and (SEQ ID NO: 112) 5'- TTTCTTCC GTGTGATGA-3' for R1236H) and mutant SEQ ID NO: 111 (5'-TTCTCCCAAAGCTGAG-3'R302W and SEQ ID NO: 112 5'-TTTCTTCCATGTGATGA-3'for R1236H) oligonucleotides were end-labeled with γ³²P-[ATP] and hybridized to dot-blotted target PCR products as previously ss novel DdeI site. The wild-type PCR product digests into a 117 and 71bp product, whereas the mutant allele yields three fragments of 56, 61 and 71bps in length. PCR in all of the above assays was performed with 50 ng of genomic DNA for 37 cycles of 94°C for 25 sec, 50°C for 25 sec and 72°C for 35 sec.

Generation of an anti-FANCD2 antiserum: A rabbit polyclonal antiserum against FANCD2 was generated using a GST-FANCD2 (N-terminal) fusion protein as an antigen source. A 5' fragment was amplified by polymerase chain reaction (PCR) from the full length FANCD2 cDNA with the primers (SEQ ID NO: 113) DF4EcoRI (5'― AGCCTCgaattcGTTTCC AAAAGAAGACTGTCA-3') and (SEQ ID NO: 114) DR816Xh (5'- GGTATCctcgagTCAAGA CGACAACTTATCCATCA-3'). The resulting PCR product of 841 bp, encoding the amino-terminal 272 amino acids of the FANCD2 polypeptide was digested with EcoRI/XhoI and subcloned into the EcoRI/XhoI sites of the plasmid pGEX4T-1 (Pharmacia). A GST-FANCD2 (N-terminal) fusion protein of the expected size (54 kD) was expressed in E. coli strain DH5□, purified over glutathione-S-sepharose, and used to immunize a New Zealand White rabbit. An FANCD2-specific immune antiserum was affinity-purified over an AminoLink Plus column (Pierce) loaded with GST protein and over an AminoLink Plus column loaded with the GST-FANCD2 (N-terminal) fusion protein.

### Immunoblotting As in Example 1.

Cell Lines and Transfections: PD20i is an immortalized and PD733 a primary FA fibroblast cell line generated by the Oregon Health Sciences Fanconi Anemia cell repository (Jakobs, et al., 1996, Somet.Cell.Mol.Genet. 22: 151-157). PD20 lymphoblasts were derived from bone marrow samples. VU008 is a lymphoblast and VU423 a fibroblast line generated by the European Fanconi Anemia Registry (EUFAR). VU423i was an immortalized line derived by transfection with SV40 T-antigen (Jakobs, et al., 1996) and telomerase (Bodnar, et al.,1998, Science 279: 349-352). The other FA cell lines have been previously described. Human fibroblasts were cultured in MEM and 20% fetal calf serum. Transformed lymphoblasts were cultured in RPMI 1640 supplemented with 15% heat-inactivated fetal calf serum.

To generate FANCD2 expression constructs, the full-length cDNA was assembled from cloned RT-PCR products in pBluescript and the absence of PCR induced mutations was confirmed by sequencing. The expression vectors pIRES-Neo, pEGFP-N1, pRevTRE and pRevTet-off were from ClonTech (Palo Alto, CA). The FANCD2 was inserted into the appropriate multi-cloning site of these vectors. Expression constructs were electroporated into cell line PD20 and a normal control fibroblast cell line, GM639 using standard conditions (van den Hoff, et al., 1992). Neomycin selection was carried out with 400 µg/ml active G418 (Gibco).

Whole cell fusions: For the whole cell fusion experiments, a PD20 cell line (PD20i) resistant to hygromycin B and deleted for the HPRT locus was used (Jakobs, et al.,1997, Somat. Cell. Mol. Genet. Vol. 23, pp. 1-7). Controls included PD 24 (primary fibroblasts from affected sibling of PD20) and PD 319i (Jakobs, et al., 1997) (immortal fibroblasts from a non-A, C, D or G FA patient). 2.5 x 10⁵ cells from each cell line were mixed in a T25 flask and allowed to recover for 24 hours. The cells were washed with serum-free medium and then fused with 50% PEG for 1 min. After removal of the PEG, the cells were washed 3x with serum-free medium and allowed to recover overnight in complete medium without selection. The next day, cells were split 1:10 into selective medium containing 400 µg/mlygromycin B (Roche Molecular) and 1X HAT. After the selection was complete, hybrids were passaged once and then analyzed as described below.

Retroviral Transduction of FA-D2 cells and complementation analysis: The full length FANCD2 cDNA was subcloned into the vector, pMMP-puro (Pulsipher, et al., 1998). Retroviral supernatants were used to transduce PD20F, and puromycin resistant cells were selected. Cells were analyzed for MMC sensitivity by the crystal violet assay (Naf, et al., 1998).

Chromosome Breakage Analysis: Chromosome breakage analysis was performed by the Cytogenetics Core Lab at OHSU (Portland, OR). For the analysis (Cohen, et al., 1982) cells were plated into T₂₅ flasks, allowed to recover and then treated with 300 ng/ml of DEB for two days. After treatment, the cells were exposed to colcemid for 3 hours and harvested using 0.075 M KCl and 3:1 methanol:acetic acid. Slides were stained with Wright's stain and 50-100 metaphases were scored for radials.

### Example 10: Mouse models for FA for use in screening potential therapeutic agents.

Murine models of FANCD2 can be made using homologous recombination in embryonic stem cells or targeted disruption as described in D'Andrea et al. (1997) 90: 1725-1736, and Yang et al. (2001) Blood 98; 1-6. The knockout of FANCD2 locus in mice is not a lethal mutation. These knock-out animals have increased susceptibility to cancer and furthermore display other symptoms characteristic of FA. It is expected that administering certain therapeutic agents to the knock-out mice will reduce their susceptibility to cancer. Moreover, it is expected that certain established chemotherapeutic agents will be identified that are more effective for treating knock-out mice who have developed cancers as a result of the particular genetic defect and this will also be useful in treating human subjects with susceptibility to cancer or who have developed cancers as a result of a mutation in the FANCD2 locus.

We can generate experimental mice models with targeted disruptions of FANCD2 using for example the approach described by Chen et al (1996) Nat. Genet. Vol. 12, pp. 448-451, for Fancc who created a disruption in an exon of the gene, and by Whitney et al (1996) Vol. 88, pp. 49-58, who used homologous recombination to create a disruption of an exon of the gene. In both animal models, spontaneous chromosome breakage and an increase in chromosome breaks in splenic lymphocytes in response to bifunctional alkylating agents are observed. In both models, Fancd2-/- mice have germ cell defects and decreased fertility. The Fancd2 murine knockout model is useful in examining (1) the role of the Fancd2 gene in the physiologic response of hematopoietic cells to DNA damage, (2) the in vivo effects of inhibitory cvtokines on FA marrow cells. 129/Sv and C57BL can be generated following standard protocols. Mouse tail genomic DMA can be prepared as previously described and used as a template for polymerase chain reaction (PCR) genotyping.

Splenocytes can be prepared from 6-week-old mice of known Fancd2 genotype. The spleen is dissected, crushed in RPMI medium into a single-cell suspension, and filtered through a 70 µm filter. Red cells are lysed in hypotonic ammonium chloride. The remaining splenic lymphocytes are washed in phosphate-buffered saline and resuspended in RPMI/10% fetal bovine serum plus phytohemagglutinin. Cells are tested for viability by the trypan blue exclusion assay. Cells are cultured for 24 hours in media and exposed to MMC or DEB for an additional 48 hours. Alternatively, cells are cultured for 50 hours, exposed to IR (2 or 4 Gy, as indicated), and allowed to recover for 12 hours before chromosome breakage or trypan blue exclusion (viability) analysis.

Mononuclear cells can be isolated from the femurs and tibiae of 4- to 6-week-old Fancd2+/- or Fancd2 -/- mice, as previously described. A total of 2 x 104 cells were cultured in 1 mL of MethoCult M343 media (StemCell Technologies, Vancouver, BC) with or without MMC treatment. Colonies are scored at day 7, when most of the colonies belong to the granulocyte-macrophage colony-forming unit or erythroid burst-forming unit lineages. Each number are averaged from duplicate plates, and the data derived from 2 independent experiments.

Lymphocytes isolated from thymus, spleen, and peripheral lymph nodes are stained for T- or B-lymphocyte surface molecules with fluorescein isothiocyanate-conjugated anti-CD3, CD4, and CD19 and PE-conjugated anti-CD8, CD44, CD 45B, immonuglubulin M, and B220 (BD PharMingen, CA). Stained cells were analyzed on a Counter Epics XL flow cytometry system.

Mice ovaries and testes were isolated and fixed in 4% paraformaldehyde and further processed by the core facility of the Department of Pathology at Massachusetts General Hospital.

### Example 11: Screening assays using antibody reagents for detecting increased cancer susceptibility in human subjects

Blood samples or tissue samples can be taken from subjects for testing for the relative amounts of FANCD2-S compared to FANCD2-L and the presence or absence of FANCD2-L. Using antibody reagents specific for FANCD2-S and FANCD2-L proteins (Example 1), positive samples can be identified on Western blots as shown in Figure 14. Other antibody assays may be utilized such as, for example, one step migration binding banded assays described in 5,654,162 and 5,073,484. Enzyme linked immunosorbent assays (ELISA), sandwich assays, radioimmune assays and other immunodiagnostic assays known in the art may be used to determine relative binding concentrations of FANCD2-S and FANCD2-L.

The feasibility of this approach is illustrated by the following:
FANCD2 Diagnostic Western Blot for Screening Human Cancer Cell Lines

Human cancer cell lines were treated with or without ionizing radiation (as indicated in Figure 14) and total cell proteins were electrophoresed, transferred to nitrocellulose and immunoblotted with the anti-FANCD2 monoclonal antibody of Example 1. Ovarian cancer cell line (TOV21G) expressed FANCD2-S but not FANCD2-L (see lanes 9, 10). This cell line has a deletion of human chromosome 3p overlapping the FANCD2 gene and is hemizygous for FANCD2 and is predicted to have a mutation in the second FANCD2 allele which therefore fails to be monoubiquinated by the FA complex hence no FANCD2-L (lanes 9, 10). This example demonstrates that antibody based tests are suited for determining lesions in the FANCD2 gene which lead to increased cancer susceptibility.

### Example 12: Screening assays using nucleic acid reagents for detecting increased cancer susceptibility in human subjects

Blood samples or tissue samples can be taken from subjects and screened using sequencing techniques or nucleic acid probes to determine the size and location of the genetic lesion if any in the genome of the subject. The screening method may include sequencing the entire gene or by using sets of probes or single probes to identify lesions. It is expected that a single lesion may predominant in the population but that other lesions may arise throughout the gene with low frequency as is the case for other genetic conditions such as cystic fibrosis and the P53 tumor suppressor gene.

The feasibility of this approach is illustrated by the following:
Peripheral blood lymphocytes are isolated from the patient using standard Ficoll-Hypaque gradients and genomic DNA is isolated from these lymphocytes. We use genomic PCR to amplify 44 exons of the human FANCD2 gene (see primer Table 7) and sequence the two FANCD2 alleles to identify mutations. Where such mutations are found, we distinguish these from benign polymorphisms by their ability to ablate the functional complementation of an FA-D2 indicator cell line.

**Table 1 Complementation groups and responsible genes of Fanconi Anemia**

| **Subtype** | Estimated percentage of patients | Responsible gene | Chromosome location | Number of exons | Protein product |
|---|---|---|---|---|---|
| A | 66% | FANCA | 16q24.3 | 43 | 163Kd |
| B | 4.3% | FANCB | - | - | - |
| C | 12.7% | FANCC | 9q22.3 | 14 | 63Kd |
| D1 | rare | FANCD1 | - | - | - |
| D2 | rare | FANCD2 | 3p25.3 | 44 | 155,162kD |
| E | 12.7% | FANCE | 6p21.2-21.3 | 10 | 60kD |
| F | rare | FANCF | 11p15 | 1 | 42kD |
| G | rare | FANCG (XRCC9) | 9p13 | 14 | 68kD |

**TABLE 2**

| Diseases of Genomic Instability | | | |
|---|---|---|---|
| Disease | Damaging Agent | Neoplasm | Function |
| FA | Cross-linking agents leukemia, hepatic, gastrointestinal, and gynecological tumors | Acute myeloblastic | Unknown |
| XP | UV light | Squamous cell carcinomas | Excision repair |
| AT | Ionizing radiation | Lymphoma | Afferent pathway to p53 |
| Bloom's Syndrome | Alkylating agents | Acute lymphoblastic leukemia | Cell-cycle regulation |
| Cockayne's Syndrome | UV light | Basal cell carcinoma repair | Transcription coupled |
| Hereditary non-polyposis colon cancer (HNPCC) | Unknown | Adenocarcinoma of colon, ovarian cancer | DNA mismatch repair |

**Table 3**

| *FANCD2* Sequence Alterations | | |
|---|---|---|
| Mutations | | |
| PD20 | nt376a→g | S126G/splice |
| | nt3707g→a | R1236H |
| VU008 | nt904c→t | R302W |
| | nt958c→t | Q320X |
| PD733 | | deletion of exon 17 |

| Polymorphisms | | |
|---|---|---|
| | nt1122a→g | V374V |
| | nt1440t→c* | H480H |
| | nt1509c→t 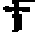 | N503N |
| | nt2141c→t* 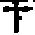 | L714P |
| | nt2259t→c | D753D |
| | nt4098t→g* 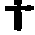 | L1366L |
| | nt4453g→a 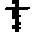 | 3UTR |
| *PD20 is heterozygous; 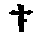 VU008 is heterozygous. | | |

**TABLE 4 Chromosome Breakage Analysis of Whole-cell Fusions.**

| Cell line/hybrids | DEB (ng/ml) | MMC (ng/ml) | % of Cells with radials | phenotype |
|---|---|---|---|---|
| PD20i | 300 | | 58 | S |
| PD24p | 300 | | na* | S |
| VU423p | 300 | | na* | S |
| PD319i | 300 | | 52 | S |
| PD20i/VU423p | 300 | | 6 | R |
| PD20i/PD24p | 300 | | 30 | S |
| PD20i/PD319i | 300 | | 0 | R |
| PD20i | | 40 | 48 | S |
| VU423i | | 40 | 78 | S |
| PD20i/VU423i | | 40 | 10 | R |
| VU423i + chr.3, clone 1 | | 40 | 74 | S |
| VU423i + chr.3, clone 2 | | 40 | 68 | S |
| VU423i + chr. 3, clone 3 | | 40 | 88 | S |
| PD20i + empty vector | 0 | 0 | 2 | |
| | | 40 | 24 | S |
| | 200 | | 62 | S |
| PD20i + FANCD2 vector | 0 | 0 | 0 | |
| | | 40 | 2 | R |
| | 200 | | 10 | R |

| | | | | |
|---|---|---|---|---|
| Groups of experiments are separated by line spaces. S, cross-linker sensitive; R, cross-linker resistant; i = immortal fibroblast line; p = primary fibroblasts * Cell viability at this concentration was too low to score for radial formation, indicating the exquisite sensitivity of primary fibroblasts to interstrand DNA-crosslinks. | | | | |

| Table 5 | | | | | |
|---|---|---|---|---|---|
| Cell line/plasmid | | FA Group | FA protein complex (1) | MMC sensitivity (2) | IR/Bleomycin sensitivity (3) |
| Lymphoblasts | PD7 | Wt | + | R | R |
| | HSC72 | A | - | S | |
| | HSC72+A | A | + | R | |
| | PD4 | C | - | S | |
| | PD4+C | C | + | R | |
| | EUFA316 | G | - | S | |
| | EUFA316+G | G | + | R | |
| | EUFA121 | F | - | S | S |
| | EUFA121+F | F | + | R | R |
| | PD20 | D | + | S | S |
| | PD20(R) | D | + | R | R |
| Fibroblasts | GM0637 | Wt | + | R | R |
| | GM6914 | A | - | S | S |
| | GM694+A | A | + | R | R |
| | PD426 | C | - | S | |
| | PD426+C | C | + | R | |
| | FAG326SV | G | - | S | |
| | FAG326SV+G | G | + | R | |
| | PD20F | D | + | S | S |
| | 20-3-15 (+D) | D | + | R | R |
| | NBS (-/-) | NBS | + | S | S |
| | ATM(-/-) | ATM | + | S | S |
| | BRCA1 (-/-) | BRCA1 | + | S | S |

| | | | | | |
|---|---|---|---|---|---|
| 1) The presence of the FA protein complex (FANCA/FANCG/FANCC) was determined as previously described (Garcia-Higuera et al., MCB 19:4866-4873,1999) 2) MMC sensitivity for determined by the XTT assay for lymphoblasts or by the crystal violet assay for fibroblasts. 2) IR/Bleomycin sensitivity was determined by analysis of chromosome breakage (See Materials and Methods) | | | | | |

**Table 6. The Intron/Exon Junctions of FANCD**

| Exon | Size | SEQ ID NO. | 5'-Donor site Score | Intron | | SEQ ID NO. | 3'-Acceptor site | Score | Exon |
|---|---|---|---|---|---|---|---|---|---|
| 1 | 30 | 9 | TCG gtgagtaagtg | 87 | | 52 | gtttcccgattttgctctag GAA | 85 | 2 |
| 2 | 97 | 10 | CCA gtaagtatcta | 83 | | 53 | gaaaatttttctattttcag AAA | 83 | 3 |
| 3 | 141 | 11 | TAG gtaatatttta | 78 | | 54 | ctcttcttttttctgcatag CTG | 88 | 4 |
| 4 | 68 | 12 | AAA gtatgtatttt | 81 | 159 | 55 | attttttaaatctccttaag ATA | 78 | 5 |
| 5 | 104 | 13 | CAG gtgtggagagg | 86 | 375 | 56 | gatttcttttttttttacag TAT | 91 | 6 |
| 6 | 61 | 14 | CAG gtaagactgtc | 89 | | 57 | ccctatgtcttcttttttag CCT | 86 | 7 |
| 7 | 53 | 15 | AAA gtaagtggcgt | 87 | | 58 | ttctcttcctaacattttag CAA | 80 | 8 |
| 8 | 79 | 16 | AAG gtaggcttatg | 83 | 364 | 59 | aatagtgtcttctactgcag GAC | 85 | 9 |
| 9 | 125 | 17 | CAG gtggataaacc | 80 | | 60 | tctttttctaccattcacag TGA | 86 | 10 |
| 10 | 88 | 18 | AAG gtagaaaagac | 76 | | 61 | tctgtgcttttaatttttag GTT | 85 | 11 |
| 11 | 105 | 19 | GAG gtatgctctta | 80 | 387 | 62 | ctaatatttactttctgcag GTA | 87 | 12 |
| 12 | 101 | 20 | AAG gtaaagagctc | 85 | 342 | 63 | ttcctctctgctacttgtag TTC | 84 | 13 |
| 13 | 101 | 21 | AAG gtgagatcttt | 89 | 237 | 64 | actctctcctgttttttcag GCA | 92 | 14 |
| 14 | 36 | 22 | AAG gtaatgttcat | 82 | | 65 | tgcatatttattgacaatag GTG | 73 | 15 |
| 15 | 144 | 23 | TTA gtaagtgtcag | 80 | | 66 | tctactcttccccactcaag GTT | 86 | 16 |
| 16 | 135 | 24 | CAG gtatgttgaaa | 85 | | 67 | gttgactctcccctgtatag GAA | 84 | 17 |
| 17 | 132 | 25 | AAG gtatcttattg | 77 | | 68 | tggcatcattttttccacag GGC | 89 | 18 |
| 18 | 111 | 26 | CAG gttagaggcaa | 83 | | 69 | tcttcatcatctcattgcag GAT | 87 | 19 |
| 19 | 110 | 27 | CAG gtacacgtgga | 82 | | 70 | aaaaaattctttgtttttag AAG | 79 | 20 |
| 20 | 61 | 28 | CAG gtgagttcttt | 93 | | 71 | attcttcctctttgctcaag GTG | 93 | 21 |
| 21 | 120 | 29 | CTG gtaaagccaat | 81 | 445 | 72 | tgtttgtttgcttcctgaag GAA | 85 | 22 |
| 22 | 74 | 30 | AGG gtaggtattgt | 84 | 300 | 73 | attctggtttttctccgcag TGA | 88 | 23 |
| 23 | 147 | 31 | AAA gtcagtatagt | 73 | | 74 | aatttatttctccttctcag ATT | 89 | 24 |
| 24 | 101 | 32 | TAG gtatgggatga | 84 | 370 | 75 | aaatgtttgttctctctcag ATT | 86 | 25 |
| 25 | 116 | 33 | GAG gtgagcagagt | 88 | | 76 | atgtaatttgtactttgcag ATT | 82 | 26 |
| 26 | 109 | 34 | CAG gtaagagaagt | 89 | | 77 | cagactgctgtttgtttcag TCA | 81 | 27 |
| 27 | 111 | 35 | TAG gtaagtatgtt | 90 | 272 | 78 | ttctctttttaatataaaag AAA | 73 | 28 |
| 28 | 110 | 36 | AAG gtattggaatg | 78 | | 79 | ttgctgtgacttccccatag GAG | 85 | 29 |
| 29 | 144 | 37 | GAA gtaagtgacag | 85 | | 80 | tcctttcctccatgtgacag GCT | 84 | 30 |
| 30 | 117 | 38 | AAG gttagtgtagg | 86 | | 81 | taactctgoatttattatag AAC | 80 | 31 |
| 31 | 129 | 39 | CAG gtcagaagcct | 82 | 118 | 82 | aaaatcatttttatttttag TGT | 79 | 32 |
| 32 | 119 | 40 | TTG gtaagtatgtg | 85 | | 83 | tcttaccttgacttccttag GAG | 85 | 33 |
| 33 | 111 | 41 | CAG gtgagtcataa | 90 | | 84 | tttttcttgtctccttacag CCA | 91 | 34 |
| 34 | 131 | 42 | TTG gtgatgggcct | 73 | | 85 | tttgtcttcttttctaacag CTT | 89 | 35 |
| 35 | 94 | 43 | CTG gtgagatgttt | 84 | 286 | 86 | atatttgactctcaatgcag TAT | 78 | 36 |
| 36 | 123 | 44 | CAG gtaagggagtt | 92 | | 87 | atgcttttcccgtcttctag GCA | 88 | 37 |
| 37 | 94 | 45 | CAG gtgagtaagat | 92 | | 88 | catatatttggctgccccag ATT | 81 | 38 |
| 38 | 72 | 46 | AAG gtgagtatgga | 93 | | 89 | cttgtctttcacctctccag GTA | 93 | 39 |
| 39 | 39 | 47 | AAG gtgagagattt | 89 | | 90 | agtgtgtctctcttcttcag TAT | 86 | 40 |
| 40 | 75 | 48 | CGG gtaagagctaa | 86 | | 91 | tataaacttattggttatag GAA | 77 | 41 |
| 41 | 75 | 49 | AAG gtaagaagggg | 91 | | 92 | tgttatttatttccattcag ATT | 86 | 42 |
| 42 | 147 | 50 | CAG gtaagccttgg | 91 | | 93 | cttggtccattcacatttag GGT | 80 | 43 |
| 43 | 228 | | CCA taa + 3'UTR | | | 94 | atttattctttgccccttag GAT | | 44 |
| | 96 | 51 | GAG GTATCTCTACA | | | | | | |
| 44 | 72 | | GAT tag + 3'UTR | | | | | | |

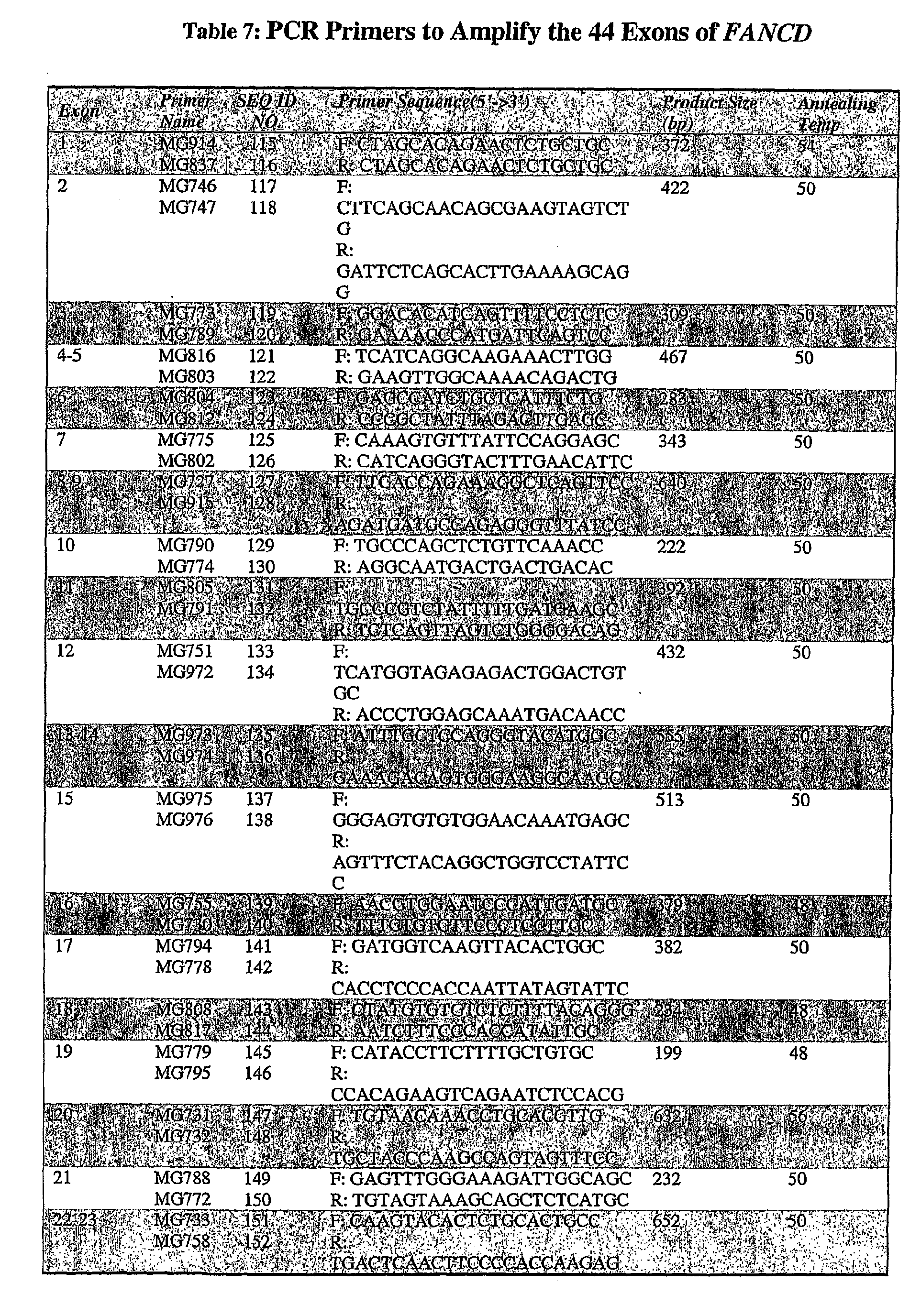

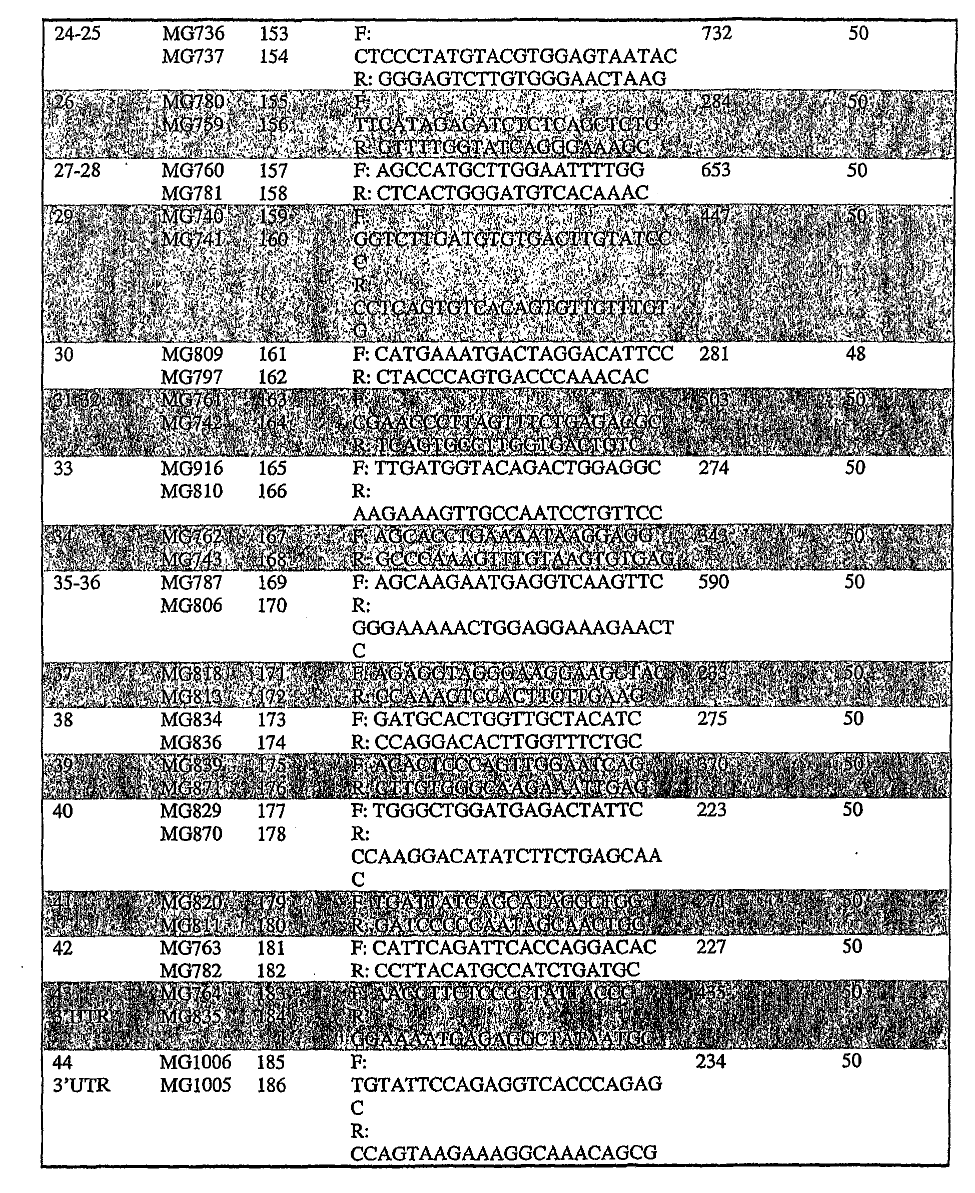

Embodiments of the Invention
1. An isolated nucleic acid molecule, comprising: a polynucleotide selected from
   (a) a nucleotide sequence encoding a polypeptide having an aminoacid sequence as shown in SEQ ID NO: 4
   (b) a nucleotide sequence at least 90% identical to the nucleotide sequence of (a);
   (c) a nucleotide sequence complementary to the nucleotide sequence of (b);
   (d) a nucleotide sequence at least 90% identical to the nucleotide sequence shown in SEQ ID NO: 5-8, 187-188; and
   (e) a nucleotide sequence complementary to the nucleotide sequence of (d).
2. An isolated nucleic acid molecule according to embodiment, wherein the polynucleotide is a DNA molecule
3. An isolated nucleic acid molecule of embodiment 2, wherein the polynucleotide is cDNA.
4. An isolated nucleic acid molecule according to embodiment 1, wherein the polynucleotide is an RNA molecule.
5. An isolated nucleic acid molecule consisting essentially of a nucleotide sequence encoding a polypeptide having an amino acid sequence sufficiently similar to that of SEQ ID NO: 4 to retain the biological property of conversion from a short form to a long form of FANCD2 in the nucleus of a cell for facilitating DNA repair.
6. An isolated nucleic acid molecule consisting essentially of a polynucleotide having a nucleotide sequence at least 90% identical to SEQ ID NO: 9-191 or complementary to a nucleotide sequence that is at least 90% identical to SEQ ID NO: 9-191.
7. An isolated nucleic acid molecule according to embodiment 6, wherein the sequence is an intron/exon sequence selected from SEQ ID NO: 9-94 disclosed in Table 6.
8. An isolated nucleic acid molecule according to embodiment 6, wherein the sequence is a PCR primer selected from SEQ ID NO: 115-186 disclosed in Table 7.
9. A method for making a recombinant vector comprising inserting the isolated nucleic acid molecule of embodiment 1 into a vector.
10. A recombinant vector produced by the method of embodiment.
11. A method of making a recombinant host cell comprising:
   introducing the recombinant vector of embodiment 10 into a host cell.
12. A recombinant host cell produced by the method of embodiment 11.
13. A method of making an FA-D2 cell line, comprising:
   (a) obtaining cells from a subject having a biallelic mutation in a complementation group associated with FA-D2; and
   (b) infecting the cells with a transforming virus to make the FA-D2 cell line.
14. A method according to embodiment 13, wherein the cells are selected from fibroblasts and lymphocytes.
15. A method according to embodiment 13, wherein the transforming virus is selected from Epstein Barr virus and retrovirus.
16. A method according to embodiment 13, further comprising:
   characterizing the FA-D2 cell line by determining the presence of a defective FANDC2.
17. A method according to embodiment 16, wherein characterizing the FA-D2 cell line further comprises:
   performing a diagnostic assay on the cell line, the diagnostic assay selected from (i) a Western blot or nuclear immunofluorescence using an antibody specific for FANCD2 and (ii) a DNA hybridization assay.
18. A recombinant method for producing a polypeptide, comprising:
   culturing a recombinant host cell wherein the host cell comprises the isolated nucleic acid molecule of embodiment 1.
19. An isolated polypeptide, comprising an aminoacid sequence selected from
   (a) SEQ ID NO: 4;
   (b) an aminoacid sequence at least 90% identical to (a);
   (c) an aminoacid sequence which is encoded by a polynucleotide having a nucleotide sequence which is at least 90% identical to at least one of SEQ ID NO: 5-8, 187-188; and
   (d) a polypeptide fragment of (a) ―(d) wherein the fragment is at least 50 aminoacids in length.
20. An isolated polypeptide according to embodiment 19, encoded by a DNA having a mutation selected from nt 376 A to G, nt 3707 G to A, nt904C to T and nt 958C to T.
21. An isolated polypeptide according to embodiment 19, the polypeptide characterized by a polymorphism in DNA encoding the polypeptide, the polymorphism being selected from nt 1122A to G, nt 1440T to C, nt1509C to T, nt2141C to T, nt2259T to C, nt4098T to G, nt4453G to A.
22. An isolated polypeptide according to embodiment 19, the polypeptide characterized by a mutation at aminoacid 222 or aminoacid 561.
23. An antibody preparation having a binding specificity for a FANCD2 protein.
24. An antibody preparation according to embodiment 23, further comprising: monoclonal antibodies.
25. An antibody preparation according to embodiment 23, further comprising: polyclonal antibodies.
26. An antibody preparation according to embodiment 23, wherein the FANCD2 protein is FANCD2-S
27. An antibody preparation according to embodiment 23, wherein the FANCD2 protein is FANCD2-L.
28. A diagnostic method for measuring FANCD2 isoforms in a biological sample, the method comprising:
   (a) exposing the sample to a first antibody for forming a first complex with FANCD2-L and optionally a second antibody for forming a second complex with FANCD2-S ; and
   (b) detecting with a marker, the amount of the first complex and the second complex in the sample.
29. A diagnostic method according to embodiment 28, wherein the sample comprises intact cells.
30. A diagnostic method according to embodiment 28, wherein the sample comprises lysed cells in a lysate.
31. A diagnostic method according to embodiment 28, wherein the biological sample is from a human subject with a susceptibility to cancer or having the initial stages of cancer.
32. A diagnostic method according to embodiment 31, wherein the biological sample is from a cancer in a human subject, wherein the cancer is selected from melanoma, leukemia, astocytoma, glioblastoma, lymphoma, glioma, Hodgkins lymphoma, chronic lymphocyte leukemia and cancer of the pancreas, breast, thyroid, ovary, uterus, testis, pituitary, kidney, stomach, esophagus and rectum.
33. A diagnostic method according to embodiment 28, wherein the biological sample is from a human fetus.
34. A diagnostic method according to embodiment 28, wherein the biological sample is from an adult human.
35. A diagnostic method according to embodiment 28, wherein the biological sample is selected from: a blood sample, a biopsy sample of tissue from the subject and a cell line.
36. A diagnostic method according to embodiment 28, wherein the biological sample is derived from heart, brain, placenta, liver, skeletal muscle, kidney, pancreas, spleen, thymus, prostate, testis, uterus, small intestine, colon, peripheral blood and lymphocytes.
37. A diagnostic method according to embodiment 28, wherein the marker is a fluorescent marker, the fluorescent marker optionally conjugated to the FANCD2-L antibody.
38. A diagnostic method according to embodiment 28, wherein the marker is a chemiluminescent marker, the chemiluminescent marker optionally conjugated to the FANCD2-L antibody.
39. A diagnostic method according to embodiment 28, further comprising: binding the first and the second complex to a third antibody conjugated to a substrate.
40. A diagnostic method according to embodiment 30, wherein the lysate is subjected to a separation procedure to separate FANCD2 isoforms and the seperated isoforms are identified by determining binding to the first or the second FANCD2 antibody.
41. A diagnostic test for identifying a defect in the Fanconi Anemia pathway in a cell population from a subject, comprising:
   selecting an antibody to FANCD2 protein and determining whether the amount of an FAND2-L isoform is reduced in the cell population compared with amounts, in a wild type cell population; such that if the amount of the FANCD2-L protein is reduced, then determining whether an amount of any of FANCA, FANCB, FANCC, FANCD1, FANCE, FANCF or FANCG protein is altered in the cell population compared with the wild type so as to identify the defect in the Fanconi Anemia pathway in the cell population.
42. A diagnostic test according to embodiment 41, wherein determining the amount of an isoform relies on a separation of the FANCD2-L and FANCD2-S isoforms.
43. A diagnostic test according to embodiment 41, wherein the separation is achieved by gel electrophoresis.
44. A diagnostic test according to embodiment 41, wherein the separation is achieved by a migration binding banded test strip.
45. A screening assay for identifying a therapeutic agent, comprising:
   selecting a cell population in which FAND2-L is made in reduced amounts;
   exposing the cell population to individual members of a library of candidate therapeutic molecules; and
   identifying those individual member molecules that cause the amount of FANCD2-L to be increased in the cell population.
46. A screening assay according to embodiment 45, wherein the cell population is an in vitro cell population.
47. A screening assay according to embodiment 45, wherein the cell population is an in vivo cell population, the in vivo population being within an experimental animal, the experimental animal having a mutant FANCD2 gene.
48. A screening assay according to embodiment 45, wherein the experimental animal is a knock-out mouse in which the mouse FAND2 gene has been replaced by a human mutant FANCD2 gene.
49. A screening assay according to embodiment 45, wherein a chemical carcinogen is added to the cell population in which FANCD2 is made in reduced amounts, to determine if any member molecules can cause the amount of FANCD2-L to be increased so as to protect the cells form the harmful effects of the chemical carcinogen.
50. An experimental animal model in which the animal FANCD2 gene has been removed and optionally replaced by a nucleic acid molecule of embodiment 1.
51. A method for identifying in a cell sample from a subject, a mutant FANCD2 nucleotide sequence in a suspected mutant FANCD2 allele which comprises comparing the nucleotide sequence of the suspected mutant FANCD2 allele with the wild type FANCD2 nucleotide sequence wherein a difference between the suspected mutant and the wild type sequence identifies a mutant FANCD2 nucleotide sequence in the cell sample.
52. A method according to embodiment 51, wherein the suspected mutant allele is a germline allele.
53. A method according to embodiment 51, wherein identification of a mutant FANCD2 nucleotide sequence is diagnostic for a predisposition for a cancer in the subject. 54. A method according to embodiment 51, wherein identification of a mutant
54. A method according to embodiment 51, wherein identification of a mutant FANCD2 nucleotide sequence is diagnostic for an increased risk of the subject bearing an offspring with Fanconi Anemia.
55. A method according to embodiment 51, wherein the suspected mutant allele is a somatic allele in a tumor type and identifying a mutant FANCD2 nucleotide sequence is diagnostic for the tumor type.
56. A method according to embodiment 51, wherein the nucleotide sequence of the wild type and the suspected mutant FANCD2 nucleotide sequence is selected from a gene, a mRNA and a cDNA made from a mRNA.
57. A method according to embodiment 51, wherein comparing the polynucleotide sequence of the suspected mutant FANCD2 allele with the wild type FANCD2 polynucleotide sequence, further comprises: selecting a FANCD2 probe which specifically hybridizes to the mutant FANCD2 nucleotide sequence, and detecting the presence of the mutant sequence by hybridization with the probe.
58. A method according to embodiment 51, wherein comparing the polynucleotide sequence of the suspected mutant FANCD2 allele with the wild type FANCD2 polynucleotide sequence, further comprises amplifying all or part of the FANCD2 gene using a set of primers specific for wild type FANCD2 DNA to produce amplified FANCD2 DNA and sequencing the FANCD2 DNA so as to identify the mutant sequence.
59. A method according to embodiment 51, wherein the mutant FANCD2 nucleotide sequence is a germline alteration in the FANCD2 allele of the human subject, the alteration selected from the alterations set forth in Table 3.
60. A method according to embodiment 51, wherein the mutant FANCD2 nucleotide sequence is a somatic alteration in the FANCD2 allele of the human subject, the alteration selected from the alterations set forth in Table 3.
61. A method for diagnosing a susceptibility to cancer in a subject which comprises comparing the germline sequence of the FANCD2 gene or the sequence of its mRNA in a tissue sample from the subject with the germline sequence of the FANCD2 gene or the sequence of its mRNA wherein an alteration in the germline sequence of the FANCD2 gene or the sequence of its mRNA of the subject indicates the susceptibility to the cancer.
62. A method according to embodiment 61, wherein an alteration is detected in a regulatory region of the FANCD2 gene.
63. A method according to embodiment 61, wherein the detection in the alteration in the germline sequence is determined by an assay selected from the group consisting of (a) observing shifts in electrophoretic mobility of single-stranded DNA on non-denaturing polyacrylamide gels, (b) hybridizing a FANCD2 gene probe to genomic DNA isolated from the tissue sample, (c) hybridizing an allele-specific probe to genomic DNA of the tissue sample, (d) amplifying all or part of the FANCD2 gene from the tissue sample to produce an amplified sequence and sequencing the amplified sequence, (e) amplifying all or part of the FANCD2 gene from the tissue sample using primers for a specific FANCD2 mutant allele, (f) molecular cloning all or part of the FANCD2 gene from the tissue sample to produce a cloned sequence and sequencing the cloned sequence, (g) identifying a mismatch between (i) a FANCD2 gene or a FANCD2 mRNA isolated from the tissue sample, and (ii) a nucleic acid probe complementary to the human wild-type FANCD2 gene sequence, when molecules (i) and (ii) are hybridized to each other to form a duplex, (h) amplification of FANCD2 gene sequences in the tissue sample and hybridization of the amplified sequences to nucleic acid probes which comprise wild-type FANCD2 gene sequences, (I) amplification of FANCD2 gene sequences in the tissue sample and hybridization of the amplified sequences to nucleic acid probes which comprise mutant FANCD2 gene sequences, (j) screening for a deletion mutation in the tissue sample, (k) screening for a point mutation in the tissue sample, (1) screening for an insertion mutation in the tissue sample, (m) in situ hybridization of the FANCD2 gene of the tissue sample with nucleic acid probes which comprise the FANCD2 gene.
64. A method of diagnosing a susceptibility for cancer in a subject, comprising:
   (a) accessing genetic material from the subject so as to determine defective DNA repair;
   (b) determining the presence of mutations in a set of genes, the set comprising FAND2 and at least one of FANCA, FANCB, FANCC, FANCD1, FANCDE, FANDF, FANDG, BRACA1 and ATM; and
   (c) diagnosing susceptibility for cancer from the presence of mutations in the set of genes.
65. A method for detecting a mutation in a neoplastic lesion at the FANCD2 gene in a human subject which comprises:
   comparing the sequence of the FANCD2 gene or the sequence of its mRNA in a tissue sample from a lesion of the subject with the sequence of the wild-type FANCD2 gene or the sequence of its mRNA, wherein an alteration in the sequence of the FANCD2 gene or the sequence of its mRNA of the subject indicates a mutation at the FANCD2 gene of the neoplastic lesion.
66. A method according to embodiment 65, further comprising:
   determining a therapeutic protocol for treating the neoplastic lesion according to the mutation at the FANCD2 gene of the neoplastic lesion.
67. A method for confirming the lack of a FANCD2 mutation in a neoplastic lesion from a human subject which comprises comparing the sequence of the FANCD2 gene or the sequence of its mRNA in a tissue sample from a lesion of said subject with the sequence of the wild-type FANCD2 gene or the sequence of its RNA, wherein the presence of the wild-type sequence in the tissue sample indicates the lack of a mutation at the FANCD2 gene.
68. A method for determining a therapeutic protocol for a subject having a cancer, comprising:
   (a) determining if a deficiency in FANCD2-L occurs in a cell sample from the subject by measuring FANCD2 isoforms according to claim 25;
   (b) if a deficiency is detected in (a) , then determining whether the deficiency is a result of genetic defect in non-cancer cells; and
   (c) if (b) is positive, reducing the use of a therapeutic protocol that causes increased DNA damage so as to protect normal tissue in the subject and if (b) is negative, and the deficiency is contained within a genetic defect in cancer cells only, then increasing the use of a therapeutic protocol that causes increased DNA damage so as to adversely affect the cancer cells.
69. A method of treating a FA pathway defect in a cell target, comprising:
   administering an effective amount of FANCD2 protein or an exogenous nucleic acid to the target.
70. A method according to embodiment 69, wherein the FA pathway defect is a defective FANCD2 gene and the exogenous nucleic acid vector further comprises introducing a vector according to embodiment 10.
71. A method according to embodiment 69, wherein the vector is selected from a mutant herpesvirus, a E1/E4 deleted recombinant adenovirus, a mutant retrovirus, the viral vector being defective in respect of a viral gene essential for production of infectious new virus particles.
72. A method according to embodiment 69, wherein the vector is contained in a lipid micelle.
73. A method for treating a patient with a defective FANCD2 gene, comprising:
   providing a polypeptide described in SEQ ID No: 4, for functionally correcting a defect arising from a condition arising from the defective FANCD2 gene.
74. A cell based assay for detecting a FA pathway defect, comprising:
   (a) obtaining a cell sample from a subject;
   (b) exposing the cell sample to DNA damaging agents; and
   (c) detecting whether FANCD2-L is upregulated, the absence of upregulation being indicative of the FA pathway defect
75. A cell based assay according to embodiment 74, wherein amounts of FANCD2 are measured by an analysis technique selected from: immunoblotting for detecting nuclear foci; Western blots to detect amounts of FANCD2 isoforms and quantifying mRNA by hybridising with DNA probes.
76. A kit for use in detecting a cancer cell in a biological sample, comprising:
   (a) a primer pair which binds under high stringency conditions to a sequence in the FANCD2 gene, the primer pair being selected to specifically amplify an altered nucleic acid sequence described in Table 7; and
   (b) containers for each of the primers.

## Claims

1. An isolated monoubiquitinated FANCD-2 polypeptide comprising an amino acid sequence selected from:
(a) an amino acid sequence of SEQ ID NO: 4, and
(b) an amino acid sequence encoded by a nucleotide sequence at least 90% identical to the nucleotide sequence of any one of SEQ ID NOs: 5-8 and 187-188,
wherein the monoubiquitinated polypeptide is ubiquitinated at lysine 561.

2. A method for identifying a defect in the Fanconi Anemia pathway in a cell population from a subject, comprising:
a) determining in vitro whether the amount of a monoubiquitinated polypeptide is reduced in the cell population compared with the amount of the monoubiquitinated polypeptide in a wild-type cell population; and
b) if the amount of the monoubiquitinated polypeptide is reduced, determining whether the amount of any of FANCA, FANCB, FANCC, FANCD1, FANCE, FANCF or FANCG protein is altered in the cell population compared with the wild-type cell population,
wherein the monoubiquitinated polypeptide is the monoubiquitinated polypeptide of claim 1.

3. A screening assay for identifying a therapeutic agent, comprising:
i) selecting a cell population in which a monoubiquitinated polypeptide is made in a reduced amount,;
ii) exposing the cell population to individual members of a library of candidate therapeutic molecules; and
iii) identifying those individual member molecules that cause the amount of the monoubiquitinated polypeptide to increase or decrease,
wherein the monoubiquitinated polypeptide is the monoubiquitinated polypeptide of claim 1.

4. The screening assay according to claim 3, wherein the cell population is an in vitro cell population or an in vivo cell population having a mutant FANCD2 gene.

5. The screening assay according to claim 3, wherein a chemical carcinogen is added to the cell population in which the monoubiquitinated polypeptide is made in reduced amounts to determine if any individual member molecules can cause the amount of the monoubiquitinated polypeptide to increase so as to protect the cells from the harmful effects of the chemical carcinogen.

6. A method for identifying a cell having a mutant FANCD2 allele from a subject, comprising:
(a) determining the amount of a monoubiquitinated polypeptide in the cell; and
(b) comparing the amount in (a) with the amount of the monoubiquitinated polypeptide in a wild-type cell,
wherein the monoubiquitinated polypeptide is the monoubiquitinated polypeptide of claim 1, and wherein a reduction of the amount in (a) compared with the amount in (b) indicates that the cell has a mutant FANCD2 allele.

7. The method according to claim 6, wherein the mutant FANCD2 allele is a somatic allele in a tumor type and identifying the cell having the mutant FANCD2 allele is diagnostic for the tumor type.

8. The method according to claim 6, wherein the mutant FANCD2 allele is a germline allele.

9. The method according to claim 6, wherein identification of the cell having a mutant FANCD2 allele is indicative of a predisposition for a cancer in the subject or an increased risk of the subject bearing an offspring with Fanconi Anemia.

10. A method for determining a therapeutic protocol for a subject having a cancer, comprising determining the amount of monoubiquitinated polypeptide in cancer cells from the subject, comprising:
(a) determining if the amount of the monoubiquitinated polypeptide in the cancer cells is reduced relative to the amount present in noncancerous cells from the subject;
(b) if a reduction is detected in (a), determining whether the reduction is a result of the same genetic defect present in both the cancer cells and the noncancerous cells; and
(c) if (b) is positive, reducing the use of the therapeutic protocol that causes increased DNA damage, or
(d) if (b) is negative, increasing the use of the therapeutic protocol that causes increased DNA damage,
wherein the monoubiquitinated polypeptide is the monoubiquitinated polypeptide of claim 1.

11. Use of the monoubiquitinated FANCD-2 polypeptide of claim 1 in the manufacture of a medicament for the treatment of a FA pathway defect in a cell target.

12. A monoubiquitinated FANCD-2 polypeptide of claim 1, for use in the treatment of a FA pathway defect in a cell target.

13. A cell based assay for detecting a FA pathway defect, comprising:
(a) obtaining a cell sample from a subject;
(b) exposing the cell sample to DNA damaging agents; and
(c) detecting whether the amount of the monoubiquitinated polypeptide of claim 1 is upregulated,
wherein the absence of upregulation is indicative of a FA pathway defect.

14. The cell based assay according to claim 13, wherein the amount of monoubiquitinated polypeptide is measured by an analysis technique selected from:
immunohistochemitry for detecting the monoubiquitinated polypeptide in nuclear foci and Western blots to detect amounts of monoubiquitinated polypeptide.


SEQUENCE LISTING

<110> Dana-Farber Cancer Institute

<120> Methods and Compositions for the Diagnosis of Cancer
      Susceptibilities and Defective DNA Repair Mechanisms
      and Treatment Thereof

<130> 07023/2058 (formerly 2486/107 WO)

<140> PCT/US01/45561
<141> 2001-11-02

<150> US 60/245,756
<151> 2000-11-03

<160> 191

<170> FastSEQ for Windows Version 4.0

<210> 1
<211> 1451
<212> PRT
<213> Homo sapiens

<220>
<221> PEPTIDE
<222> (1)...(1451)
<223> Human fancd2

<400> 1
Met Val Ser Lys Arg Arg Leu Ser Lys Ser Glu Asp Lys Glu Ser Leu
 1               5                  10                  15
Thr Glu Asp Ala Ser Lys Thr Arg Lys Gln Pro Leu Ser Lys Lys Thr
            20                  25                  30
Lys Lys Ser His Ile Ala Asn Ala Val Glu Glu Asn Asp Ser Ile Phe
        35                  40                  45
Val Lys Leu Leu Lys Ile Ser Gly Ile Ile Leu Lys Thr Gly Glu Ser
    50                  55                  60
Gln Asn Gln Leu Ala Val Asp Gln Ile Ala Phe Gln Lys Lys Leu Phe
65                  70                  75                  80
Gln Thr Leu Arg Arg His Pro Ser Tyr Pro Lys Ile Ile Glu Glu Phe
                85                  90                  95
Val Ser Gly Leu Glu Ser Tyr Ile Glu Asp Glu Asp Ser Phe Arg Asn
            100                 105                 110
Cys Leu Leu Ser Cys Glu Arg Leu Gln Asp Glu Glu Ala Ser Met Gly
        115                 120                 125
Ala Ser Tyr Ser Lys Ser Leu Ile Lys Leu Leu Leu Gly Ile Asp Ile
    130                 135                 140
Leu Gln Pro Ala Ile Ile Lys Thr Leu Phe Glu Lys Leu Pro Glu Tyr
145                 150                 155                 160
Phe Phe Glu Asn Arg Asn Ser Asp Glu Ile Asn Ile Phe Arg Leu Ile
                165                 170                 175
Val Ser Gln Leu Lys Trp Leu Asp Arg Val Val Asp Gly Lys Asp Leu
            180                 185                 190
Thr Thr Lys Ile Met Gln Leu Ile Ser Ile Ala Pro Glu Asn Leu Gln
        195                 200                 205
His Asp Ile Ile Thr Ser Lys Pro Glu Ile Leu Gly Asp Ser Gln His
    210                 215                 220
Ala Asp Val Gly Lys Glu Leu Ser Asp Leu Leu Ile Glu Asn Thr Ser
225                 230                 235                 240
Leu Thr Val Pro Ile Leu Asp Val Leu Ser Ser Leu Arg Leu Asp Pro
                245                 250                 255
Asn Phe Leu Leu Lys Val Arg Gln Leu Val Met Asp Lys Leu Ser Ser
            260                 265                 270
Ile Arg Leu Glu Asp Leu Pro Val Ile Ile Lys Phe Ile Leu His Ser
        275                 280                 285
Val Thr Ala Met Asp Thr Leu Glu Val Ile Ser Glu Leu Arg Glu Lys

290                 295                 300
Leu Asp Leu Gln His Cys Val Leu Pro Ser Arg Leu Gln Ala Ser Gln
305                 310                 315                 320
Val Lys Leu Lys Ser Lys Gly Arg Ala Ser Ser Ser Gly Asn Gln Glu
                325                 330                 335
Ser Ser Gly Gln Ser Cys Ile Ile Leu Leu Phe Asp Val Ile Lys Ser
            340                 345                 350
Ala Ile Arg Tyr Glu Lys Thr Ile Ser Glu Ala Trp Ile Lys Ala Ile
        355                 360                 365
Glu Asn Thr Ala Ser Val Ser Glu His Lys Val Phe Asp Leu Val Met
    370                 375                 380
Leu Phe Ile Ile Val Ser Thr Asn Thr Gln Thr Lys Lys Tyr Ile Asp
385                 390                 395                 400
Arg Val Leu Arg Asn Lys Ile Arg Ser Gly Cys Ile Gln Glu Gln Leu
                405                 410                 415
Leu Gln Ser Thr Phe Ser Val His Tyr Leu Val Leu Lys Asp Met Cys
            420                 425                 430
Ser Ser Ile Leu Ser Leu Ala Gln Ser Leu Leu His Ser Leu Asp Gln
        435                 440                 445
Ser Ile Ile Ser Phe Gly Ser Leu Leu Tyr Lys Tyr Ala Phe Lys Phe
    450                 455                 460
Phe Asp Thr Tyr Cys Gln Gln Glu Val Val Gly Ala Leu Val Thr His
465                 470                 475                 480
Ile Cys Ser Gly Asn Glu Ala Glu Val Asp Asp Ala Leu Asp Val Leu
                485                 490                 495
Leu Glu Leu Val Val Leu Asn Pro Ser Ala Met Met Met Asn Ala Val
            500                 505                 510
Phe Val Gln Gly Ile Leu Asp Tyr Leu Asp Asn Ile Ser Pro Gln Gln
        515                 520                 525
Ile Arg Lys Leu Phe Tyr Val Leu Ser Thr Leu Ala Phe Ser Lys Gln
    530                 535                 540
Asn Glu Ala Ser Ser His Ile Gln Asp Asp Met His Leu Val Ile Arg
545                 550                 555                 560
Lys Gln Leu Ser Ser Thr Val Phe Lys Tyr Lys Leu Ile Gly Ile Ile
                565                 570                 575
Gly Ala Val Thr Met Ala Gly Ile Met Ala Ala Asp Arg Ser Glu Ser
            580                 585                 590
Pro Ser Leu Thr Gln Glu Arg Ala Asn Leu Ser Asp Glu Gln Cys Thr
        595                 600                 605
Gln Val Thr Ser Leu Leu Gln Leu Val His Ser Cys Ser Glu Gln Ser
    610                 615                 620
Pro Gln Ala Ser Ala Leu Tyr Tyr Asp Glu Phe Ala Asn Leu Ile Gln
625                 630                 635                 640
His Glu Lys Leu Asp Pro Lys Ala Leu Glu Trp Val Gly His Thr Ile
                645                 650                 655
Cys Asn Asp Phe Gln Asp Ala Phe Val Val Asp Ser Cys Val Val Pro
            660                 665                 670
Glu Gly Asp Phe Pro Phe Pro Val Lys Ala Leu Tyr Gly Leu Glu Glu
        675                 680                 685
Tyr Asp Thr Gln Asp Gly Ile Ala Ile Asn Leu Leu Pro Leu Leu Phe
    690                 695                 700
Ser Gln Asp Phe Ala Lys Asp Gly Gly Pro Val Thr Ser Gln Glu Ser
705                 710                 715                 720
Gly Gly Lys Leu Val Ser Pro Leu Cys Leu Ala Pro Tyr Phe Arg Leu
                725                 730                 735
Leu Arg Leu Cys Val Glu Arg Gln His Asn Gly Asn Leu Glu Glu Ile
            740                 745                 750
Asp Gly Leu Leu Asp Cys Pro Ile Phe Leu Thr Asp Leu Glu Pro Gly
        755                 760                 765
Glu Lys Leu Glu Ser Met Ser Ala Lys Glu Ala Ser Phe Met Cys Ser
    770                 775                 780
Leu Ile Phe Leu Thr Leu Asn Trp Phe Arg Glu Ile Val Asn Ala Phe
785                 790                 795                 800
Cys Gln Glu Thr Ser Pro Glu Asn Lys Gly Lys Val Leu Thr Arg Leu
                805                 810                 815
Lys His Ile Val Glu Leu Gln Ile Leu Leu Glu Lys Tyr Leu Ala Val
            820                 825                 830
Thr Pro Asp Tyr Val Pro Pro Leu Gly Asn Phe Asp Val Glu Thr Leu

835                 840                 845
Asp Ile Thr Pro His Thr Val Thr Ala Ile Ser Ala Lys Ile Arg Lys
    850                 855                 860
Lys Gly Lys Ile Glu Arg Lys Gln Lys Thr Asp Gly Ser Lys Thr Ser
865                 870                 875                 880
Ser Ser Asp Thr Leu Ser Glu Glu Lys Asn Ser Glu Cys Asp Pro Thr
                885                 890                 895
Pro Ser His Arg Gly Gln Leu Asn Lys Glu Phe Thr Gly Lys Glu Glu
            900                 905                 910
Lys Thr Ser Leu Leu Leu His Asn Ser His Ala Phe Phe Arg Glu Leu
        915                 920                 925
Asp Ile Glu Val Phe Ser Ile Leu His Cys Gly Leu Val Thr Lys Phe
    930                 935                 940
Ile Leu Asp Thr Glu Met His Thr Glu Ala Thr Glu Val Val Gln Leu
945                 950                 955                 960
Gly Pro Pro Glu Leu Leu Phe Leu Leu Glu Asp Leu Ser Gln Lys Leu
                965                 970                 975
Glu Ser Met Leu Thr Pro Pro Ile Ala Arg Arg Val Pro Phe Leu Lys
            980                 985                 990
Asn Lys Gly Ser Arg Asn Ile Gly Phe Ser His Leu Gln Gln Arg Ser
        995                 1000                1005
Ala Gln Glu Ile Val His Cys Val Glu Gln Leu Leu Thr Pro Met Cys
    1010                1015                1020
Asn His Leu Glu Asn Ile His Asn Tyr Ile Gln Cys Leu Ala Ala Glu
1025                1030                1035                1040
Asn His Gly Val Val Asp Gly Pro Gly Val Lys Val Gln Glu Tyr His
                1045                1050                1055
Ile Met Ser Ser Cys Tyr Gln Arg Leu Leu Gln Ile Phe His Gly Leu
            1060                1065                1070
Phe Ala Trp Ser Gly Phe Ser Gln Pro Glu Asn Gln Asn Leu Leu Tyr
        1075                1080                1085
Ser Ala Leu His Val Leu Ser Ser Arg Leu Lys Gln Gly Glu His Ser
    1090                1095                1100
Gln Pro Leu Glu Glu Leu Leu Ser Gln Ser Val His Tyr Leu Gln Asn
1105                1110                1115                1120
Phe His Gln Ser Ile Pro Ser Phe Gln Cys Ala Leu Tyr Leu Ile Arg
                1125                1130                1135
Leu Leu Met Val Ile Leu Glu Lys Ser Thr Ala Ser Ala Gln Asn Lys
            1140                1145                1150
Glu Lys Ile Ala Ser Leu Ala Arg Gln Phe Leu Cys Arg Val Trp Pro
        1155                1160                1165
Ser Gly Asp Lys Glu Lys Ser Asn Ile Ser Asn Asp Gln Leu His Ala
    1170                1175                1180
Leu Leu Cys Ile Tyr Leu Glu His Thr Glu Ser Ile Leu Lys Ala Ile
1185                1190                1195                1200
Glu Glu Ile Ala Gln Val Gly Val Pro Glu Leu Ile Asn Ser Pro Lys
                1205                1210                1215
Asp Ala Ser Ser Ser Thr Phe Pro Thr Leu Thr Arg His Thr Pro Val
            1220                1225                1230
Val Phe Phe Arg Val Met Met Ala Glu Leu Glu Lys Ile Val Lys Lys
        1235                1240                1245
Ile Glu Pro Gly Thr Ala Ala Asp Ser Gln Gln Ile His Glu Glu Lys
    1250                1255                1260
Leu Leu Tyr Trp Asn Met Ala Val Arg Asp Phe Ser Ile Leu Ile Asn
1265                1270                1275                1280
Leu Ile Lys Val Phe Asp Ser His Pro Val Leu His Val Cys Leu Lys
                1285                1290                1295
Val Gly Arg Leu Phe Val Glu Ala Phe Leu Lys Gln Cys Met Pro Leu
            1300                1305                1310
Leu Asp Ile Ser Phe Arg Lys His Arg Glu Asp Val Leu Ser Leu Leu
        1315                1320                1325
Glu Thr Phe Gln Leu Asp Thr Arg Leu Leu His His Leu Cys Gly His
    1330                1335                1340
Ser Lys Ile His Gln Asp Thr Arg Leu Thr Gln His Val Pro Leu Leu
1345                1350                1355                1360
Lys Lys Thr Leu Glu Leu Leu Val Cys Arg Val Lys Ala Met Leu Thr
                1365                1370                1375
Leu Asn Asn Cys Arg Glu Ala Phe Trp Leu Gly Asn Leu Lys Asn Arg

1380                    1385                    1390
Asp Leu Gln Gly Glu Glu Ile Lys Ser Gln Asn Ser Gln Glu Ser Thr
        1395                    1400                    1405
Ala Asp Glu Ser Glu Asp Asp Met Ser Ser Gln Ala Ser Lys Ser Lys
    1410                    1415                    1420
Ala Thr Glu Asp Gly Glu Glu Asp Glu Val Ser Ala Gly Glu Lys Glu
1425                    1430                    1435                    1440
Gln Asp Ser Asp Glu Ser Tyr Asp Asp Ser Asp
                1445                    1450

<210> 2
<211> 1269
<212> PRT
<213> Drosophila melanogaster

<220>
<221> PEPTIDE
<222> (1)...(1269)
<223> Drosophila fancd2

<400> 2
Met Tyr Lys Gln Phe Lys Lys Arg Ser Lys Lys Pro Leu Asn Thr Ile
 1               5                  10                  15
Asp Glu Asn Ala Thr Ile Lys Val Pro Arg Leu Ala Glu Thr Thr Thr
            20                  25                  30
Asn Ile Ser Val Glu Ser Ser Ser Gly Gly Ser Glu Glu Asn Ile Pro
        35                  40                  45
Ala Ser Gln Glu His Thr Gln Arg Phe Leu Ser Gln His Ser Val Ile
    50                  55                  60
Leu Ala Ala Thr Leu Gly Ala Thr Gly Glu Ser Ser Arg Asp Ile Ala
65                  70                  75                  80
Thr Leu Ser Arg Gln Pro Asn Asn Phe Phe Glu Leu Val Leu Val Arg
                85                  90                  95
Ala Gly Val Gln Leu Asp Gln Gly Asp Ser Leu Ile Leu Ala Cys Asp
            100                 105                 110
His Val Pro Ile Val Ser Lys Leu Ala Glu Ile Phe Thr Ser Ala Ser
        115                 120                 125
Ser Tyr Thr Asp Lys Met Glu Thr Phe Lys Thr Gly Leu Asn Ala Ala
    130                 135                 140
Met Ala Pro Gly Ser Lys Leu Val Gln Lys Leu Leu Thr Gly Cys Thr
145                 150                 155                 160
Val Asp Ala Ala Gly Glu Glu Gln Ile Tyr Gln Ser Gln Asn Ser Met
                165                 170                 175
Phe Met Asn Phe Leu Met Ile Asp Phe Met Arg Asp Ala Cys Val Glu
            180                 185                 190
Val Leu Leu Asn Lys Ile Glu Glu Val Ala Lys Ser Asp Arg Val Ile
        195                 200                 205
Met Gly Lys Ala Ala Ile Pro Leu Pro Leu Leu Pro Leu Met Leu Thr
    210                 215                 220
Gln Leu Arg Tyr Leu Thr Ala Ser His Lys Val Glu Ile Tyr Ser Arg
225                 230                 235                 240
Ile Glu Val Ile Phe Asn Arg Ala Thr Glu Ser Ala Lys Leu Asp Ile
                245                 250                 255
Ile Ala Asn Ala Glu Leu Ile Leu Asp Ala Ser Met His Asp Glu Phe
            260                 265                 270
Val Glu Leu Leu Asn Thr Glu Asp Leu Phe His Met Thr Thr Val Gln
        275                 280                 285
Thr Leu Gly Asn Leu Ser Leu Ser Asp Arg Thr Gln Ala Lys Leu Arg
    290                 295                 300
Val Arg Ile Leu Asp Phe Ala Thr Ser Gly Gln Cys Ser Asp Ala Ile
305                 310                 315                 320
Leu Pro His Leu Ile Arg Leu Leu Leu Asn Val Leu Lys Ile Asp Thr
                325                 330                 335
Asp Asp Ser Val Arg Asp Leu Arg Arg Arg Ile Lys Leu Glu His
            340                 345                 350
Ile Thr Val Ser Ile Leu Glu Glu Ile Gln His Tyr Arg His Ile Leu
        355                 360                 365
Glu Gln His Ile Thr Thr Leu Met Asn Ile Leu His Asp Phe Met Arg

370                 375                 380
Glu Lys Asn Arg Ile Val Ser Asp Phe Ala Lys Ser Ser Tyr Ser Ile
385                 390                 395                 400
Leu Phe Lys Ile Phe Asn Ser Ile Gln Lys Asn Ile Leu Lys Lys Leu
                405                 410                 415
Leu Glu Leu Thr Cys Asp Lys Ser Ser Pro His Leu Thr Thr His Ala
            420                 425                 430
Leu Glu Leu Leu Arg Glu Leu Gln Arg Lys Ser Ala Lys Asp Val Gln
        435                 440                 445
Asn Cys Ala Thr Leu Leu Ile Pro Met Leu Asp Arg Thr Ser Asp Leu
    450                 455                 460
Ser Leu Thr Gln Thr Arg Val Ala Met Asp Leu Leu Cys His Val Ala
465                 470                 475                 480
Phe Pro Asp Pro Asn Leu Ser Pro Cys Leu Gln Leu Gln Glu Gln Val
                485                 490                 495
Asp Met Val Val Lys Lys Gln Leu Ile Asn Ser Ile Asp Asn Ile Lys
            500                 505                 510
Lys Gln Gly Ile Ile Gly Cys Val Gln Leu Ile Asp Ala Met Ala Arg
        515                 520                 525
Ile Ala Asn Asn Gly Val Asp Arg Asp Phe Phe Ile Ala Ser Val Glu
    530                 535                 540
Asn Val Asp Ser Leu Pro Asp Gly Arg Gly Lys Met Ala Ala Asn Leu
545                 550                 555                 560
Ile Ile Arg Thr Glu Ala Ser Ile Gly Asn Ser Thr Glu Ser Leu Ala
                565                 570                 575
Leu Phe Phe Glu Glu Leu Ala Thr Val Phe Asn Gln Arg Asn Glu Gly
            580                 585                 590
Thr Ser Gly Cys Glu Leu Asp Asn Gln Phe Ile Ala Trp Ala Cys Asp
        595                 600                 605
Leu Val Thr Phe Arg Phe Gln Ala Ser Phe Val Thr Glu Asn Val Pro
    610                 615                 620
Glu Thr Lys Ala Cys Asp Ser Ile Tyr Val Leu Ala Pro Leu Phe Asn
625                 630                 635                 640
Tyr Val Arg Val Leu Tyr Lys His Arg His Gln Asp Ser Leu Glu Ser
                645                 650                 655
Ile Asn Ala Leu Leu Gly Cys Ala Ile Val Leu Pro Ser Phe Phe Glu
            660                 665                 670
Asp Asp Asn Tyr Val Ser Val Phe Glu Asn Phe Glu Ala Glu Gln Gln
        675                 680                 685
Lys Asp Ile Leu Ser Ile Tyr Phe His Thr Val Asn Trp Met Arg Val
    690                 695                 700
Ser Ile Ser Ala Phe Ala Ser Gln Arg Asp Pro Pro Thr Arg Arg Arg
705                 710                 715                 720
Val Leu Ser Arg Leu Gly Glu Leu Ile Arg Ile Glu Gln Arg Met Lys
                725                 730                 735
Pro Leu Leu Ala Arg Ala Pro Val Asp Phe Val Ala Pro Pro Tyr Gln
            740                 745                 750
Phe Leu Thr Asn Val Lys Leu Ser Asn Gln Asn Gln Lys Arg Pro Gly
        755                 760                 765
Pro Lys Pro Ala Ala Lys Leu Asn Ala Thr Leu Pro Glu Pro Asp Leu
    770                 775                 780
Thr Gly Asn Gln Pro Ser Ile Ala Asp Phe Thr Ile Lys Val Gly Gln
785                 790                 795                 800
Cys Lys Thr Val Lys Thr Lys Thr Asp Phe Glu Gln Met Tyr Gly Pro
                805                 810                 815
Arg Glu Arg Tyr Arg Pro Met Glu Val Glu Ile Ile Met Leu Leu Val
            820                 825                 830
Glu Gln Lys Phe Val Leu Asn His Gln Leu Glu Glu Glu Gln Met Gly
        835                 840                 845
Glu Phe Leu Gly Leu Leu Glu Leu Arg Phe Leu Leu Glu Asp Val Val
    850                 855                 860
Gln Lys Leu Glu Ala Ala Val Leu Arg His His Asp Ser Tyr Asp Ala
865                 870                 875                 880
Asp Ser Phe Arg Pro His Leu Ala Lys Pro Glu Asp Phe Ile Cys Asp
                885                 890                 895
Leu Leu Pro Cys Leu His Glu Val Asn Asn His Leu Ile Thr Leu Gly
            900                 905                 910
Glu Ala Ile Asp Asn Gln Leu Thr Glu Val Ser Ser His Val Tyr Ser

915                         920                         925
Asn Leu Asp Leu Phe Lys Asp Gln Phe Cys Tyr Ile Lys Ser Cys Phe
    930                         935                         940
Gly Leu Cys Val Arg Leu Phe Ala Leu Tyr Phe Ala Trp Ser Glu Trp
945                         950                         955                         960
Ser Asp Lys Ser Gln Glu Gln Leu Leu His Arg Ile Leu Cys Gly Thr
                965                         970                         975
Leu Leu Arg Arg Lys Trp Phe His Tyr Ser Gly Thr Leu Asp Lys Gly
            980                         985                         990
Gly Gln Cys Asn Ile Tyr Leu Asp Glu Leu Val Lys Gly Phe Leu Lys
        995                         1000                        1005
Lys Ser Asn Ala Lys Ser Gln Thr Glu Leu Leu Thr Glu Leu Val Lys
    1010                        1015                        1020
Gln Cys Ser Ile Leu Asn Thr Lys Asp Lys Ala Leu Thr Ser Phe Pro
1025                        1030                        1035                        1040
Asn Phe Lys Lys Ala Asn Phe Pro Leu Leu Phe Arg Gly Leu Cys Glu
                1045                        1050                        1055
Val Leu Ile His Ser Leu Ser Gly Gln Val Ser Val Asp Ser Arg Gly
            1060                        1065                        1070
Asp Lys Leu Lys Leu Trp Glu Ser Ala Val Asp Leu Leu Asn Gly Leu
        1075                        1080                        1085
Leu Ser Ile Val Gln Gln Val Glu Gln Pro Arg Asn Phe Gly Leu Phe
    1090                        1095                        1100
Leu Lys His Ser Leu Leu Phe Leu Lys Leu Leu Leu Gln His Gly Met
1105                        1110                        1115                        1120
Ser Ala Leu Glu Ser Ile Val Arg Glu Asp Pro Glu Arg Leu Thr Arg
                1125                        1130                        1135
Phe Leu His Glu Leu Gln Lys Val Thr Arg Phe Leu His Gln Leu Cys
            1140                        1145                        1150
Cys His Ser Lys Ser Ile Lys Asn Thr Ala Ile Ile Ser Tyr Ile Pro
        1155                        1160                        1165
Ser Leu Arg Glu Thr Ile Glu Thr Leu Val Phe Arg Val Lys Ala Leu
    1170                        1175                        1180
Leu Ala Ala Asn Asn Cys His Ser Ala Phe His Met Gly Asn Met Ile
1185                        1190                        1195                        1200
Asn Arg Asp Leu His Gly Asp Ser Ile Ile Thr Pro Arg Ser Ser Phe
                1205                        1210                        1215
Ala Gly Glu Glu Asn Ser Asp Asp Glu Leu Pro Ala Asp Asp Thr Ser
            1220                        1225                        1230
Val Asp Glu Thr Val Leu Gly Asp Asp Met Gly Ile Thr Ala Val Ser
        1235                        1240                        1245
Val Ser Thr Arg Pro Ser Asp Gly Ser Arg Arg Ser Lys Ser Ser Ser
    1250                        1255                        1260
Arg Ser Lys Cys Phe
    1265

<210> 3
<211> 1286
<212> PRT
<213> Arabidopsis thaliana

<220>
<221> PEPTIDE
<222> (1)...(1286)
<223> Arabidopsis fancd2

<400> 3
Met Val Phe Leu Ser Arg Lys Lys Pro Pro Pro Pro Pro Ser Ser Ser
 1               5                   10                  15
Ser Ala Ala Pro Ser Leu Lys Ile Pro Gln Pro Gln Lys Glu Ser Val
            20                  25                  30
Glu Phe Asp Ala Val Glu Lys Met Thr Ala Ile Leu Ala Glu Val Gly
        35                  40                  45
Cys Thr Leu Met Asn Pro Tyr Gly Pro Pro Cys Leu Pro Ser Asp Leu
    50                  55                  60
His Ala Phe Arg Arg Asn Leu Thr Gly Arg Leu Ser Ser Phe Ser Ala
65                  70                  75                  80
Asn Ser Gly Glu Arg Asp Asn Val Gly Ala Leu Cys Ser Val Phe Val

85                  90                  95
Ala Gly Phe Ser Leu Tyr Ile Gln Ser Pro Ser Asn Leu Arg Arg Met
            100                 105                 110
Leu Ser Ser Ser Ser Thr Thr Lys Arg Asp Glu Ser Leu Val Arg Asn
        115                 120                 125
Leu Leu Leu Val Ser Pro Ile Gln Leu Asp Ile Gln Glu Met Leu Leu
    130                 135                 140
Glu Lys Leu Pro Glu Tyr Phe Asp Val Val Thr Gly Cys Ser Leu Glu
145                 150                 155                 160
Glu Asp Val Ala Arg Leu Ile Ile Asn His Phe Arg Thr Leu Asp Phe
                165                 170                 175
Ile Val Asn Pro His Val Phe Thr Asp Lys Leu Met Gln Val Leu Ser
            180                 185                 190
Ile Cys Pro Leu Glu Leu Lys Lys Glu Ile Ile Gly Ser Leu Pro Glu
        195                 200                 205
Ile Ile Gly Asp His Asn Cys Gln Ala Val Val Asp Ser Leu Glu Lys
    210                 215                 220
Met Leu Gln Glu Asp Ser Ala Val Val Val Ala Val Leu Asp Ser Phe
225                 230                 235                 240
Ser Asn Leu Asn Leu Asp Asp Gln Leu Gln Glu Gln Ala Ile Thr Val
                245                 250                 255
Ala Ile Ser Cys Ile Arg Thr Ile Asp Gly Glu His Met Pro Tyr Leu
            260                 265                 270
Leu Arg Phe Leu Leu Leu Ala Ala Thr Pro Val Asn Val Arg Arg Ile
        275                 280                 285
Ile Ser Gln Ile Arg Glu Gln Leu Lys Phe Thr Gly Met Ser Gln Pro
    290                 295                 300
Cys Ala Ser Gln Asn Lys Leu Lys Gly Lys Val Pro Ala Tyr Asn Ala
305                 310                 315                 320
Glu Gly Ser Ile Leu His Ala Leu Arg Ser Ser Leu Arg Phe Lys Asn
                325                 330                 335
Ile Leu Cys Gln Glu Ile Ile Lys Glu Leu Asn Ser Leu Glu Lys Pro
            340                 345                 350
Arg Asp Phe Lys Val Ile Asp Val Trp Leu Leu Ile Asp Met Tyr Met
        355                 360                 365
Asn Gly Asp Pro Val Arg Lys Ser Ile Glu Lys Ile Phe Lys Lys Lys
    370                 375                 380
Val Val Asp Glu Cys Ile Gln Glu Ala Leu Leu Asp Gln Cys Ile Gly
385                 390                 395                 400
Gly Asn Lys Glu Phe Val Lys Ile Leu Gly Ala Leu Val Thr His Val
                405                 410                 415
Gly Ser Asp Asn Lys Phe Glu Val Ser Ser Val Leu Glu Met Met Thr
            420                 425                 430
Ala Leu Val Lys Lys Tyr Ala Gln Gln Leu Leu Pro Phe Ser Ser His
        435                 440                 445
Ile Asn Gly Ile Ser Gly Thr Cys Ile Leu Asp Tyr Leu Glu Gly Phe
    450                 455                 460
Thr Ile Asp Asn Leu His Lys Thr Tyr Ser Gln Val Tyr Glu Val Phe
465                 470                 475                 480
Ser Leu Leu Ala Leu Ser Ala Arg Ala Ser Gly Asp Ser Phe Arg Ser
                485                 490                 495
Ser Thr Ser Asn Glu Leu Met Met Ile Val Arg Lys Gln Leu Thr Pro
            500                 505                 510
Ser Cys Leu Val Leu Tyr Trp Gln Val Ser His Pro Asp Leu Lys Tyr
        515                 520                 525
Lys Lys Met Gly Leu Val Gly Ser Leu Arg Ile Val Ser Ser Leu Gly
    530                 535                 540
Asp Ala Lys Ser Val Pro Asp Phe Ser Ser Ser Gln Val Glu Arg Leu
545                 550                 555                 560
Thr Asn Asp Gly Ser Leu Ala Gly Val Asp Ala Leu Leu Gly Cys Pro
                565                 570                 575
Leu His Leu Pro Ser Ser Lys Leu Val Gly Ser Leu Trp Gly Arg Ser
            580                 585                 590
Arg Lys Lys Ser Ser Pro Ser Arg Tyr Ile Met Leu Gln Thr Gly Tyr
        595                 600                 605
Glu Asn Ser Leu Val Thr Leu Pro Cys Ile Phe Cys Asp Leu Leu Asn
    610                 615                 620
Ala Phe Ser Ser Gln Ile Asp Glu Lys Ile Gly Cys Ile Ser Gln Ala

625                 630                 635                 640
Thr Val Lys Asp Val Thr Thr Lys Leu Leu Lys Arg Leu Arg Asn Leu
                645                 650                 655
Val Phe Leu Glu Ser Leu Leu Ser Asn Leu Ile Thr Leu Ser Pro Gln
            660                 665                 670
Ser Leu Pro Glu Leu His Pro Tyr Ser Glu Ser His Val Glu His Pro
        675                 680                 685
Arg Lys Lys Asn Glu Lys Arg Lys Leu Asp Asp Asp Ala Ser Gln Arg
    690                 695                 700
Lys Val Ser Met Lys Asn Asn Leu Lys Lys Ser Lys His Ser Asp Val
705                 710                 715                 720
Asn Glu Lys Leu Arg Gln Pro Thr Ile Met Asp Ala Phe Lys Lys Ala
                725                 730                 735
Gly Ala Val Met Ser His Ser Gln Thr Gln Leu Arg Gly Thr Pro Ser
            740                 745                 750
Leu Pro Ser Met Asp Gly Ser Thr Ala Ala Gly Ser Met Asp Glu Asn
        755                 760                 765
Cys Ser Asp Asn Glu Ser Leu Ile Val Lys Ile Pro Gln Val Ser Ser
    770                 775                 780
Ala Leu Glu Ala Gln Pro Phe Lys Phe Arg Pro Leu Leu Pro Gln Cys
785                 790                 795                 800
Leu Ser Ile Leu Asn Phe Pro Lys Val Leu Ser Gln Asp Met Gly Ser
                805                 810                 815
Pro Glu Tyr Arg Ala Glu Leu Pro Leu Tyr Leu Tyr Leu Leu His Asp
            820                 825                 830
Leu His Thr Lys Leu Asp Cys Leu Val Pro Pro Gly Lys Gln His Pro
        835                 840                 845
Phe Lys Arg Gly Ser Ala Pro Gly Tyr Phe Gly Arg Phe Lys Leu Val
    850                 855                 860
Glu Leu Leu Asn Gln Ile Lys Arg Leu Phe Pro Ser Leu Asn Ile Lys
865                 870                 875                 880
Leu Asn Ile Ala Ile Ser Leu Leu Ile Arg Gly Asp Glu Thr Ser Gln
                885                 890                 895
Thr Thr Trp Arg Asp Glu Phe Ala Leu Ser Gly Asn Pro Asn Thr Ser
            900                 905                 910
Ser Ile Val Val Ser Glu Ser Leu Val Tyr Thr Met Val Cys Lys Glu
        915                 920                 925
Val Leu Tyr Cys Phe Ser Lys Ile Leu Thr Leu Pro Glu Phe Glu Thr
    930                 935                 940
Asp Lys Ser Leu Leu Leu Asn Leu Leu Glu Ala Phe Gln Pro Thr Glu
945                 950                 955                 960
Ile Pro Val Ala Asn Phe Pro Asp Phe Gln Pro Phe Pro Ser Pro Gly
                965                 970                 975
Thr Lys Glu Tyr Leu Tyr Ile Gly Val Ser Tyr Phe Phe Glu Asp Ile
            980                 985                 990
Leu Asn Lys Gly Asn Tyr Phe Cys Ser Phe Thr Asp Asp Phe Pro Tyr
        995                 1000                1005
Pro Cys Ser Phe Ser Phe Asp Leu Ala Phe Glu Cys Leu Leu Thr Leu
    1010                1015                1020
Gln Leu Val Val Thr Ser Val Gln Lys Tyr Leu Gly Lys Val Ser Glu
1025                1030                1035                1040
Glu Ala Asn Arg Lys Arg Asn Pro Gly His Phe His Gly Leu Val Pro
                1045                1050                1055
Asn Leu His Ala Lys Leu Gly Thr Ser Ala Glu Lys Leu Leu Arg His
            1060                1065                1070
Lys Trp Val Asp Glu Ser Thr Asp Asn Lys Gly Leu Lys Asn Lys Val
        1075                1080                1085
Cys Pro Phe Val Ser Asn Leu Arg Ile Val Gln Phe Thr Gly Glu Met
    1090                1095                1100
Val Gln Thr Ile Leu Arg Ile Tyr Leu Glu Ala Ser Gly Ser Thr Ser
1105                1110                1115                1120
Asp Leu Leu Asp Glu Leu Ala Cys Thr Ile Leu Pro Gln Ala Ser Leu
                1125                1130                1135
Ser Lys Ser Thr Gly Glu Asp Asp Asp Ala Arg Asp His Glu Phe Pro
            1140                1145                1150
Thr Leu Cys Ala Ala Thr Phe Arg Gly Trp Tyr Lys Thr Leu Leu Glu
        1155                1160                1165
Glu Asn Leu Ala Ile Leu Asn Lys Leu Val Lys Thr Val Ser Ser Glu

1170                    1175                    1180
Lys Arg Gly Asn Cys Gln Pro Lys Thr Thr Glu Ala His Leu Lys Asn
1185                1190                1195                1200
Ile Gln Lys Thr Val Asn Val Val Val Ser Leu Val Asn Leu Cys Arg
                1205                1210                1215
Ser His Glu Lys Val Thr Ile His Gly Met Ala Ile Lys Tyr Gly Gly
            1220                1225                1230
Lys Tyr Val Asp Ser Phe Leu Lys Gly Ser Leu Lys His Lys Asp Leu
        1235                1240                1245
Arg Gly Gln Ile Val Ser Ser Gln Ala Tyr Ile Asp Asn Glu Ala Asp
    1250                1255                1260
Glu Val Glu Glu Thr Met Ser Gly Glu Glu Glu Pro Met Gln Glu Asp
1265                1270                1275                1280
Glu Leu Pro Leu Thr Pro
                1285

<210> 4
<211> 1471
<212> PRT
<213> Homo sapiens

<220>
<221> PEPTIDE
<222> (1)...(1471)
<223> Human fancd2

<400> 4
Met Val Ser Lys Arg Arg Leu Ser Lys Ser Glu Asp Lys Glu Ser Leu
 1               5                  10                  15
Thr Glu Asp Ala Ser Lys Thr Arg Lys Gln Pro Leu Ser Lys Lys Thr
            20                  25                  30
Lys Lys Ser His Ile Ala Asn Ala Val Glu Glu Asn Asp Ser Ile Phe
        35                  40                  45
Val Lys Leu Leu Lys Ile Ser Gly Ile Ile Leu Lys Thr Gly Glu Ser
    50                  55                  60
Gln Asn Gln Leu Ala Val Asp Gln Ile Ala Phe Gln Lys Lys Leu Phe
65                  70                  75                  80
Gln Thr Leu Arg Arg His Pro Ser Tyr Pro Lys Ile Ile Glu Glu Phe
                85                  90                  95
Val Ser Gly Leu Glu Ser Tyr Ile Glu Asp Glu Asp Ser Phe Arg Asn
            100                 105                 110
Cys Leu Leu Ser Cys Glu Arg Leu Gln Asp Glu Glu Ala Ser Met Gly
        115                 120                 125
Ala Ser Tyr Ser Lys Ser Leu Ile Lys Leu Leu Leu Gly Ile Asp Ile
    130                 135                 140
Leu Gln Pro Ala Ile Ile Lys Thr Leu Phe Glu Lys Leu Pro Glu Tyr
145                 150                 155                 160
Phe Phe Glu Asn Arg Asn Ser Asp Glu Ile Asn Ile Phe Arg Leu Ile
                165                 170                 175
Val Ser Gln Leu Lys Trp Leu Asp Arg Val Val Asp Gly Lys Asp Leu
            180                 185                 190
Thr Thr Lys Ile Met Gln Leu Ile Ser Ile Ala Pro Glu Asn Leu Gln
        195                 200                 205
His Asp Ile Ile Thr Ser Lys Pro Glu Ile Leu Gly Asp Ser Gln His
    210                 215                 220
Ala Asp Val Gly Lys Glu Leu Ser Asp Leu Leu Ile Glu Asn Thr Ser
225                 230                 235                 240
Leu Thr Val Pro Ile Leu Asp Val Leu Ser Ser Leu Arg Leu Asp Pro
                245                 250                 255
Asn Phe Leu Leu Lys Val Arg Gln Leu Val Met Asp Lys Leu Ser Ser
            260                 265                 270
Ile Arg Leu Glu Asp Leu Pro Val Ile Ile Lys Phe Ile Leu His Ser
        275                 280                 285
Val Thr Ala Met Asp Thr Leu Glu Val Ile Ser Glu Leu Arg Glu Lys
    290                 295                 300
Leu Asp Leu Gln His Cys Val Leu Pro Ser Arg Leu Gln Ala Ser Gln
305                 310                 315                 320
Val Lys Leu Lys Ser Lys Gly Arg Ala Ser Ser Ser Gly Asn Gln Glu

                325                 330                 335
Ser Ser Gly Gln Ser Cys Ile Ile Leu Leu Phe Asp Val Ile Lys Ser
            340                 345                 350
Ala Ile Arg Tyr Glu Lys Thr Ile Ser Glu Ala Trp Ile Lys Ala Ile
        355                 360                 365
Glu Asn Thr Ala Ser Val Ser Glu His Lys Val Phe Asp Leu Val Met
    370                 375                 380
Leu Phe Ile Ile Val Ser Thr Asn Thr Gln Thr Lys Lys Tyr Ile Asp
385                 390                 395                 400
Arg Val Leu Arg Asn Lys Ile Arg Ser Gly Cys Ile Gln Glu Gln Leu
                405                 410                 415
Leu Gln Ser Thr Phe Ser Val His Tyr Leu Val Leu Lys Asp Met Cys
            420                 425                 430
Ser Ser Ile Leu Ser Leu Ala Gln Ser Leu Leu His Ser Leu Asp Gln
        435                 440                 445
Ser Ile Ile Ser Phe Gly Ser Leu Leu Tyr Lys Tyr Ala Phe Lys Phe
    450                 455                 460
Phe Asp Thr Tyr Cys Gln Gln Glu Val Val Gly Ala Leu Val Thr His
465                 470                 475                 480
Ile Cys Ser Gly Asn Glu Ala Glu Val Asp Asp Ala Leu Asp Val Leu
                485                 490                 495
Leu Glu Leu Val Val Leu Asn Pro Ser Ala Met Met Met Asn Ala Val
            500                 505                 510
Phe Val Gln Gly Ile Leu Asp Tyr Leu Asp Asn Ile Ser Pro Gln Gln
        515                 520                 525
Ile Arg Lys Leu Phe Tyr Val Leu Ser Thr Leu Ala Phe Ser Lys Gln
    530                 535                 540
Asn Glu Ala Ser Ser His Ile Gln Asp Asp Met His Leu Val Ile Arg
545                 550                 555                 560
Lys Gln Leu Ser Ser Thr Val Phe Lys Tyr Lys Leu Ile Gly Ile Ile
                565                 570                 575
Gly Ala Val Thr Met Ala Gly Ile Met Ala Ala Asp Arg Ser Glu Ser
            580                 585                 590
Pro Ser Leu Thr Gln Glu Arg Ala Asn Leu Ser Asp Glu Gln Cys Thr
        595                 600                 605
Gln Val Thr Ser Leu Leu Gln Leu Val His Ser Cys Ser Glu Gln Ser
    610                 615                 620
Pro Gln Ala Ser Ala Leu Tyr Tyr Asp Glu Phe Ala Asn Leu Ile Gln
625                 630                 635                 640
His Glu Lys Leu Asp Pro Lys Ala Leu Glu Trp Val Gly His Thr Ile
                645                 650                 655
Cys Asn Asp Phe Gln Asp Ala Phe Val Val Asp Ser Cys Val Val Pro
            660                 665                 670
Glu Gly Asp Phe Pro Phe Pro Val Lys Ala Leu Tyr Gly Leu Glu Glu
        675                 680                 685
Tyr Asp Thr Gln Asp Gly Ile Ala Ile Asn Leu Leu Pro Leu Leu Phe
    690                 695                 700
Ser Gln Asp Phe Ala Lys Asp Gly Gly Pro Val Thr Ser Gln Glu Ser
705                 710                 715                 720
Gly Gly Lys Leu Val Ser Pro Leu Cys Leu Ala Pro Tyr Phe Arg Leu
                725                 730                 735
Leu Arg Leu Cys Val Glu Arg Gln His Asn Gly Asn Leu Glu Glu Ile
            740                 745                 750
Asp Gly Leu Leu Asp Cys Pro Ile Phe Leu Thr Asp Leu Glu Pro Gly
        755                 760                 765
Glu Lys Leu Glu Ser Met Ser Ala Lys Glu Ala Ser Phe Met Cys Ser
    770                 775                 780
Leu Ile Phe Leu Thr Leu Asn Trp Phe Arg Glu Ile Val Asn Ala Phe
785                 790                 795                 800
Cys Gln Glu Thr Ser Pro Glu Asn Lys Gly Lys Val Leu Thr Arg Leu
                805                 810                 815
Lys His Ile Val Glu Leu Gln Ile Leu Leu Glu Lys Tyr Leu Ala Val
            820                 825                 830
Thr Pro Asp Tyr Val Pro Pro Leu Gly Asn Phe Asp Val Glu Thr Leu
        835                 840                 845
Asp Ile Thr Pro His Thr Val Thr Ala Ile Ser Ala Lys Ile Arg Lys
    850                 855                 860
Lys Gly Lys Ile Glu Arg Lys Gln Lys Thr Asp Gly Ser Lys Thr Ser

865                 870                 875                 880
Ser Ser Asp Thr Leu Ser Glu Glu Lys Asn Ser Glu Cys Asp Pro Thr
                885                 890                 895
Pro Ser His Arg Gly Gln Leu Asn Lys Glu Phe Thr Gly Lys Glu Glu
            900                 905                 910
Lys Thr Ser Leu Leu Leu His Asn Ser His Ala Phe Phe Arg Glu Leu
        915                 920                 925
Asp Ile Glu Val Phe Ser Ile Leu His Cys Gly Leu Val Thr Lys Phe
    930                 935                 940
Ile Leu Asp Thr Glu Met His Thr Glu Ala Thr Glu Val Val Gln Leu
945                 950                 955                 960
Gly Pro Pro Glu Leu Leu Phe Leu Leu Glu Asp Leu Ser Gln Lys Leu
                965                 970                 975
Glu Ser Met Leu Thr Pro Pro Ile Ala Arg Arg Val Pro Phe Leu Lys
            980                 985                 990
Asn Lys Gly Ser Arg Asn Ile Gly Phe Ser His Leu Gln Gln Arg Ser
        995                 1000                1005
Ala Gln Glu Ile Val His Cys Val Glu Gln Leu Leu Thr Pro Met Cys
    1010                1015                1020
Asn His Leu Glu Asn Ile His Asn Tyr Ile Gln Cys Leu Ala Ala Glu
1025                1030                1035                1040
Asn His Gly Val Val Asp Gly Pro Gly Val Lys Val Gln Glu Tyr His
                1045                1050                1055
Ile Met Ser Ser Cys Tyr Gln Arg Leu Leu Gln Ile Phe His Gly Leu
            1060                1065                1070
Phe Ala Trp Ser Gly Phe Ser Gln Pro Glu Asn Gln Asn Leu Leu Tyr
        1075                1080                1085
Ser Ala Leu His Val Leu Ser Ser Arg Leu Lys Gln Gly Glu His Ser
    1090                1095                1100
Gln Pro Leu Glu Glu Leu Leu Ser Gln Ser Val His Tyr Leu Gln Asn
1105                1110                1115                1120
Phe His Gln Ser Ile Pro Ser Phe Gln Cys Ala Leu Tyr Leu Ile Arg
                1125                1130                1135
Leu Leu Met Val Ile Leu Glu Lys Ser Thr Ala Ser Ala Gln Asn Lys
            1140                1145                1150
Glu Lys Ile Ala Ser Leu Ala Arg Gln Phe Leu Cys Arg Val Trp Pro
        1155                1160                1165
Ser Gly Asp Lys Glu Lys Ser Asn Ile Ser Asn Asp Gln Leu His Ala
    1170                1175                1180
Leu Leu Cys Ile Tyr Leu Glu His Thr Glu Ser Ile Leu Lys Ala Ile
1185                1190                1195                1200
Glu Glu Ile Ala Gln Val Gly Val Pro Glu Leu Ile Asn Ser Pro Lys
                1205                1210                1215
Asp Ala Ser Ser Ser Thr Phe Pro Thr Leu Thr Arg His Thr Pro Val
            1220                1225                1230
Val Phe Phe Arg Val Met Met Ala Glu Leu Glu Lys Ile Val Lys Lys
        1235                1240                1245
Ile Glu Pro Gly Thr Ala Ala Asp Ser Gln Gln Ile His Glu Glu Lys
    1250                1255                1260
Leu Leu Tyr Trp Asn Met Ala Val Arg Asp Phe Ser Ile Leu Ile Asn
1265                1270                1275                1280
Leu Ile Lys Val Phe Asp Ser His Pro Val Leu His Val Cys Leu Lys
                1285                1290                1295
Val Gly Arg Leu Phe Val Glu Ala Phe Leu Lys Gln Cys Met Pro Leu
            1300                1305                1310
Leu Asp Ile Ser Phe Arg Lys His Arg Glu Asp Val Leu Ser Leu Leu
        1315                1320                1325
Glu Thr Phe Gln Leu Asp Thr Arg Leu Leu His His Leu Cys Gly His
    1330                1335                1340
Ser Lys Ile His Gln Asp Thr Arg Leu Thr Gln His Val Pro Leu Leu
1345                1350                1355                1360
Lys Lys Thr Leu Glu Leu Leu Val Cys Arg Val Lys Ala Met Leu Thr
                1365                1370                1375
Leu Asn Asn Cys Arg Glu Ala Phe Trp Leu Gly Asn Leu Lys Asn Arg
            1380                1385                1390
Asp Leu Gln Gly Glu Glu Ile Lys Ser Gln Asn Ser Gln Glu Ser Thr
        1395                1400                1405
Ala Asp Glu Ser Glu Asp Asp Met Ser Ser Gln Ala Ser Lys Ser Lys

1410                    1415                    1420
Ala Thr Glu Val Ser Leu Gln Asn Pro Pro Glu Ser Gly Thr Asp Gly
1425                1430                1435                1440
Cys Ile Leu Leu Ile Val Leu Ser Trp Trp Ser Arg Thr Leu Pro Thr
                1445                1450                1455
Tyr Val Tyr Cys Gln Met Leu Leu Cys Pro Phe Pro Phe Pro Pro
            1460                1465                1470

<210> 5
<211> 5189
<212> DNA
<213> Artificial Sequence

<220>
<223> cDNA sequence

<400> 5
tcgaaaacta cgggcggcga cggcttctcg gaagtaattt aagtgcacaa gacattggtc 60
aaaatggttt ccaaaagaag actgtcaaaa tctgaggata aagagagcct gacagaagat 120
gcctccaaaa ccaggaagca accactttcc aaaaagacaa agaaatctca tattgctaat 180
gaagttgaag aaaatgacag catctttgta aagcttctta agatatcagg aattattctt 240
aaaacgggag agagtcagaa tcaactagct gtggatcaaa tagctttcca aaagaagctc 300
tttcagaccc tgaggagaca cccttcctat cccaaaataa tagaagaatt tgttagtggc 360
ctggagtctt acattgagga tgaagacagt ttcaggaact gccttttgtc ttgtgagcgt 420
ctgcaggatg aggaagccag tatgggtgca tcttattcta agagtctcat caaactgctt 480
ctggggattg acatactgca gcctgccatt atcaaaacct tatttgagaa gttgccagaa 540
tatttttttg aaaacaagaa cagtgatgaa atcaacatac ctcgactcat tgtcagtcaa 600
ctaaaatggc ttgacagagt tgtggatggc aaggacctca ccaccaagat catgcagctg 660
atcagtattg ctccagagaa cctgcagcat gacatcatca ccagcctacc tgagatccta 720
ggggattccc agcacgctga tgtggggaaa gaactcagtg acctactgat agagaatact 780
tcactcactg tcccaatcct ggatgtcctt tcaagcctcc gacttgaccc aaacttccta 840
ttgaaggttc gccagttggt gatggataag ttgtcgtcta ttagattgga ggatttacct 900
gtgataataa agttcattct tcattccgta acagccatgg atacacttga ggtaatttct 960
gagcttcggg agaagttgga tctgcagcat tgtgttttgc catcacggtt acaggcttcc 1020
caagtaaagt tgaaaagtaa aggacgagca agttcctcag gaaatcaaga aagcagcggt 1080
cagagctgta ttattctcct ctttgatgta ataaagtcag ctattagata tgagaaaacc 1140
atttcagaag cctggattaa ggcaattgaa aacactgcct cagtatctga acacaaggtg 1200
tttgacctgg tgatgctttt catcatctat agcaccaata ctcagacaaa gaagtacatt 1260
gacagggtgc taagaaataa gattcgatca ggctgcattc aagaacagct gctccagagt 1320
acattctctg ttcattactt agttcttaag gatatgtgtt catccattct gtcgctggct 1380
cagagtttgc ttcactctct agaccagagt ataatttcat ttggcagtct cctatacaaa 1440
tatgcattta agttttttga cacgtactgc cagcaggaag tggttggtgc cttagtgacc 1500
catatctgca gtgggaatga agctgaagtt gatactgcct tagatgtcct tctagagttg 1560
gtagtgttaa acccatctgc tatgatgatg aatgctgtct ttgtaaaggg cattttagat 1620
tatctggata acatatcccc tcagcaaata cgaaaactct tctatgttct cagcacactg 1680
gcatttagca aacagaatga agccagcagc cacatccagg atgacatgca cttggtgata 1740
agaaagcagc tctctagcac cgtattcaag tacaagctca ttgggattat tggtgctgtg 1800
accatggctg gcatcatggc ggcagacaga agtgaatcac ctagtttgac ccaagagaga 1860
gccaacctga gcgatgagca gtgcacacag gtgacctcct tgttgcagtt ggttcattcc 1920
tgcagtgagc agtctcctca ggcctctgca ctttactatg atgaatttgc caacctgatc 1980
caacatgaaa agctggatcc aaaagccctg gaatgggttg ggcataccat ctgtaatgat 2040
ttccaggatg ccttcgtagt ggactcctgt gttgttccgg aaggtgactt tccatttcct 2100
gtgaaagcac tgtacggact ggaagaatac gacactcagg atgggattgc cataaacctc 2160
ctgccgctgc tgttttctca ggactttgca aaagatgggg gtccggtgac ctcacaggaa 2220
tcaggccaaa aattggtgtc tccgctgtgc ctggctccgt atttccggtt actgagactt 2280
tgtgtggaga gacagcataa cggaaacttg gaggagattg atggtctact agattgtcct 2340
atattcctaa ctgacctgga gcctggagag aagttggagt ccatgtctgc taaagagcgt 2400
tcattcatgt gttctctcat atttcttact ctcaactggt tccgagagat tgtaaatgcc 2460
ttctgccagg aaacatcacc tgagatgaag gggaaggtgc tcactcggtt aaagcacatt 2520
gtagaattgc aaataatcct ggaaaagtac ttggcagtca ccccagacta tgtccctcct 2580
cttggaaact ttgatgtgga aactttagat ataacacctc atactgttac tgctatttca 2640
gcaaaaatca gaaagaaagg aaaaatagaa aggaaacaaa aaacagatgg cagcaagaca 2700
tcctcctctg acacactttc agaagagaaa aattcagaat gtgaccctac gccatctcat 2760
agaggccagc taaacaagga gttcacaggg aaggaagaaa agacatcatt gttactacat 2820
aattcccatg cttttttccg agagctggac attgaggtct tctctattct acattgtgga 2880
cttgtgacga agttcatctt agatactgaa atgcacactg aagctacaga agttgtgcaa 2940
cttgggcccc ctgagctgct tttcttgctg gaagatctct cccagaagct ggagagtatg 3000
ctgacacctc ctattgccag gagagtcccc tttctcaaga acaaaggaag ccggaatatt 3060

ggattctcac atctccaaca gagatctgcc caagaaattg ttcattgtgt ttttcaactg 3120
ctgaccccaa tgtgtaacca cctggagaac attcacaact attttcagtg tttagctgct 3180
gagaatcacg gtgtagttga tggaccagga gtgaaagttc aggagtacca cataatgtct 3240
tcctgctatc agaggctgct gcagattttt catgggcttt ttgcttggag tggattttct 3300
caacctgaaa atcagaattt actgtattca gccctccatg tccttagtag ccgactgaaa 3360
cagggagaac acagccagcc tttggaggaa ctactcagcc agagcgtcca ttacttgcag 3420
aatttccatc aaagcattcc cagtttccag tgtgctcttt atctcatcag acttttgatg 3480
gttattttgg agaaatcaac agcttctgct cagaacaaag aaaaaattgc ttcccttgcc 3540
agacaattcc tctgtcgggt gtggccaagt ggggataaag agaagagcaa catctctaat 3600
gaccagctcc atgctctgct ctgtatctac ctggagcaca cagagagcat tctgaaggcc 3660
atagaggaga ttgctggtgt tggtgtccca gaactgatca actctcctaa agatgcatct 3720
tcctccacat tccctacact gaccaggcat acttttgttg ttttcttccg tgtgatgatg 3780
gctgaactag agaagacggt gaaaaaaatt gagcctggca cagcagcaga ctcgcagcag 3840
attcatgaag agaaactcct ctactggaac atggctgttc gagacttcag tatcctcatc 3900
aacttgataa aggtatttga tagtcatcct gttctgcatg tatgtttgaa gtatgggcgt 3960
ctctttgtgg aagcatttct gaagcaatgt atgccgctcc tagacttcag ttttagaaaa 4020
caccgggaag atgttctgag cttactggaa accttccagt tggacacaag gctgcttcat 4080
cacctgtgtg ggcattccaa gattcaccag gacacgagac tcacccaaca tgtgcctctg 4140
ctcaaaaaga ccctggaact tttagtttgc agagtcaaag ctatgctcac tctcaacaat 4200
tgtagagagg ctttctggct gggcaatcta aaaaaccggg acttgcaggg tgaagagatt 4260
aagtcccaaa attcccagga gagcacagca gatgagagtg aggatgacat gtcatcccag 4320
gcctccaaga gcaaagccac tgaggtatct ctacaaaacc caccagagtc tggcactgat 4380
ggttgcattt tgttaattgt tctaagttgg tggagcagaa ctttgcctac ttatgtttat 4440
tgtcaaatgc ttctatgccc atttccattc cctccataac agcttctgtg cttatataat 4500
ttttgggacc cagaagaaac aacgacacaa tcttagaatc actcctgagt atctcgagtt 4560
gtggcatttg ttatagagtt gacaattttc tgcattatag cctctcattt tccatgaatt 4620
catatctgaa accattttag aagggagaag tcatcgaagt attttctgag tgttgagaag 4680
aatgagttaa accatttaaa cacatttgaa acatacaaaa atagaaatgt gaaagcattt 4740
ggtgaaagcc aaagcacaga gtcagaagct gccaccttag agaactgaaa taaaaataga 4800
agttcttacg cttttttgtg gtacagatgc tttcgacaat ttaaagaaag ctaaataaaa 4860
atgtagacat ggctggcgca gtggctcatg cttgtaatcc tagcactttt tgaggccaag 4920
gtaggaggat tgcttgagtc cgggagctca aggcaaagct gcacaacata acaagaccct 4980
atctccacaa aaaaaatgaa aaataaacct gggtgcggtg gctcacacct gtaatcccag 5040
cactttggga ggccgatgtg ggcagatcac aaggtcagga ggtcaagacc agcctggcca 5100
acatagtgaa accccatctc tactgaaaat acaaaaatta gctgggtgtg gtggcacgtg 5160
cctgttatct cagctacttg ggaagctga 5189

<210> 6
<211> 5194
<212> DNA
<213> Homo sapiens

<400> 6
tagaatcgaa aactacgggc ggcgacggct tctcggaagt aatttaagtg cacaagacat 60
tggtcaaaat ggtttccaaa agaagactgt caaaatctga ggataaagag agcctgacag 120
aagatgcctc caaaaccagg aagcaaccac tttccaaaaa gacaaagaaa tctcatattg 180
ctaatgaagt tgaagaaaat gacagcatct ttgtaaagct tcttaagata tcaggaatta 240
ttcttaaaac gggagagagt cagaatcaac tagctgtgga tcaaatagct ttccaaaaga 300
agctctttca gaccctgagg agacaccctt cctatcccaa aataatagaa gaatttgtta 360
gtggcctgga gtcttacatt gaggatgaag acagtttcag gaactgcctt ttgtcttgtg 420
agcgtctgca ggatgaggaa gccagtatgg gtgcatctta ttctaagagt ctcatcaaac 480
tgcttctggg gattgacata ctgcagcctg ccattatcaa aaccttattt gagaagttgc 540
cagaatattt ttttgaaaac aagaacagtg atgaaatcaa catacctcga ctcattgtca 600
gtcaactaaa atggcttgac agagttgtgg atggcaagga cctcaccacc aagatcatgc 660
agctgatcag tattgctcca gagaacctgc agcatgacat catcaccagc ctacctgaga 720
tcctagggga ttcccagcac gctgatgtgg ggaaagaact cagtgaccta ctgatagaga 780
atacttcact cactgtccca atcctggatg tcctttcaag cctccgactt gacccaaact 840
tcctattgaa ggttcgccag ttggtgatgg ataagttgtc gtctattaga ttggaggatt 900
tacctgtgat aataaagttc attcttcatt ccgtaacagc catggataca cttgaggtaa 960
tttctgagct tcgggagaag ttggatctgc agcattgtgt tttgccatca cggttacagg 1020
cttcccaagt aaagttgaaa agtaaaggac gagcaagttc ctcaggaaat caagaaagca 1080
gcggtcagag ctgtattatt ctcctctttg atgtaataaa gtcagctatt agatatgaga 1140
aaaccatttc agaagcctgg attaaggcaa ttgaaaacac tgcctcagta tctgaacaca 1200
aggtgtttga cctggtgatg cttttcatca tctatagcac caatactcag acaaagaagt 1260
acattgacag ggtgctaaga aataagattc gatcaggctg cattcaagaa cagctgctcc 1320
agagtacatt ctctgttcat tacttagttc ttaaggatat gtgttcatcc attctgtcgc 1380
tggctcagag tttgcttcac tctctagacc agagtataat ttcatttggc agtctcctat 1440
acaaatatgc atttaagttt tttgacacgt actgccagca ggaagtggtt ggtgccttag 1500

tgacccatat ctgcagtggg aatgaagctg aagttgatac tgccttagat gtccttctag 1560
agttggtagt gttaaaccca tctgctatga tgatgaatgc tgtctttgta aagggcattt 1620
tagattatct ggataacata tcccctcagc aaatacgaaa actcttctat gttctcagca 1680
cactggcatt tagcaaacag aatgaagcca gcagccacat ccaggatgac atgcacttgg 1740
tgataagaaa gcagctctct agcaccgtat tcaagtacaa gctcattggg attattggtg 1800
ctgtgaccat ggctggcatc atggcggcag acagaagtga atcacctagt ttgacccaag 1860
agagagccaa cctgagcgat gagcagtgca cacaggtgac ctccttgttg cagttggttc 1920
attcctgcag tgagcagtct cctcaggcct ctgcacttta ctatgatgaa tttgccaacc 1980
tgatccaaca tgaaaagctg gatccaaaag ccctggaatg ggttgggcat accatctgta 2040
atgatttcca ggatgccttc gtagtggact cctgtgttgt tccggaaggt gactttccat 2100
ttcctgtgaa agcactgtac ggactggaag aatacgacac tcaggatggg attgccataa 2160
acctcctgcc gctgctgttt tctcaggact ttgcaaaaga tgggggtccg gtgacctcac 2220
aggaatcagg ccaaaaattg gtgtctccgc tgtgcctggc tccgtatttc cggttactga 2280
gactttgtgt ggagagacag cataacggaa acttggagga gattgatggt ctactagatt 2340
gtcctatatt cctaactgac ctggagcctg gagagaagtt ggagtccatg tctgctaaag 2400
agcgttcatt catgtgttct ctcatatttc ttactctcaa ctggttccga gagattgtaa 2460
atgccttctg ccaggaaaca tcacctgaga tgaaggggaa ggtgctcact cggttaaagc 2520
acattgtaga attgcaaata atcctggaaa agtacttggc agtcacccca gactatgtcc 2580
ctcctcttgg aaactttgat gtggaaactt tagatataac acctcatact gttactgcta 2640
tttcagcaaa aatcagaaag aaaggaaaaa tagaaaggaa acaaaaaaca gatggcagca 2700
agacatcctc ctctgacaca ctttcagaag agaaaaattc agaatgtgac cctacgccat 2760
ctcatagagg ccagctaaac aaggagttca cagggaagga agaaaagaca tcattgttac 2820
tacataattc ccatgctttt ttccgagagc tggacattga ggtcttctct attctacatt 2880
gtggacttgt gacgaagttc atcttagata ctgaaatgca cactgaagct acagaagttg 2940
tgcaacttgg gccccctgag ctgcttttct tgctggaaga tctctcccag aagctggaga 3000
gtatgctgac acctcctatt gccaggagag tcccctttct caagaacaaa ggaagccgga 3060
atattggatt ctcacatctc caacagagat ctgcccaaga aattgttcat tgtgtttttc 3120
aactgctgac cccaatgtgt aaccacctgg agaacattca caactatttt cagtgtttag 3180
ctgctgagaa tcacggtgta gttgatggac caggagtgaa agttcaggag taccacataa 3240
tgtcttcctg ctatcagagg ctgctgcaga tttttcatgg gctttttgct tggagtggat 3300
tttctcaacc tgaaaatcag aatttactgt attcagccct ccatgtcctt agtagccgac 3360
tgaaacaggg agaacacagc cagcctttgg aggaactact cagccagagc gtccattact 3420
tgcagaattt ccatcaaagc attcccagtt tccagtgtgc tctttatctc atcagacttt 3480
tgatggttat tttggagaaa tcaacagctt ctgctcagaa caaagaaaaa attgcttccc 3540
ttgccagaca attcctctgt cgggtgtggc caagtgggga taaagagaag agcaacatct 3600
ctaatgacca gctccatgct ctgctctgta tctacctgga gcacacagag agcattctga 3660
aggccataga ggagattgct ggtgttggtg tcccagaact gatcaactct cctaaagatg 3720
catcttcctc cacattccct acactgacca ggcatacttt tgttgttttc ttccgtgtga 3780
tgatggctga actagagaag acggtgaaaa aaattgagcc tggcacagca gcagactcgc 3840
agcagattca tgaagagaaa ctcctctact ggaacatggc tgttcgagac ttcagtatcc 3900
tcatcaactt gataaaggta tttgatagtc atcctgttct gcatgtatgt ttgaagtatg 3960
ggcgtctctt tgtggaagca tttctgaagc aatgtatgcc gctcctagac ttcagtttta 4020
gaaaacaccg ggaagatgtt ctgagcttac tggaaacctt ccagttggac acaaggctgc 4080
ttcatcacct gtgtgggcat tccaagattc accaggacac gagactcacc caacatgtgc 4140
ctctgctcaa aaagaccctg gaacttttag tttgcagagt caaagctatg ctcactctca 4200
acaattgtag agaggctttc tggctgggca atctaaaaaa ccgggacttg cagggtgaag 4260
agattaagtc ccaaaattcc caggagagca cagcagatga gagtgaggat gacatgtcat 4320
cccaggcctc caagagcaaa gccactgagg tatctctaca aaacccacca gagtctggca 4380
ctgatggttg cattttgtta attgttctaa gttggtggag cagaactttg cctacttatg 4440
tttattgtca aatgcttcta tgcccatttc cattccctcc ataacagctt ctgtgcttat 4500
ataatttttg ggacccagaa gaaacaacga cacaatctta gaatcactcc tgagtatctc 4560
gagttgtggc atttgttata gagttgacaa ttttctgcat tatagcctct cattttccat 4620
gaattcatat ctgaaaccat tttagaaggg agaagtcatc gaagtatttt ctgagtgttg 4680
agaagaatga gttaaaccat ttaaacacat ttgaaacata caaaaataga aatgtgaaag 4740
catttggtga aagccaaagc acagagtcag aagctgccac cttagagaac tgaaataaaa 4800
atagaagttc ttacgctttt ttgtggtaca gatgctttcg acaatttaaa gaaagctaaa 4860
taaaaatgta gacatggctg gcgcagtggc tcatgcttgt aatcctagca ctttttgagg 4920
ccaaggtagg aggattgctt gagtccggga gctcaaggca aagctgcaca acataacaag 4980
accctatctc cacaaaaaaa atgaaaaata aacctgggtg cggtggctca cacctgtaat 5040
cccagcactt tgggaggccg atgtgggcag atcacaaggt caggagttca agaccagcct 5100
ggccaacata gtgaaacccc atctctactg aaaatacaaa aattagctgg gtgtggtggc 5160
acgtgcctgt tatctcagct acttgggaag ctga 5194

<210> 7
<211> 4455
<212> DNA
<213> Homo sapiens

<400> 7
tcgaaaacta cgggcggcga cggcttctcg gaagtaattt aagtgcacaa gacattggtc 60
aaaatggttt ccaaaagaag actgtcaaaa tctgaggata aagagagcct gacagaagat 120
gcctccaaaa ccaggaagca accactttcc aaaaagacaa agaaatctca tattgctaat 180
gaagttgaag aaaatgacag catctttgta aagcttctta agatatcagg aattattctt 240
aaaacgggag agagtcagaa tcaactagct gtggatcaaa tagctttcca aaagaagctc 300
tttcagaccc tgaggagaca cccttcctat cccaaaataa tagaagaatt tgttagtggc 360
ctggagtctt acattgagga tgaagacagt ttcaggaact gccttttgtc ttgtgagcgt 420
ctgcaggatg aggaagccag tatgggtgca tcttattcta agagtctcat caaactgctt 480
ctggggattg acatactgca gcctgccatt atcaaaacct tatttgagaa gttgccagaa 540
tatttttttg aaaacaagaa cagtgatgaa atcaacatac ctcgactcat tgtcagtcaa 600
ctaaaatggc ttgacagagt tgtggatggc aaggacctca ccaccaagat catgcagctg 660
atcagtattg ctccagagaa cctgcagcat gacatcatca ccagcctacc tgagatccta 720
ggggattccc agcacgctga tgtggggaaa gaactcagtg acctactgat agagaatact 780
tcactcactg tcccaatcct ggatgtcctt tcaagcctcc gacttgaccc aaacttccta 840
ttgaaggttc gccagttggt gatggataag ttgtcgtcta ttagattgga ggatttacct 900
gtgataataa agttcattct tcattccgta acagccatgg atacacttga ggtaatttct 960
gagcttcggg agaagttgga tctgcagcat tgtgttttgc catcacggtt acaggcttcc 1020
caagtaaagt tgaaaagtaa aggacgagca agttcctcag gaaatcaaga aagcagcggt 1080
cagagctgta ttattctcct ctttgatgta ataaagtcag ctattagata tgagaaaacc 1140
atttcagaag cctggattaa ggcaattgaa aacactgcct cagtatctga acacaaggtg 1200
tttgacctgg tgatgctttt catcatctat agcaccaata ctcagacaaa gaagtacatt 1260
gacagggtgc taagaaataa gattcgatca ggctgcattc aagaacagct gctccagagt 1320
acattctctg ttcattactt agttcttaag gatatgtgtt catccattct gtcgctggct 1380
cagagtttgc ttcactctct agaccagagt ataatttcat ttggcagtct cctatacaaa 1440
tatgcattta agttttttga cacgtactgc cagcaggaag tggttggtgc cttagtgacc 1500
catatctgca gtgggaatga agctgaagtt gatactgcct tagatgtcct tctagagttg 1560
gtagtgttaa acccatctgc tatgatgatg aatgctgtct ttgtaaaggg cattttagat 1620
tatctggata acatatcccc tcagcaaata cgaaaactct tctatgttct cagcacactg 1680
gcatttagca aacagaatga agccagcagc cacatccagg atgacatgca cttggtgata 1740
agaaagcagc tctctagcac cgtattcaag tacaagctca ttgggattat tggtgctgtg 1800
accatggctg gcatcatggc ggcagacaga agtgaatcac ctagtttgac ccaagagaga 1860
gccaacctga gcgatgagca gtgcacacag gtgacctcct tgttgcagtt ggttcattcc 1920
tgcagtgagc agtctcctca ggcctctgca ctttactatg atgaatttgc caacctgatc 1980
caacatgaaa agctggatcc aaaagccctg gaatgggttg ggcataccat ctgtaatgat 2040
ttccaggatg ccttcgtagt ggactcctgt gttgttccgg aaggtgactt tccatttcct 2100
gtgaaagcac tgtacggact ggaagaatac gacactcagg atgggattgc cataaacctc 2160
ctgccgctgc tgttttctca ggactttgca aaagatgggg gtccggtgac ctcacaggaa 2220
tcaggccaaa aattggtgtc tccgctgtgc ctggctccgt atttccggtt actgagactt 2280
tgtgtggaga gacagcataa cggaaacttg gaggagattg atggtctact agattgtcct 2340
atattcctaa ctgacctgga gcctggagag aagttggagt ccatgtctgc taaagagcgt 2400
tcattcatgt gttctctcat atttcttact ctcaactggt tccgagagat tgtaaatgcc 2460
ttctgccagg aaacatcacc tgagatgaag gggaaggtgc tcactcggtt aaagcacatt 2520
gtagaattgc aaataatcct ggaaaagtac ttggcagtca ccccagacta tgtccctcct 2580
cttggaaact ttgatgtgga aactttagat ataacacctc atactgttac tgctatttca 2640
gcaaaaatca gaaagaaagg aaaaatagaa aggaaacaaa aaacagatgg cagcaagaca 2700
tcctcctctg acacactttc agaagagaaa aattcagaat gtgaccctac gccatctcat 2760
agaggccagc taaacaagga gttcacaggg aaggaagaaa agacatcatt gttactacat 2820
aattcccatg cttttttccg agagctggac attgaggtct tctctattct acattgtgga 2880
cttgtgacga agttcatctt agatactgaa atgcacactg aagctacaga agttgtgcaa 2940
cttgggcccc ctgagctgct tttcttgctg gaagatctct cccagaagct ggagagtatg 3000
ctgacacctc ctattgccag gagagtcccc tttctcaaga acaaaggaag ccggaatatt 3060
ggattctcac atctccaaca gagatctgcc caagaaattg ttcattgtgt ttttcaactg 3120
ctgaccccaa tgtgtaacca cctggagaac attcacaact attttcagtg tttagctgct 3180
gagaatcacg gtgtagttga tggaccagga gtgaaagttc aggagtacca cataatgtct 3240
tcctgctatc agaggctgct gcagattttt catgggcttt ttgcttggag tggattttct 3300
caacctgaaa atcagaattt actgtattca gccctccatg tccttagtag ccgactgaaa 3360
cagggagaac acagccagcc tttggaggaa ctactcagcc agagcgtcca ttacttgcag 3420
aatttccatc aaagcattcc cagtttccag tgtgctcttt atctcatcag acttttgatg 3480
gttattttgg agaaatcaac agcttctgct cagaacaaag aaaaaattgc ttcccttgcc 3540
agacaattcc tctgtcgggt gtggccaagt ggggataaag agaagagcaa catctctaat 3600
gaccagctcc atgctctgct ctgtatctac ctggagcaca cagagagcat tctgaaggcc 3660
atagaggaga ttgctggtgt tggtgtccca gaactgatca actctcctaa agatgcatct 3720
tcctccacat tccctacact gaccaggcat acttttgttg ttttcttccg tgtgatgatg 3780
gctgaactag agaagacggt gaaaaaaatt gagcctggca cagcagcaga ctcgcagcag 3840
attcatgaag agaaactcct ctactggaac atggctgttc gagacttcag tatcctcatc 3900
aacttgataa aggtatttga tagtcatcct gttctgcatg tatgtttgaa gtatgggcgt 3960
ctctttgtgg aagcatttct gaagcaatgt atgccgctcc tagacttcag ttttagaaaa 4020

caccgggaag atgttctgag cttactggaa accttccagt tggacacaag gctgcttcat 4080
cacctgtgtg ggcattccaa gattcaccag gacacgagac tcacccaaca tgtgcctctg 4140
ctcaaaaaga ccctggaact tttagtttgc agagtcaaag ctatgctcac tctcaacaat 4200
tgtagagagg ctttctggct gggcaatcta aaaaaccggg acttgcaggg tgaagagatt 4260
aagtcccaaa attcccagga gagcacagca gatgagagtg aggatgacat gtcatcccag 4320
gcctccaaga gcaaagccac tgaggatggt gaagaagacg aagtaagtgc tggagaaaag 4380
gagcaagata gtgatgagag ttatgatgac tctgattaga ccccagataa attgttgcct 4440
gcttctgtgt ctcaa 4455

<210> 8
<211> 5516
<212> DNA
<213> Mus musculus

<400> 8
ggaaagtcga aaacgaaggg aagcaactgg cgggtcccca ggaagtaata taagtggcag 60
aagacgttag tcaaaatgat ttccaaaaga cgtcggctag attctgagga taaagaaaac 120
ctgacagaag atgcctccaa aaccatgccc ctttccaagc tggcaaagaa gtctcacaat 180
tctcatgaag ttgaagaaaa tggcagtgtc tttgtaaagc ttcttaaggc ttcaggactc 240
actcttaaaa ctggagagaa ccaaaatcag ctaggtgtgg atcaggtaat cttccaaagg 300
aagctctttc aggccttgag gaagcatcct gcttatccca aagtaataga agagtttgtt 360
aatggcctgg agtcctacac tgaggacagt gagagtctca ggaactgcct gctgtcttgt 420
gagcgcctgc aggatgagga agccagcatg ggcacatttt actccaagag tctgatcaag 480
ctacttctgg ggattgacat tttacagcct gccattatca aaatgttatt tgaaaaagtg 540
cctcagtttc tttttgaaag tgagaacaga gatggaatca acatggccag actcattatc 600
aatcaactaa aatggctgga tagaattgtg gatggcaagg acctcacggc ccagatgatg 660
cagttgatca gtgttgctcc cgtgaactta cagcatgact tcatcacgag ccttcctgaa 720
atcctagggg attcccagca tgctaatgtg gggaaagagc ttggcgagct gctggtgcag 780
aatacttccc tgactgttcc aattttggat gtcttttcca gtctccgact tgaccccaac 840
ttcctgtcca agatccgcca gttggtgatg ggcaagctgt catctgtccg tctagaggat 900
ttccctgtga ttgtaaagtt ccttcttcat tctgtaacag acaccacttc ccttgaggtc 960
attgccgagc ttcgggagaa cttgaacgtc cagcagttta ttttgccgtc acgaattcag 1020
gcttcccaaa gcaaattgaa aagtaaagga ctagcaagct cttcaggaaa tcaagagaac 1080
agtgataaag actgtattgt tcttgtcttt gatgtaataa agtcagccat tagatatgag 1140
aaaaccattt cagaggcctg gtttaaggca attgaacgca ttgagtccgc ggctgaacat 1200
aaggctttgg acgtggtcat gctgctcatc atctacagca ccagcacgca gaccaagaag 1260
ggcgtggaga agctgctgag aaacaagatt cagtcagact gcattcaaga acagctgctt 1320
gacagtgcgt tctctacaca ttacctggtt cttaaggata tttgcccatc tattcttttg 1380
ctggctcaga ctttgtttca ctctcaagac cagaggatca ttttgtttgg cagtcttctg 1440
tacaaatatg cttttaagtt ttttgatact tactgccagc aggaagtggt tggtgcccta 1500
gtcacccatg tctgcagtgg gactgaggct gaagtcgaca ctgcactgga tgtcctcctg 1560
gagctgattg tgctaaacgc ctctgctatg aggctcaatg ctgcttttgt taagggcatc 1620
ttagattatt tggaaaatat gtcccctcag caaatacgaa aaatcttctg tattctcagc 1680
actcttgcat ttagccaaca gcccggaacc agcaaccata tccaggacga catgcacctg 1740
gtgatccgga agcagctctc tagcactgtg ttcaagtaca agctcattgg gatcattggt 1800
gcagtcacca tggccggcat catggcggaa gacagaagtg taccatctaa ctcatcccag 1860
aggagcgcca atgtgagcag tgagcagcgc acacaggtga cttctttgct acaactagtt 1920
cattcttgca ctgagcactc tccttgggcc tcttctctgt attatgatga atttgccaac 1980
ctgatccaag aaaggaagtt ggctccaaaa accttggagt gggttgggca gaccatcttc 2040
aatgatttcc aagatgcctt tgtggtagac ttctgtgctg ctccagaggg tgactttcca 2100
tttcctgtga aagcgctcta tggactggaa gagtacagca ctcaagacgg cattgtcatc 2160
aacctcctgc cgctgttcta tcaggaatgt gcaaaagatg ccagtcgagc gacatcacaa 2220
gaatcgagcc agagatcaat gtcttctttg tgcctggctt cccatttccg gctgctgaga 2280
ctttgcgtgg caagacaaca tgatggaaac ttggatgaga tcgatggtct cttagattgt 2340
cccctgttcc tccctgacct ggaacctgga gagaaactgg agtccatgtc tgctaaagac 2400
cgttcgctta tgtgttcgct cacattccta actttcaact ggttccgaga ggttgtgaat 2460
gccttctgcc aacaaacatc tcctgagatg aagggcaagg ttcttagtcg gctaaaggac 2520
cttgtagaac ttcagggaat cctagagaag tacttggcag tcatcccaga ctatgttccg 2580
cctttcgcaa gcgttgactt ggacacttta gatatgatgc ctaggagcag ttctgctgtt 2640
gcagcaaaaa acagaaacaa gggaaagacg gggggaaaga aacaaaaagc tgatagcaac 2700
aaagcatcct gttcggacac acttctaaca gaagacactt cagagtgtga catggcgcca 2760
tctgggagaa gccacgtaga caaggagtcc acagggaagg aaggaaagac gtttgtgtca 2820
ctgcagaatt accgcgcttt tttccgagag ctggacattg aggtcttctc tattctacat 2880
tctggacttg tgaccaagtt catcttagac actgaaatgc acactgaagc tacagaggtc 2940
gtacagctgg ggcctgctga gctgctcttc ttgctggaag atctttccca gaagctagag 3000
aatatgctga ctgctccttt tgccaagaga atctgctgct ttaagaataa aggaaggcag 3060
aatattggct tctcacatct tcatcagaga tctgtccagg acattgtgca ctgtgtggtt 3120
cagctgctaa ccccgatgtg taaccatctg gagaacattc acaacttctt tcagtgctta 3180

ggtgctgagc atctcagtgc agatgacaag gcgagagcga cagctcagga gcagcacacc 3240
atggcctgct gctaccagaa gctgctgcag gtcttgcacg cgctctttgc gtggaaggga 3300
tttactcacc aatcaaagca ccgcctcctg cactcagccc ttgaggtcct ctcgaaccga 3360
ctaaagcaga tggaacagga ccagcccttg gaggaactgg tcagccagag cttcagttac 3420
ttgcagaact tccaccatag tgttcccagt ttccagtgtg gtctctacct tctcagactt 3480
ctgatggccc ttctggagaa gtctgcagta cctaaccaga agaaagaaaa acttgcctct 3540
ctggccaaac agctgctttg ccgagcatgg cctcatgggg aaaaagagaa gaaccccact 3600
tttaatgacc acctgcatga tgtgctttac atctacttgg agcacacaga caatgttctg 3660
aaggccatag aggagatyac tggtgttggt gtcccagaac tggtcagtgc tccgaaagac 3720
gccgcctcct ctacattccc tacgttgacc grgcacacct ttgtcatatt cttccgtgtg 3780
atgatggctg aactcgagaa gacggtgaag ggtctycagg ctggcacagc agcagattcg 3840
cagcaggttc acgaagagaa gctcctctat tkgaacatgg ctgtccgaga tttcagyatc 3900
cttytcaatc tgatgaaagt atttgacagt tatcctgttc tgcatgtgtg tttaaagtat 3960
ggccgtcgct ttgtggaggc atttctgaag caatgtatgc cactcctcga cttcagcttt 4020
agaaagcatc gggaagatgt tctgagcttg ctgcaaaccc ttcagttgaa cacgaggcta 4080
cttcatcacc tttgtggaca ctccaagatt cgccaggaca caagactcac caagcaygtg 4140
cctttactca aaaagtcact ggaactgtta gtttgcagag tcaaagccat gcttgtcctc 4200
aacaactgta gagaggcttt ctggttgggt actctcaaaa accgagactt acagggtgaa 4260
gaaattattt cccaggatcc ctcttcctca gagagcaatg cagaggacag tgaggatggc 4320
gtgacatctc acgtctccag gaacagagca acagaggatg gggaagatga agcaagtgat 4380
gaacagaagg accaggacag tgatgaaagt gacgacagct ccagttagag ccgagtggca 4440
tggctgccct gctcacctct gacagactct catctctttg gggtttgaag tcagatgtct 4500
gtttttctag tcagaagcat cctgtttgtc catcaagaag gggtgtttat ttaattcccc 4560
agtgggtttc acaggttgtc taacctccag gtccctggtt caggagtcca gtgtagcatc 4620
catcgttgac taggaygaac atggctgggc tgcagtgcag tkcagtgcag gtgccctagc 4680
tgggccttgg ggttttgaaa ctaaaattta ggcttataat agctttgtaa ataaatctgt 4740
ttcagagttt tgcctcagct acctttttcc tcactttaga tgtgattatt caaggatctc 4800
attattcaag gattaggtaa tattgagttg aggtttgtgc aatcgtactg gtggcctaaa 4860
agtatgttcc gtactgttat cttcctggag gaatgaccca actttcttat caatgatcaa 4920
gtgtttggtt tggtctgtgt cagggtctct ttacatagtc ctggctggtg tgttattaga 4980
tatgttgacc aggagggtct tgaacattac ttttgaattt taaacatttt tgtacatatg 5040
tgtatgggca tatatgtgcc actgtgcata tgtgtaggtc agaggatagc ttatgggagt 5100
gagctctctc cttccaccat gtgggttcca gggttcaaac tctagacctt cacctgctca 5160
gccaccttac ccttttaaaa tgtttggtta ttaatatata aaaggaagga agacaacatc 5220
aaacatgtgc tggctttgta tgtatatata gtttttattt ccacattaat ttgaattatg 5280
cctataatat atttgtaata atcatacaaa ataattgtaa tttattagaa atagaacatc 5340
aggagttaaa ataggggatt cttctgtctt ctgccaggaa gcccagtctc agagatgctg 5400
ccaggctctt cctcgctgtg ccattaagat tatttaattt ttgttaatat ttttactcat 5460
accggtatta aagttatgtt ttgttggaaa aaaaaaaaaa aaaaaaaaaa aaaaaa 5516

<210> 9
<211> 14
<212> DNA
<213> Homo sapiens

<220>
<221> MISC_FEATURE
<222> (1)..(14)
<223> Intron/Exon Junction of FANCD

<400> 9
tcggtgagta agtg 14

<210> 10
<211> 14
<212> DNA
<213> Homo sapiens

<220>
<221> MISC_FEATURE
<222> (1)..(14)
<223> Intron/Exon Junction of FANCD

<400> 10
ccagtaagta tcta 14

<210> 11
<211> 14

<212> DNA
<213> Homo sapienss

<220>
<221> MISC_FEATURE
<222> (1)..(14)
<223> Intron/Exon Junction of FANCD

<400> 11
taggtaatat ttta                                                          14

<210> 12
<211> 14
<212> DNA
<213> Homo sapienss

<220>
<221> MISC_FEATURE
<222> (1)..(14)
<223> Intron/Exon Junction of FANCD

<400> 12
aaagtatgta tttt                                                          14

<210> 13
<211> 14
<212> DNA
<213> Homo sapiens

<220>
<221> MISC_FEATURE
<222> (1)..(14)
<223> Intron/Exon Junction of FANCD

<400> 13
caggtgtgga gagg                                                          14

<210> 14
<211> 14
<212> DNA
<213> Homo sapiens

<220>
<221> MISC_FEATURE
<222> (1)..(14)
<223> Intron/Exon Junction of FANCD

<400> 14
caggtaagac tgtc                                                          14

<210> 15
<211> 14
<212> DNA
<213> Homo sapiens

<220>
<221> MISC_FEATURE
<222> (1)..(14)
<223> Intron/Exon Junction of FANCD

<400> 15
aaagtaagtg gcgt                                                          14

<210> 16
<211> 14
<212> DNA
<213> Homo sapiens

<220>
<221> MISC_FEATURE
<222> (1)..(14)
<223> Intron/Exon Junction of FANCD

<400> 16
aaggtaggct tatg                                                          14

<210> 17
<211> 14
<212> DNA
<213> Homo sapiens

<220>
<221> MISC_FEATURE
<222> (1)..(14)
<223> Intron/Exon Junction of FANCD

<400> 17
caggtggata aacc                                                          14

<210> 18
<211> 14
<212> DNA
<213> Homo sapiens

<220>
<221> MISC_FEATURE
<222> (1)..(14)
<223> Intron/Exon Junction of FANCD

<400> 18
aaggtagaaa agac                                                          14

<210> 19
<211> 14
<212> DNA
<213> Homo sapiens

<220>
<221> MISC_FEATURE
<222> (1)..(14)
<223> Intron/Exon Junction of FANCD

<400> 19
gaggtatgct ctta                                                          14

<210> 20
<211> 14
<212> DNA
<213> Homo sapiens

<220>
<221> MISC_FEATURE
<222> (1)..(14)
<223> Intron/Exon Junction of FANCD

<400> 20
aaggtaaaga gctc                                                          14

<210> 21
<211> 14
<212> DNA
<213> Homo sapiens

<220>
<221> MISC_FEATURE
<222> (1)..(14)

<223> Intron/Exon Junction of FANCD

<400> 21
aaggtgagat cttt                                                    14

<210> 22
<211> 14
<212> DNA
<213> Homo sapiens

<220>
<221> MISC_FEATURE
<222> (1)..(14)
<223> Intron/Exon Junction of FANCD

<400> 22
aaggtaatgt tcat                                                    14

<210> 23
<211> 14
<212> DNA
<213> Homo sapiens

<220>
<221> MISC_FEATURE
<222> (1)..(14)
<223> Intron/Exon Junction of FANCD

<400> 23
ttagtaagtg tcag                                                    14

<210> 24
<211> 14
<212> DNA
<213> Homo sapiens

<220>
<221> MISC_FEATURE
<222> (1)..(14)
<223> Intron/Exon Junction of FANCD

<400> 24
caggtatgtt gaaa                                                    14

<210> 25
<211> 14
<212> DNA
<213> Homo sapiens

<220>
<221> MISC_FEATURE
<222> (1)..(14)
<223> Intron/Exon Junction of FANCD

<400> 25
aaggtatctt attg                                                    14

<210> 26
<211> 14
<212> DNA
<213> Homo sapiens

<220>
<221> MISC_FEATURE
<222> (1)..(14)
<223> Intron/Exon Junction of FANCD

<400> 26

caggttagag gcaa                                                    14

<210> 27
<211> 14
<212> DNA
<213> Homo sapiens

<220>
<221> MISC_FEATURE
<222> (1)..(14)
<223> Intron/Exon Junction of FANCD

<400> 27
caggtacacg tgga                                                    14

<210> 28
<211> 14
<212> DNA
<213> Homo sapiens

<220>
<221> MISC_FEATURE
<222> (1)..(14)
<223> Intron/Exon Junction of FANCD

<400> 28
caggtgagtt cttt                                                    14

<210> 29
<211> 14
<212> DNA
<213> Homo sapiens

<220>
<221> MISC_FEATURE
<222> (1)..(14)
<223> Intron/Exon Junction of FANCD

<400> 29
ctggtaaagc caat                                                    14

<210> 30
<211> 14
<212> DNA
<213> Homo sapiens

<220>
<221> MISC_FEATURE
<222> (1)..(14)
<223> Intron/Exon Junction of FANCD

<400> 30
agggtaggta ttgt                                                    14

<210> 31
<211> 14
<212> DNA
<213> Homo sapiens

<220>
<221> MISC_FEATURE
<222> (1)..(14)
<223> Intron/Exon Junction of FANCD

<400> 31
aaagtcagta tagt                                                    14

<210> 32

<211> 14
<212> DNA
<213> Homo sapiens

<220>
<221> MISC_FEATURE
<222> (1)..(14)
<223> Intron/Exon Junction of FANCD

<400> 32
taggtatggg atga                                                          14

<210> 33
<211> 14
<212> DNA
<213> Homo sapiens

<220>
<221> MISC_FEATURE
<222> (1)..(14)
<223> Intron/Exon Junction of FANCD

<400> 33
gaggtgagca gagt                                                          14

<210> 34
<211> 14
<212> DNA
<213> Homo sapiens

<220>
<221> MISC_FEATURE
<222> (1)..(14)
<223> Intron/Exon Junction of FANCD

<400> 34
caggtaagag aagt                                                          14

<210> 35
<211> 14
<212> DNA
<213> Homo sapiens

<220>
<221> MISC_FEATURE
<222> (1)..(14)
<223> Intron/Exon Junction of FANCD

<400> 35
taggtaagta tgtt                                                          14

<210> 36
<211> 14
<212> DNA
<213> Homo sapiens

<220>
<221> MISC_FEATURE
<222> (1)..(14)
<223> Intron/Exon Junction of FANCD

<400> 36
aaggtattgg aatg                                                          14

<210> 37
<211> 14
<212> DNA
<213> Homo sapiens

<220>
<221> MISC_FEATURE
<222> (1)..(14)
<223> Intron/Exon Junction of FANCD

<400> 37
gaagtaagtg acag                                                         14

<210> 38
<211> 14
<212> DNA
<213> Homo sapiens

<220>
<221> MISC_FEATURE
<222> (1)..(14)
<223> Intron/Exon Junction of FANCD

<400> 38
aaggttagtg tagg                                                         14

<210> 39
<211> 14
<212> DNA
<213> Homo sapiens

<220>
<221> MISC_FEATURE
<222> (1)..(14)
<223> Intron/Exon Junction of FANCD

<400> 39
caggtcagaa gcct                                                         14

<210> 40
<211> 14
<212> DNA
<213> Homo sapiens

<220>
<221> MISC_FEATURE
<222> (1)..(14)
<223> Intron/Exon Junction of FANCD

<400> 40
ttggtaagta tgtg                                                         14

<210> 41
<211> 14
<212> DNA
<213> Homo sapiens

<220>
<221> MISC_FEATURE
<222> (1)..(14)
<223> Intron/Exon Junction of FANCD

<400> 41
caggtgagtc ataa                                                         14

<210> 42
<211> 14
<212> DNA
<213> Homo sapiens

<220>
<221> MISC_FEATURE

<222> (1)..(14)
<223> Intron/Exon Junction of FANCD

<400> 42
ttggtgatgg gcct                                                    14

<210> 43
<211> 14
<212> DNA
<213> Homo sapiens

<220>
<221> MISC_FEATURE
<222> (1)..(14)
<223> Intron/Exon Junction of FANCD

<400> 43
ctggtgagat gttt                                                    14

<210> 44
<211> 14
<212> DNA
<213> Homo sapiens

<220>
<221> MISC_FEATURE
<222> (1)..(14)
<223> Intron/Exon Junction of FANCD

<400> 44
caggtaaggg agtt                                                    14

<210> 45
<211> 14
<212> DNA
<213> Homo sapiens

<220>
<221> MISC_FEATURE
<222> (1)..(14)
<223> Intron/Exon Junction of FANCD

<400> 45
caggtgagta agat                                                    14

<210> 46
<211> 14
<212> DNA
<213> Homo sapiens

<220>
<221> MISC_FEATURE
<222> (1)..(14)
<223> Intron/Exon Junction of FANCD

<400> 46
aaggtgagta tgga                                                    14

<210> 47
<211> 14
<212> DNA
<213> Homo sapiens

<220>
<221> MISC_FEATURE
<222> (1)..(14)
<223> Intron/Exon Junction of FANCD

<400> 47
aaggtgagag attt                                                          14

<210> 48
<211> 14
<212> DNA
<213> Homo sapiens

<220>
<221> MISC_FEATURE
<222> (1)..(14)
<223> Intron/Exon Junction of FANCD

<400> 48
cgggtaagag ctaa                                                          14

<210> 49
<211> 14
<212> DNA
<213> Homo sapiens

<220>
<221> MISC_FEATURE
<222> (1)..(14)
<223> Intron/Exon Junction of FANCD

<400> 49
aaggtaagaa gggg                                                          14

<210> 50
<211> 14
<212> DNA
<213> Homo sapiens

<220>
<221> MISC_FEATURE
<222> (1)..(14)
<223> Intron/Exon Junction of FANCD

<400> 50
caggtaagcc ttgg                                                          14

<210> 51
<211> 14
<212> DNA
<213> Homo sapiens

<220>
<221> MISC_FEATURE
<222> (1)..(14)
<223> Intron/Exon Junction of FANCD

<400> 51
gaggtatctc taca                                                          14

<210> 52
<211> 23
<212> DNA
<213> Homo sapiens

<220>
<221> MISC_FEATURE
<222> (1)..(23)
<223> Intron/Exon Junction of FANCD

<400> 52
gtttcccgat tttgctctag gaa                                                23

<210> 53
<211> 23
<212> DNA
<213> Homo sapiens

<220>
<221> MISC_FEATURE
<222> (1)..(23)
<223> Intron/Exon Junction of FANCD

<400> 53
gaaaattttt ctattttcag aaa                                              23

<210> 54
<211> 23
<212> DNA
<213> Homo sapiens

<220>
<221> MISC_FEATURE
<222> (1)..(23)
<223> Intron/Exon Junction of FANCD

<400> 54
ctcttctttt ttctgcatag ctg                                              23

<210> 55
<211> 23
<212> DNA
<213> Homo sapiens

<220>
<221> MISC_FEATURE
<222> (1)..(23)
<223> Intron/Exon Junction of FANCD

<400> 55
   attttttaaa tctccttaag ata                                               23

<210> 56
<211> 23
<212> DNA
<213> Homo sapiens

<220>
<221> MISC_FEATURE
<222> (1)..(23)
<223> Intron/Exon Junction of FANCD

<400> 56
gatttctttt ttttttacag tat                                              23

<210> 57
<211> 23
<212> DNA
<213> Homo sapiens

<220>
<221> MISC_FEATURE
<222> (1)..(23)
<223> Intron/Exon Junction of FANCD

<400> 57
ccctatgtct tcttttttag cct                                              23

<210> 58
<211> 23
<212> DNA

<213> Homo sapiens

<220>
<221> MISC_FEATURE
<222> (1)..(23)
<223> Intron/Exon Junction of FANCD

<400> 58
ttctcttcct aacattttag caa                                                23

<210> 59
<211> 23
<212> DNA
<213> Homo sapiens

<220>
<221> MISC_FEATURE
<222> (1)..(23)
<223> Intron/Exon Junction of FANCD

<400> 59
aatagtgtct tctactgcag gac                                                23

<210> 60
<211> 23
<212> DNA
<213> Homo sapiens

<220>
<221> MISC_FEATURE
<222> (1)..(23)
<223> Intron/Exon Junction of FANCD

<400> 60
tctttttcta ccattcacag tga                                                23

<210> 61
<211> 23
<212> DNA
<213> Homo sapiens

<220>
<221> MISC_FEATURE
<222> (1)..(23)
<223> Intron/Exon Junction of FANCD

<400> 61
tctgtgcttt taatttttag gtt                                                23

<210> 62
<211> 23
<212> DNA
<213> Homo sapiens

<220>
<221> MISC_FEATURE
<222> (1)..(23)
<223> Intron/Exon Junction of FANCD

<400> 62
ctaatattta ctttctgcag gta                                                23

<210> 63
<211> 23
<212> DNA
<213> Homo sapiens

<220>

<221> MISC_FEATURE
<222> (1)..(23)
<223> Intron/Exon Junction of FANCD

<400> 63
ttcctctctg ctacttgtag ttc                                                23

<210> 64
<211> 23
<212> DNA
<213> Homo sapiens

<220>
<221> MISC_FEATURE
<222> (1)..(23)
<223> Intron/Exon Junction of FANCD

<400> 64
   actctctcct gttttttcag gca                                                 23

<210> 65
<211> 23
<212> DNA
<213> Homo sapiens

<220>
<221> MISC_FEATURE
<222> (1)..(23)
<223> Intron/Exon Junction of FANCD

<400> 65
tgcatattta ttgacaatag gtg                                                23

<210> 66
<211> 23
<212> DNA
<213> Homo sapiens

<220>
<221> MISC_FEATURE
<222> (1)..(23)
<223> Intron/Exon Junction of FANCD

<400> 66
tctactcttc cccactcaag gtt                                                23

<210> 67
<211> 23
<212> DNA
<213> Homo sapiens

<220>
<221> MISC_FEATURE
<222> (1)..(23)
<223> Intron/Exon Junction of FANCD

<400> 67
gttgactctc ccctgtatag gaa                                                23

<210> 68
<211> 23
<212> DNA
<213> Homo sapiens

<220>
<221> MISC_FEATURE
<222> (1)..(23)
<223> Intron/Exon Junction of FANCD

<400> 68
tggcatcatt ttttccacag ggc                                              23

<210> 69
<211> 23
<212> DNA
<213> Homo sapiens

<220>
<221> MISC_FEATURE
<222> (1)..(23)
<223> Intron/Exon Junction of FANCD

<400> 69
tcttcatcat ctcattgcag gat                                              23

<210> 70
<211> 23
<212> DNA
<213> Homo sapiens

<220>
<221> MISC_FEATURE
<222> (1)..(23)
<223> Intron/Exon Junction of FANCD

<400> 70
aaaaaattct ttgtttttag aag                                              23

<210> 71
<211> 23
<212> DNA
<213> Homo sapiens

<220>
<221> MISC_FEATURE
<222> (1)..(23)
<223> Intron/Exon Junction of FANCD

<400> 71
attcttcctc tttgctccag gtg                                              23

<210> 72
<211> 23
<212> DNA
<213> Homo sapiens

<220>
<221> MISC_FEATURE
<222> (1)..(23)
<223> Intron/Exon Junction of FANCD

<400> 72
tgtttgtttg cttcctgaag gaa                                              23

<210> 73
<211> 23
<212> DNA
<213> Homo sapiens

<220>
<221> MISC_FEATURE
<222> (1)..(23)
<223> Intron/Exon Junction of FANCD

<400> 73
attctggttt ttctccgcag tga                                              23

<210> 74
<211> 23
<212> DNA
<213> Homo sapiens

<220>
<221> MISC_FEATURE
<222> (1)..(23)
<223> Intron/Exon Junction of FANCD

<400> 74
aatttatttc tccttctcag att                                                23

<210> 75
<211> 23
<212> DNA
<213> Homo sapiens

<220>
<221> MISC_FEATURE
<222> (1)..(23)
<223> Intron/Exon Junction of FANCD

<400> 75
aaatgtttgt tctctctcag att                                                23

<210> 76
<211> 23
<212> DNA
<213> Homo sapiens

<220>
<221> MISC_FEATURE
<222> (1)..(23)
<223> Intron/Exon Junction of FANCD

<400> 76
atgtaatttg tactttgcag att                                                23

<210> 77
<211> 23
<212> DNA
<213> Homo sapiens

<220>
<221> MISC_FEATURE
<222> (1)..(23)
<223> Intron/Exon Junction of FANCD

<400> 77
cagcctgctg tttgtttcag tca                                                23

<210> 78
<211> 23
<212> DNA
<213> Homo sapiens

<220>
<221> MISC_FEATURE
<222> (1)..(23)
<223> Intron/Exon Junction of FANCD

<400> 78
ttctcttttt aatataaaag aaa                                                23

<210> 79
<211> 23

<212> DNA
<213> Homo sapiens

<220>
<221> MISC_FEATURE
<222> (1)..(23)
<223> Intron/Exon Junction of FANCD


<400> 79
ttgctgtgac ttccccatag gag                                              23

<210> 80
<211> 23
<212> DNA
<213> Homo sapiens

<220>
<221> MISC_FEATURE
<222> (1)..(23)
<223> Intron/Exon Junction of FANCD

<400> 80
tcctttcctc catgtgacag gct                                              23

<210> 81
<211> 23
<212> DNA
<213> Homo sapiens

<220>
<221> MISC_FEATURE
<222> (1)..(23)
<223> Intron/Exon Junction of FANCD

<400> 81
taactctgca tttattatag aac                                              23

<210> 82
<211> 23
<212> DNA
<213> Homo sapiens

<220>
<221> MISC_FEATURE
<222> (1)..(23)
<223> Intron/Exon Junction of FANCD

<400> 82
aaaatcattt ttatttttag tgt                                              23

<210> 83
<211> 23
<212> DNA
<213> Homo sapiens

<220>
<221> MISC_FEATURE
<222> (1)..(23)
<223> Intron/Exon Junction of FANCD

<400> 83
tcttaccttg acttccttag gag                                              23

<210> 84
<211> 23
<212> DNA
<213> Homo sapiens

<220>
<221> MISC_FEATURE
<222> (1)..(23)
<223> Intron/Exon Junction of FANCD

<400> 84
tttttcttgt ctccttacag cca                                                23

<210> 85
<211> 23
<212> DNA
<213> Homo sapiens

<220>
<221> MISC_FEATURE
<222> (1)..(23)
<223> Intron/Exon Junction of FANCD

<400> 85
tttgtcttct tttctaacag ctt                                                23

<210> 86
<211> 23
<212> DNA
<213> Homo sapiens

<220>
<221> MISC_FEATURE
<222> (1)..(23)
<223> Intron/Exon Junction of FANCD

<400> 86
atatttgact ctcaatgcag tat                                                23

<210> 87
<211> 23
<212> DNA
<213> Homo sapiens

<220>
<221> MISC_FEATURE
<222> (1)..(23)
<223> Intron/Exon Junction of FANCD

<400> 87
atgcttttcc cgtcttctag gca                                                23

<210> 88
<211> 23
<212> DNA
<213> Homo sapiens

<220>
<221> MISC_FEATURE
<222> (1)..(23)
<223> Intron/Exon Junction of FANCD

<400> 88
catatatttg gctgccccag att                                                23

<210> 89
<211> 23
<212> DNA
<213> Homo sapiens

<220>
<221> MISC_FEATURE

<222> (1)..(23)
<223> Intron/Exon Junction of FANCD

<400> 89
cttgtctttc acctctccag gta                                              23

<210> 90
<211> 23
<212> DNA
<213> Homo sapiens

<220>
<221> MISC_FEATURE
<222> (1)..(23)
<223> Intron/Exon Junction of FANCD

<400> 90
agtgtgtctc tcttcttcag tat                                              23

<210> 91
<211> 23
<212> DNA
<213> Homo sapiens

<220>
<221> MISC_FEATURE
<222> (1)..(23)
<223> Intron/Exon Junction of FANCD

<400> 91
tataaactta ttggttatag gaa                                              23

<210> 92
<211> 23
<212> DNA
<213> Homo sapiens

<220>
<221> MISC_FEATURE
<222> (1)..(23)
<223> Intron/Exon Junction of FANCD

<400> 92
tgttatttat ttccattcag att                                              23

<210> 93
<211> 23
<212> DNA
<213> Homo sapiens

<220>
<221> MISC_FEATURE
<222> (1)..(23)
<223> Intron/Exon Junction of FANCD

<400> 93
cttggtccat tcacatttag ggt                                              23

<210> 94
<211> 23
<212> DNA
<213> Homo sapiens

<220>
<221> MISC_FEATURE
<222> (1)..(23)
<223> Intron/Exon Junction of FANCD

<400> 94
atttattctt tgccccttag gat                                              23

<210> 95
<211> 33
<212> DNA
<213> Artificial Sequence

<220>
<223> DF4EcoRI

<400> 95
agcctcgaat tcgtttccaa aagaagactg tca                                   33

<210> 96
<211> 35
<212> DNA
<213> Artificial Sequence

<220>
<223> DR816Xh

<400> 96
ggtatcctcg agtcaagacg acaacttatc catca                                 35

<210> 97
<211> 18
<212> DNA
<213> Artificial Sequence

<220>
<223> MG471

<400> 97
aatcgaaaac tacgggcg                                                    18

<210> 98
<211> 20
<212> DNA
<213> Artificial Sequence

<220>
<223> MG457

<400> 98
gagaacacat gaatgaacgc                                                  20

<210> 99
<211> 28
<212> DNA
<213> Artificial Sequence

<220>
<223> MG492

<400> 99
ggcgacggct tctcggaagt aatttaag                                         28

<210> 100
<211> 18
<212> DNA
<213> Artificial Sequence

<220>
<223> MG472

<400> 100
agcggcagga ggtttatg                                                    18

<210> 101
<211> 18
<212> DNA
<213> Artificial Sequence

<220>
<223> MG474

<400> 101
tggcggcaga cagaagtg                                                    18

<210> 102
<211> 18
<212> DNA
<213> Artificial Sequence

<220>
<223> MG475

<400> 102
tggcggcaga cagaagtg                                                    18

<210> 103
<211> 21
<212> DNA
<213> Artificial Sequence

<220>
<223> MG491

<400> 103
agagagccaa cctgagcgat g                                                21

<210> 104
<211> 18
<212> DNA
<213> Artificial Sequence

<220>
<223> MG476

<400> 104
gtgccagact ctggtggg                                                    18

<210> 105
<211> 20
<212> DNA
<213> Artificial Sequence

<220>
<223> MG792

<400> 105
aggagacacc cttcctatcc                                                  20

<210> 106
<211> 20
<212> DNA
<213> Artificial Sequence

<220>
<223> MG803

<400> 106
gaagttggca aaacagactg                                                  20

<210> 107

<211> 21
<212> DNA
<213> Artificial Sequence

<220>
<223> MG924

<400> 107
tgtcttgtga gcgtctgcag g                                                21

<210> 108
<211> 20
<212> DNA
<213> Artificial Sequence

<220>
<223> MG753

<400> 108
aggttttgat aatggcaggc                                                  20

<210> 109
<211> 24
<212> DNA
<213> Artificial Sequence

<220>
<223> MG979

<400> 109
actggactgt gcctacccac tatg                                             24

<210> 110
<211> 20
<212> DNA
<213> Artificial Sequence

<220>
<223> MG984

<400> 110
cctgtgtgag gatgagctct                                                  20

<210> 111
<211> 16
<212> DNA
<213> Artificial Sequence

<220>
<223> R302W

<400> 111
ttctcccgaa gctcag                                                      16

<210> 112
<211> 17
<212> DNA
<213> Artificial Sequence

<220>
<223> R1236H

<400> 112
tttcttccgt gtgatga                                                     17

<210> 113
<211> 33
<212> DNA

<213> Artificial Sequence

<220>
<223> DF4EcoRI

<400> 113
agcctcgaat tcgtttccaa aagaagactg tca                                33

<210> 114
<211> 35
<212> DNA
<213> Artificial Sequence

<220>
<223> DR816Xh

<400> 114
ggtatcctcg agtcaagacg acaacttatc catca                              35

<210> 115
<211> 20
<212> DNA
<213> Artificial Sequence

<220>
<223> MG914

<400> 115
ctagcacaga actctgctgc                                               20

<210> 116
<211> 20
<212> DNA
<213> Artificial Sequence

<220>
<223> MG837

<400> 116
ctagcacaga actctgctgc                                               20

<210> 117
<211> 24
<212> DNA
<213> Artificial Sequence

<220>
<223> MG746

<400> 117
cttcagcaac agcgaagtag tctg                                          24

<210> 118
<211> 24
<212> DNA
<213> Artificial Sequence

<220>
<223> MG747

<400> 118
gattctcagc acttgaaaag cagg                                          24

<210> 119
<211> 21
<212> DNA
<213> Artificial Sequence

<220>
<223> MG773

<400> 119
ggacacatca gttttcctct c                                                21

<210> 120
<211> 20
<212> DNA
<213> Artificial Sequence

<220>
<223> MG789

<400> 120
gaaaacccat gattcagtcc                                                  20

<210> 121
<211> 20
<212> DNA
<213> Artificial Sequence

<220>
<223> MG816

<400> 121
tcatcaggca agaaacttgg                                                  20

<210> 122
<211> 20
<212> DNA
<213> Artificial Sequence

<220>
<223> MG803

<400> 122
gaagttggca aaacagactg                                                  20

<210> 123
<211> 20
<212> DNA
<213> Artificial Sequence

<220>
<223> MG804

<400> 123
gagccatctg ctcatttctg                                                  20

<210> 124
<211> 20
<212> DNA
<213> Artificial Sequence

<220>
<223> MG812

<400> 124
cccgctattt agacttgagc                                                  20

<210> 125
<211> 21
<212> DNA
<213> Artificial Sequence

<220>
<223> MG775

<400> 125
caaagtgttt attccaggag c                                                21

<210> 126
<211> 22
<212> DNA
<213> Artificial Sequence

<220>
<223> MG802

<400> 126
catcagggta ctttgaacat tc                                               22

<210> 127
<211> 22
<212> DNA
<213> Artificial Sequence

<220>
<223> MG727

<400> 127
ttgaccagaa aggctcagtt cc                                               22

<210> 128
<211> 23
<212> DNA
<213> Artificial Sequence

<220>
<223> MG915

<400> 128
agatgatgcc agagggttta tcc                                              23

<210> 129
<211> 20
<212> DNA
<213> Artificial Sequence

<220>
<223> MG790

<400> 129
tgcccagctc tgttcaaacc                                                  20

<210> 130
<211> 20
<212> DNA
<213> Artificial Sequence

<220>
<223> MG774

<400> 130
aggcaatgac tgactgacac                                                  20

<210> 131
<211> 23
<212> DNA
<213> Artificial Sequence

<220>
<223> MG805

<400> 131

tgcccgtcta tttttgatga agc                                              23

<210> 132
<211> 21
<212> DNA
<213> Artificial Sequence

<220>
<223> MG791

<400> 132
tctcagttag tctggggaca g                                                21

<210> 133
<211> 25
<212> DNA
<213> Artificial Sequence

<220>
<223> MG751

<400> 133
tcatggtaga gagactggac tgtgc                                            25

<210> 134
<211> 21
<212> DNA
<213> Artificial Sequence

<220>
<223> MG972

<400> 134
accctggagc aaatgacaac c                                                21

<210> 135
<211> 21
<212> DNA
<213> Artificial Sequence

<220>
<223> MG973

<400> 135
atttgctcca gggtacatgg c                                                21

<210> 136
<211> 22
<212> DNA
<213> Artificial Sequence

<220>
<223> MG974

<400> 136
gaaagacagt gggaaggcaa gc                                               22

<210> 137
<211> 23
<212> DNA
<213> Artificial Sequence

<220>
<223> MG975

<400> 137
gggagtgtgt ggaacaaatg agc                                              23

<210> 138
<211> 25
<212> DNA
<213> Artificial Sequence

<220>
<223> MG976

<400> 138
agtttctaca ggctggtcct attcc                                          25

<210> 139
<211> 21
<212> DNA
<213> Artificial Sequence

<220>
<223> MG755

<400> 139
aacgtggaat cccattgatg c                                              21

<210> 140
<211> 20
<212> DNA
<213> Artificial Sequence

<220>
<223> MG730

<400> 140
tttctgtgtt ccctccttgc                                                20

<210> 141
<211> 20
<212> DNA
<213> Artificial Sequence

<220>
<223> MG794

<400> 141
gatggtcaag ttacactggc                                                20

<210> 142
<211> 24
<212> DNA
<213> Artificial Sequence

<220>
<223> MG778

<400> 142
cacctcccac caattatagt attc                                           24

<210> 143
<211> 23
<212> DNA
<213> Artificial Sequence

<220>
<223> MG808

<400> 143
ctatgtgtgt ctcttttaca ggg                                            23

<210> 144
<211> 20

<212> DNA
<213> Artificial Sequence

<220>
<223> MG817

<400> 144
aatctttccc accatattgc                                                    20

<210> 145
<211> 20
<212> DNA
<213> Artificial Sequence

<220>
<223> MG779

<400> 145
   cataccttct tttgctgtgc                                                    20

<210> 146
<211> 23
<212> DNA
<213> Artificial Sequence

<220>
<223> MG795

<400> 146
ccacagaagt cagaatctcc acg                                                23

<210> 147
<211> 20
<212> DNA
<213> Artificial Sequence

<220>
<223> MG731

<400> 147
tgtaacaaac ctgcacgttg                                                    20

<210> 148
<211> 23
<212> DNA
<213> Artificial Sequence

<220>
<223> MG732

<400> 148
tgctacccaa gccagtagtt tcc                                                23

<210> 149
<211> 22
<212> DNA
<213> Artificial Sequence

<220>
<223> MG788

<400> 149
gagtttggga aagattggca gc                                                 22

<210> 150
<211> 22
<212> DNA
<213> Artificial Sequence

<220>
<223> MG772

<400> 150
tgtagtaaag cagctctcat gc                                                22

<210> 151
<211> 20
<212> DNA
<213> Artificial Sequence

<220>
<223> MG733

<400> 151
caagtacact ctgcactgcc                                                   20

<210> 152
<211> 23
<212> DNA
<213> Artificial Sequence

<220>
<223> MG758

<400> 152
tgactcaact tccccaccaa gag                                               23

<210> 153
<211> 24
<212> DNA
<213> Artificial Sequence

<220>
<223> MG736

<400> 153
ctccctatgt acgtggagta atac                                              24

<210> 154
<211> 21
<212> DNA
<213> Artificial Sequence

<220>
<223> MG737

<400> 154
gggagtcttg tgggaactaa g                                                 21

<210> 155
<211> 23
<212> DNA
<213> Artificial Sequence

<220>
<223> MG780

<400> 155
ttcatagaca tctctcagct ctg                                               23

<210> 156
<211> 20
<212> DNA
<213> Artificial Sequence

<220>

<223> MG759

<400> 156
gttttggtat cagggaaagc                                                    20

<210> 157
<211> 20
<212> DNA
<213> Artificial Sequence

<220>
<223> MG760

<400> 157
agccatgctt ggaattttgg                                                    20

<210> 158
<211> 20
<212> DNA
<213> Artificial Sequence

<220>
<223> MG781

<400> 158
ctcactggga tgtcacaaac                                                    20

<210> 159
<211> 25
<212> DNA
<213> Artificial Sequence

<220>
<223> MG740

<400> 159
ggtcttgatg tgtgacttgt atccc                                              25

<210> 160
<211> 25
<212> DNA
<213> Artificial Sequence

<220>
<223> MG741

<400> 160
cctcagtgtc acagtgttct ttgtg                                              25

<210> 161
<211> 22
<212> DNA
<213> Artificial Sequence

<220>
<223> MG809

<400> 161
catgaaatga ctaggacatt cc                                                 22

<210> 162
<211> 20
<212> DNA
<213> Artificial Sequence

<220>
<223> MG797

<400> 162
ctacccagtg acccaaacac                                                    20

<210> 163
<211> 23
<212> DNA
<213> Artificial Sequence

<220>
<223> MG761

<400> 163
cgaaccctta gtttctgaga cgc                                                23

<210> 164
<211> 20
<212> DNA
<213> Artificial Sequence

<220>
<223> MG742

<400> 164
tcagtgcctt ggtgactgtc                                                    20

<210> 165
<211> 21
<212> DNA
<213> Artificial Sequence

<220>
<223> MG916

<400> 165
ttgatggtac agactggagg c                                                  21

<210> 166
<211> 23
<212> DNA
<213> Artificial Sequence

<220>
<223> MG810

<400> 166
aagaaagttg ccaatcctgt tcc                                                23

<210> 167
<211> 20
<212> DNA
<213> Artificial Sequence

<220>
<223> MG762

<400> 167
agcacctgaa aataaggagg                                                    20

<210> 168
<211> 22
<212> DNA
<213> Artificial Sequence

<220>
<223> MG743

<400> 168
gcccaaagtt tgtaagtgtg ag                                                 22

<210> 169
<211> 21
<212> DNA
<213> Artificial Sequence

<220>
<223> MG787

<400> 169
agcaagaatg aggtcaagtt c                                                21

<210> 170
<211> 24
<212> DNA
<213> Artificial Sequence

<220>
<223> MG806

<400> 170
gggaaaaact ggaggaaaga actc                                             24

<210> 171
<211> 21
<212> DNA
<213> Artificial Sequence

<220>
<223> MG818

<400> 171
agaggtaggg aaggaagcta c                                                21

<210> 172
<211> 20
<212> DNA
<213> Artificial Sequence

<220>
<223> MG813

<400> 172
ccaaagtcca cttcttgaag                                                  20

<210> 173
<211> 20
<212> DNA
<213> Artificial Sequence

<220>
<223> MG834

<400> 173
gatgcactgg ttgctacatc                                                  20

<210> 174
<211> 20
<212> DNA
<213> Artificial Sequence

<220>
<223> MG836

<400> 174
ccaggacact tggtttctgc                                                  20

<210> 175

<211> 20
<212> DNA
<213> Artificial Sequence

<220>
<223> MG839

<400> 175
acactcccag ttggaatcag                                                    20

<210> 176
<211> 20
<212> DNA
<213> Artificial Sequence

<220>
<223> MG871

<400> 176
cttgtgggca agaaattgag                                                    20

<210> 177
<211> 20
<212> DNA
<213> Artificial Sequence

<220>
<223> MG829

<400> 177
tgggctggat gagactattc                                                    20

<210> 178
<211> 24
<212> DNA
<213> Artificial Sequence

<220>
<223> MG870

<400> 178
ccaaggacat atcttctgag caac                                               24

<210> 179
<211> 20
<212> DNA
<213> Artificial Sequence

<220>
<223> MG820

<400> 179
tgattatcag cataggctgg                                                    20

<210> 180
<211> 20
<212> DNA
<213> Artificial Sequence

<220>
<223> MG811

<400> 180
gatcccccaa tagcaactgc                                                    20

<210> 181
<211> 21
<212> DNA

<213> Artificial Sequence

<220>
<223> MG763

<400> 181
cattcagatt caccaggaca c                                                21

<210> 182
<211> 20
<212> DNA
<213> Artificial Sequence

<220>
<223> MG782

<400> 182
ccttacatgc catctgatgc                                                  20

<210> 183
<211> 20
<212> DNA
<213> Artificial Sequence

<220>
<223> MG764

<400> 183
aaccttctcc cctattaccc                                                  20

<210> 184
<211> 22
<212> DNA
<213> Artificial Sequence

<220>
<223> MG835

<400> 184
ggaaaatgag aggctataat gc                                               22

<210> 185
<211> 24
<212> DNA
<213> Artificial Sequence

<220>
<223> MG1006

<400> 185
tgtattccag aggtcaccca gagc                                             24

<210> 186
<211> 22
<212> DNA
<213> Artificial Sequence

<220>
<223> MG1005

<400> 186
ccagtaagaa aggcaaacag cg                                               22

<210> 187
<211> 1094
<212> DNA
<213> Artificial Sequence

<220>
<223> FANCD-S.ORF.s

<400> 187
ggagaacaca gccagccttt ggaggaacta ctcagccaga gcgtccatta cttgcagaat 60
ttccatcaaa gcattcccag tttccagtgt gctctttatc tcatcagact tttgatggtt 120
attttggaga aatcaacagc ttctgctcag aacaaagaaa aaattgcttc ccttgccaga 180
caattcctct gtcgggtgtg gccaagtggg gataaagaga agagcaacat ctctaatgac 240
cagctccatg ctctgctctg tatctacctg gagcacacag agagcattct gaaggccata 300
gaggagattg ctggtgttgg tgtcccagaa ctgatcaact ctcctaaaga tgcatcttcc 360
tccacattcc ctacactgac caggcatact tttgttgttt tcttccgtgt gatgatggct 420
gaactagaga agacggtgaa aaaaattgag cctggcacag cagcagactc gcagcagatt 480
catgaagaga aactcctcta ctggaacatg gctgttcgag acttcagtat cctcatcaac 540
ttgataaagg tatttgatag tcatcctgtt ctgcatgtat gtttgaagta tgggcgtctc 600
tttgtggaag catttctgaa gcaatgtatg ccgctcctag acttcagttt tagaaaacac 660
cgggaagatg ttctgagctt actggaaacc ttccagttgg acacaaggtg cttcatcacc 720
tgtgtgggca ttccaagatt caccaggaca cgagactcac ccaacatgtg cctctgctca 780
aaaagaccct ggaactttta gtttgcagag tcaaagctat gctcactctc aacaacaatt 840
gtagagaggc tttctggctg ggcaatctaa aaaaccggga cttgcagggt gaagagatta 900
agtcccaaaa ttcccaggag agcacagcag atgagagtga ggatgacatg tcatcccagg 960
cctccaagag caaagccact gaggatggtg aagaagacga agtaagtgct ggagaaaagg 1020
agcaagatag tgatgagagt tatgatgact ctgattagac cccagataaa ttgttgcctg 1080
cttctgtgtc tcaa 1094

<210> 188
<211> 1115
<212> DNA
<213> Artificial Sequence

<220>
<223> FANCD cDNA OR

<400> 188
ggagaacaca gccagccttt ggaggaacta ctcagccaga gcgtccatta cttgcagaat 60
ttccatcaaa gcattcccag tttccagtgt gctctttatc tcatcagact tttgatggtt 120
attttggaga aatcaacagc ttctgctcag aacaaagaaa aaattgcttc ccttgccaga 180
caattcctct gtcgggtgtg gccaagtggg gataaagaga agagcaacat ctctaatgac 240
cagctccatg ctctgctctg tatctacctg gagcacacag agagcattct gaaggccata 300
gaggagattg ctggtgttgg tgtcccagaa ctgatcaact ctcctaaaga tgcatcttcc 360
tccacattcc ctacactgac caggcatact tttgttgttt tcttccgtgt gatgatggct 420
gaactagaga agacggtgaa aaaaattgag cctggcacag cagcagactc gcagcagatt 480
catgaagaga aactcctcta ctggaacatg gctgttcgag acttcagtat cctcatcaac 540
ttgataaagg tatttgatag tcatcctgtt ctgcatgtat gtttgaagta tgggcgtctc 600
tttgtggaag catttctgaa gcaatgtatg ccgctcctag acttcagttt tagaaaacac 660
cgggaagatg ttctgagctt actggaaacc ttccagttgg acacaaggtg cttcatcacc 720
tgtgtgggca ttccaagatt caccaggaca cgagactcac ccaacatgtg cctctgctca 780
aaaagaccct ggaactttta gtttgcagag tcaaagctat gctcactctc aacaattgta 840
gagaggcttt ctggctgggc aatctaaaaa accgggactt gcagggtgaa gagattaagt 900
cccaaaattc ccaggagagc acagcagatg agagtgagga tgacatgtca tcccaggcct 960
ccaagagcaa agccactgag gtatctctac aaaacccacc agagtctggc actgatggtt 1020
gcattttgtt aattgttcta agttggtgga gcagaacttt gcctacttat gtttattgtc 1080
aaatgcttct atgcccattt ccattccctc cataa 1115

<210> 189
<211> 52
<212> DNA
<213> Artificial Sequence

<220>
<223> Wild-type

<400> 189
ggaagccagg tgtggagagg aggcatggaa tcttgctgaa attcagtctg tc 52

<210> 190
<211> 52
<212> DNA
<213> Artificial Sequence

<220>
<223> Maternal Mutation

<400> 190
ggaagccggg tgtggagagg aggcatggaa tcttgctgaa attcagtctg tc          52

<210> 191
<211> 52
<212> DNA
<213> Artificial Sequence

<220>
<223> Maternal Mutation and Reversion

<400> 191
ggaagccggg tgtgaagagg aggcatggaa tcttgctgaa attcagtctg tc          52